# EUROPEAN PATENT SPECIFICATION

(11) **EP 3 512 870 B1**
(45) Date of publication and mention of the grant of the patent: **03.08.2022**
(21) Application number: 17850354.6
(22) Date of filing: 24.05.2017
(51) Int. Cl.: C12N 15/113, A61K 31/7088, A61P 25/02

(54) **SCN9A ANTISENSE OLIGONUCLEOTIDES**
SCN9A-ANTISENSE-OLIGONUKLEOTIDE
OLIGONUCLÉOTIDES ANTISENS SCN9A

(30) Priority: 16.09.2016 US 201662395814 P
(43) Date of publication of application: 24.07.2019
(73) Proprietor: Olipass Corporation, Yongin-si, Gyeonggi-do 17015 (KR)
(72) Inventor: CHUNG, Shin, Yongin-si Gyeonggi-do 16990 (KR); JUNG, Daram, Hwaseong-si Gyeonggi-do (KR); CHO, Bongjun, Yongin-si Gyeonggi-do 17077 (KR); JANG, Kangwon, Yongin-si Gyeonggi-do 16998 (KR); YOON, Heungsik, Seongnam-si Gyeonggi-do 13623 (KR)
(74) Representative: HGF
(86) International application number: PCT/IB2017/000751
(87) International publication number: WO 2018/051175

(56) References cited:
- WO-A2-2009/033027
- WO-A2-2009/113828
- KR-A- 20110 087 436
- US-A1- 2013 210 884
- US-B1- 6 617 422
- EDWARD C EMERY ET AL: "Na v 1.7 and other voltage-gated sodium channels as drug targets for pain relief", EXPERT OPINION ON THERAPEUTIC TARGETS, vol. 20, no. 8, 12 April 2016 (2016-04-12) , pages 975-983, XP55437812, UK ISSN: 1472-8222, DOI: 10.1517/14728222.2016.1162295
- AURÉLIEN CHATELIER ET AL: "Biophysical Properties of Human Na v 1.7 Splice Variants and Their Regulation by Protein Kinase A", JOURNAL OF NEUROPHYSIOLOGY, vol. 99, no. 5, 12 March 2008 (2008-03-12) , pages 2241-2250, XP55675232, US ISSN: 0022-3077, DOI: 10.1152/jn.01350.2007
- YUKIKO MUROI ET AL: "Selective silencing of Na V 1.7 decreases excitability and conduction in vagal sensory neurons", THE JOURNAL OF PHYSIOLOGY, vol. 589, no. 23, 17 October 2011 (2011-10-17), pages 5663-5676, XP55339184, GB ISSN: 0022-3751, DOI: 10.1113/jphysiol.2011.215384
- JUN PAN ET AL: "Effect of down-regulation of voltage-gated sodium channel Nav1.7 on activation of astrocytes and microglia in DRG in rats with cancer pain", ASIAN PACIFIC JOURNAL OF TROPICAL MEDICINE, vol. 8, no. 5, 20 May 2015 (2015-05-20), pages 405-411, XP55624126, Singapore ISSN: 1995-7645, DOI: 10.1016/S1995-7645(14)60352-7
- JOOST P.H. DRENTH ET AL: "Mutations in sodium-channel gene SCN9A cause a spectrum of human genetic pain disorders", JOURNAL OF CLINICAL INVESTIGATION, vol. 117, no. 12, 3 December 2007 (2007-12-03), pages 3603-3609, XP55675224, GB ISSN: 0021-9738, DOI: 10.1172/JCI33297
- CHOI, JIN-SUNG ET AL.: 'Alternative splicing may contribute to time- dependent manifestation of inherited erythromelalgia' BRAIN vol. 133, 16 May 2010, pages 1823 - 1835, XP055475230
- KARRAS, JAMES G. ET AL.: 'Peptide nucleic acids are potent modulators of endogenous pre-mRNA splicing of the murine interleukin-5 receptor- a chain' BIOCHEMISTRY vol. 40, no. 26, 06 May 2001, pages 7853 - 7859, XP055463390

## Description

This invention relates to peptide nucleic acid derivatives targeting SCN9A pre-mRNA for the treatment of pains and disorders mediated through voltage-gated sodium channel Naᵥ1.7 subtype, and claims the benefit of priority to U.S. Provisional Application No. 62/395,814 filed on September 16th of 2016.

### Background of Invention

Voltage-gated sodium channels (VGSCs) are trans-membrane proteins composed of α and β subunits. VGSCs function as a gateway for sodium ions to cross the cell membrane. Sodium channel activity is produced by α-subunit. VGSC subtype is defined according to the subtype of α-subunit. To date, there are at least 10 subtypes of VGSC, i.e. Naᵥ1.1, Naᵥ1.2, ..., Naᵥ1.9, and Naₓ.

Each VGSC subtype has a distinct α subunit, and is destined to show biological function depending on the tissue of its expression. For example, Naᵥ1.2 subtype is expressed in central neurons. Naᵥ1.2 appears to be linked to epilepsy. [Human Mol. Genet. vol 24(5), 1459-1468 (2015**)]** Naᵥ1.5 subtype is abundantly expressed in cardiomyocytes. Inhibition of Naᵥ1.5 may cause long QT syndrome and sudden death. [Handbook Exp. Pharmacol. vol 221, 137-168 (2014**)]** Naᵥ1.7 subtype is abundantly expressed in dorsal root ganglia. Upregulation of the Naᵥ1.7 activity causes erythromelalgia. [J. Med. Genet. vol 41, 171-174 (**2004**)] In the meantime, people genetically lacking the Naᵥ1.7 activity (SCN9A channelopathy) do not feel severe pains, although those individuals were found to be normal in other sensory functions. [Nature vol 444, 894-898 (2006**)]**

Tetrodotoxin (TTX) is a neurotoxin found in pufferfish. TTX is extremely toxic and its intra-peritoneal LD₅₀ is 10 µg/Kg in mice. [Toxins vol 6, 693-755 (2014**)].** Oral ingestion of TTX can cause parethesia of the lips and tongue, hypersalivation, sweating, headache, tremor, paralysis, cyanosis, seizures, incoordination, diarrhea, abdominal pain, hypotension, respiratory distress, cardiac arrhythmias, coma, and so on. TTX is known to induce such adverse effects by non-specifically binding to the active sites of VGSC subtypes. Thus nonspecific inhibition of VGSC subtypes is considered to be a dangerous therapeutic option incurring serious adverse events.

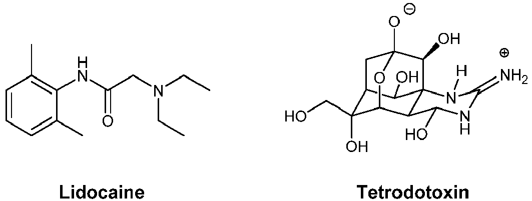

Lidocaine is a non-specific VGSC inhibitor, and has been widely used as a local anesthetic agent. Upon intravenous administration, lidocaine may induce undesirable side effects such as muscle twitching, vomiting, irregular heartbeat, sleepiness, and so on. Such side effects are considered to be due to nonspecific inhibition of VGSC subtypes. However, the inhibition of Naᵥ1.5 with lidocaine would be useful to treat ventricular tachycardia. Nevertheless, systemic administration of lidocaine is considered to be undesirable for the treatment of chronic pains due to adverse events arising from non-specific inhibition of sodium channel subtypes.

SCN9A Channelopathy: SCN9A (sodium channel subtype 9A) gene encodes the α-subunit of VGSC subtype Naᵥ1.7. There are an extremely small number of individuals who do not feel severe pains but are normal in other sensory functions. Such individuals were found to have the SCN9A gene mutated to encode nonfunctional Naᵥ1.7 subtype. [Nature vol 444, 894-898 (2006**)]** This has been termed as SCN9A channelopathy. The behavioral phenotypes of human SCN9A channelopathy are reproduced fairly much in SCN9A knockout mice. [*PLoS One* 9(9): e105895 **(2014)]** Therefore selective inhibition of Naᵥ1.7 subtype would be useful to safely treat chronic pains.

Naᵥ1.7 Selective Small Molecule Inhibitors: Reflecting the physiological function of VGSC, the active sites of VGSC subtypes are similar in their 3D structure. By directly targeting the active site with small molecule inhibitors, selective inhibition of Naᵥ1.7 subtype would be highly challenging. Lidocaine and tetrodotoxin are good examples for such non-selective inhibition of VGSC subtypes.

Naᵥ1.7 inhibitors with a modest selectivity over Naᵥ1.5 (ca. 8-fold) were identified through a high throughput screen campaign with a library of 200,000 compounds to identify Naᵥ1.8 selective inhibitors. [J. Gen. Physiol. vol 131(5), 399-405 (2008**)]** A 1-benzazepin-2one derivative provided below was found to selectively inhibit Naᵥ1.7 over Naᵥ1.5 with a modest Naᵥ1.7 selectivity (ca 8-fold) by electrophysiology assay.

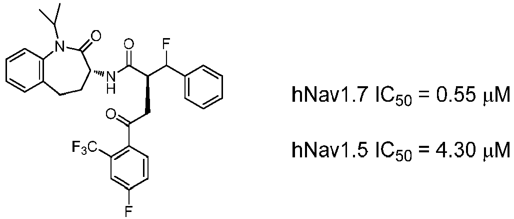

To date, a number of Naᵥ1.7 selective small molecule inhibitors have been disclosed, and several were evaluated in human patients. For example, funapide (XEN-402/TV-45070) was evaluated in a small number of erythromelalgia patients. [Pain vol 153, 80-85 (2012)] Although funapide showed analgesic activity, funapide showed treatment related and dose limiting adverse events including dizziness and somnolence in a relatively large portion of the enrolled patients. The CNS adverse events suggest that the Naᵥ1.7 selectivity of funapide may not be high enough to safely treat chronic pains.

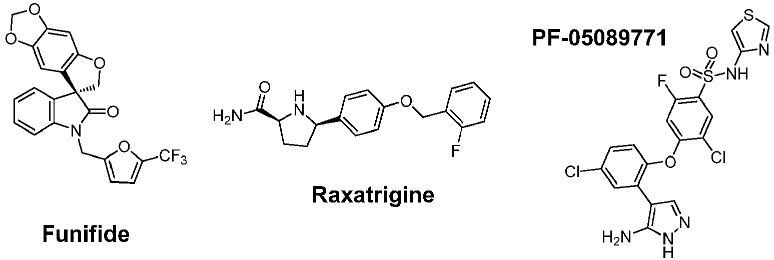

Raxatrigine (CNV1014802/GSK-1014802) inhibits Naᵥ1.7 as well as other VGSC subtypes. However, raxatrigine is said to inhibit the functional activity of sodium channel by selectively stabilizing the inactive state of sodium channel. Although raxatrigine inhibits sodium channels in the CNS, it is said to be well tolerated at therapeutic dose. [The Pharmaceutical J. 11 Mar. 2016. Naᵥ1.7: a new channel for pain treatment] In a Phase IIa clinical study in trigeminal neuralgia patients, raxatrigine 150 mg TID was well tolerated, although the dosing schedule failed to significantly meet the primary therapeutic endpoint possibly due to a limited efficacy for the number of enrolled subjects. [J. M. Zakrzewska et al. Lancet Neurol. Published Online Feb 16, 2017, http://dx.doi.org/10.1016/S1472-4422(17) 30005-4]

PF-05089771 is a Naᵥ1.7 selective inhibitor with an ICso of 11 nM. PF-05089771 was reported to stabilize the inactive form of Naᵥ1.7. [Biophysical J. vol 108(2) Suppl., 1573a-1574a (2015**)]** The therapeutic potential PF-05089771 was evaluated in patients of erythromelalgia or dental pain following a wisdom tooth extraction. A pharmacokinetic analysis of PF-05089771 suggested that the low drug concentration in the target tissue of neuropathic pain could be a possible explanation for its poor analgesic activity in human patients. [Clin. Pharmacokinet. vol 55(7), 875-87 (2016**)]**

Naᵥ1.7 selective small molecule inhibitors were reviewed from structural aspects. *[*Bioorg. Med. Chem. Lett. vol 24, 3690-3699 (2014**)]** The molecular size of such Naᵥ1.7 selective inhibitors tends to be considerably larger than lidocaine, a non-selective inhibitor of VGSC subtypes. Naᵥ1.7 selectivity was improved by making the molecular size of inhibitor large. Each Naᵥ1.7 selective inhibitor is considered to bind to a distinct domain within Naᵥ1.7 protein, and the binding domain varies depending on the chemical structure of the inhibitor. Ironically, the analgesic efficacy of Naᵥ1.7 selective inhibitors was not strong and failed to meet the expectation from the findings in people with SCN9A channelopathy. [Expert Opin. Ther. Targets vol 20(8), 975-983 (2016**)]**

Other Types of Naᵥ1.7 Selective Inhibitors: Tarantula venom peptide ProTx-II was found to selectively inhibit Naᵥ1.7 over other VGSC subtypes. However, the venom showed weak analgesic activity in animal models of acute inflammatory pain. [Mol. Pharmacol. vol 74, 1476-1484 (2008**)]** Given that the electrophysiology of the venom peptide was evaluated in HEK-293 cells engineered to abundantly express each subtype of VGSC, it is possible that ProTx-II may not bind to the active site of Naᵥ1,7 in primary neuronal cells expressing Naᵥ1.7.

Ssm6a, a 46-mer peptide isolated from centipede venom, was found to selectively inhibit Naᵥ1.7 over other VGSC subtypes. The Naᵥ1.7 ICso was observed to be 0.3 nM in HEK-293 cells engineered to overexpress Naᵥ1.7. The centipede venom peptide showed an analgesic efficacy comparable to morphine in mice formalin test, an acute inflammatory pain model. The 46-mer peptide also suppressed sodium current in rat DRG cells. Although the venom peptide showed a robust serum stability, the analgesic activity lasted only a few hours. [Proc. Nat. Acad. Sci. USA vol 110(43), 17534-17539 (2013**)]**

SVmab1 is a monoclonal antibody selectively targeting Naᵥ1.7 over other VGSC subtypes in HEK-293 cells over-expressing each VGSC subtype. SVmab1 selectively inhibited the sodium current evoked by Naᵥ1.7 with an IC₅₀ of 30 nM in HEK-293 cells. The monoclonal antibody showed a marked analgesic activity upon an intravenous (at 50 mg/Kg) or intrathecal (10 µg, i.e. ca 0.5 mg/Kg) administration in mice formalin test. Based on the difference in the analgesic potency of the two administration routes, the inhibition of Naᵥ1.7 in the spinal cord or CNS should be important for the analgesic activity against the formalin test. [Cell vol 157(6), 1393-1404 (2014**)]**

Ribosomal Protein Synthesis: Genetic information is coded in DNA (2-deoxyribose nucleic acid). DNA is transcribed to produce pre-mRNA (pre-messenger ribonucleic acid) in the nucleus. The introns of pre-mRNA are enzymatically spliced out to yield mRNA (messenger ribonucleic acid), which is then translocated into the cytosolic compartment. In the cytosol, a complex of translational machinery called ribosome binds to mRNA and carries out the protein synthesis as it scans the genetic information encoded along the mRNA. [Biochemistry vol 41, 4503-4510 (2002**);** Cancer Res. vol 48, 2659-2668 (1988**)]**

Antisense Oligonucleotide: An oligonucleotide binding to RNA or DN in a sequence specific manner (i.e. complementarily) is called antisense oligonucleotide (ASO). ASO may tightly bind to an mRNA or a pre-mRNA.

An ASO tightly binding to an mRNA can interrupt the protein synthesis by ribosome along the mRNA in the cytosol. The ASO needs to be present within the cytosol in order to inhibit the ribosomal protein synthesis of its target protein.

An ASO tightly binding to pre-mRNA can interfere with the splicing process of pre-mRNA. The ASO should be present in the nucleus to alter the splicing process and resultantly to induce exon skipping.

Unnatural Oligonucleotides: DNA or RNA oligonucleotide is susceptible to degradation by endogenous nucleases, limiting their therapeutic utility. To date, many types of unnatural oligonucleotides have been developed and studied intensively. [Clin. Exp. Pharmacol. Physiol. vol 33, 533-540 (2006**)]** Some of them show extended metabolic stability compared to DNA or RNA. Provided below are the chemical structures for some of representative unnatural oligonucleotides. Such oligonucleotides predictably bind to complementary nucleic acid as DNA or RNA does.

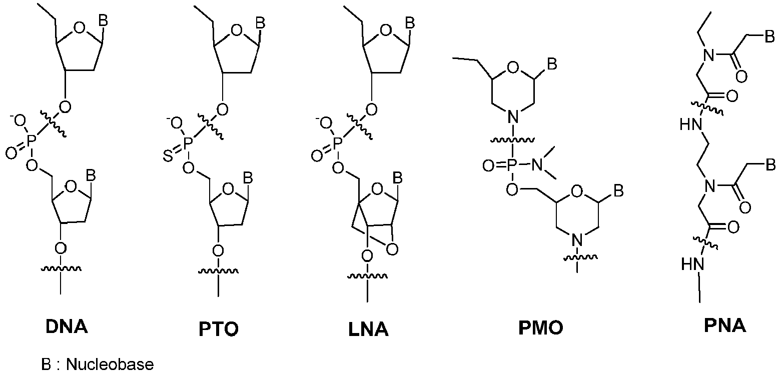

Phosphorothioate Oligonucleotide: Phosphorothioate oligonucleotide (PTO) is a DNA analog with one of the backbone phosphate oxygen atoms replaced with sulfur atom per monomer. Such a small structural change made PTO comparatively resistant to degradation by nucleases. [Ann. Rev. Biochem. vol 54, 367-402 (1985**)]**

Reflecting the structural similarity of backbone between PTO and DNA, they both poorly penetrate cell membrane in most mammalian cell types. For some types of cells abundantly expressing transporter(s) for DNA, however, DNA and PTO show good cell penetration. Systemically administered PTOs are known to readily distribute to the liver and kidney. [Nucleic Acids Res. vol 25, 3290-3296 (1997**)]**

In order to facilitate PTO's cell penetration in vitro, lipofection has been popularly practiced. However, lipofection physically alters cell membrane, potentially causes cytotoxicity, and therefore would not be ideal for chronic therapeutic use.

Over the past 30 years, antisense PTOs and variants of PTOs have been clinically evaluated to treat cancers, immunological disorders, metabolic diseases, and so on. [Biochemistry vol 41, 4503-4510 (2002**);** Clin. Exp. Pharmacol. Physiol. vol 33, 533-540 (2006**)]** Many of such antisense drug candidates have not been successfully developed partly due to PTO's poor cell penetration. In order to overcome the poor cell penetration, PTO needs to be administered at high dose for therapeutic activity. However, PTOs are known to show dose-limiting toxicity including increased coagulation time, complement activation, tubular nephropathy, Kupffer cell activation, and immune stimulation including splenomegaly, lymphoid hyperplasia, mononuclear cell infiltration. [Clin. Exp. Pharmacol. Physiol. vol 33, 533-540 (2006**)]**

Many antisense PTOs have been found to show due clinical activity for diseases with a significant contribution from the liver or kidney. Mipomersen is a PTO analog which inhibits the synthesis of apoB-100, a protein involved in LDL cholesterol transport. Mipomersen manifested due clinical activity in a certain population of atherosclerosis patients most likely due to its preferential distribution to the liver. [Circulation vol 118(7), 743-753 (2008**)]** ISIS-113715 is a PTO antisense analog inhibiting the synthesis of protein tyrosine phosphatase 1B (PTP1B), and was found to show therapeutic activity in type II diabetes patients. [Curr. Opin. Mol. Ther. vol 6, 331-336 (2004**)]**

Locked Nucleic Acid: In locked nucleic acid (LNA), the backbone ribose ring of RNA is structurally constrained to increase the binding affinity for RNA or DNA. Thus, LNA may be regarded as a high affinity DNA or RNA analog. [Biochemistry vol 45, 7347-7355 (2006**)]** However, LNA also shows poor cell penetration.

Phosphorodiamidate Morpholino Oligonucleotide: In phosphorodiamidate morpholino oligonucleotide (PMO), the backbone phosphate and 2-deoxyribose of DNA are replaced with phosphoroamidite and morpholine, respectively. [Appl. Microbiol. Biotechnol. vol 71, 575-586 (2006**)]** Whilst the DNA backbone is negatively charged, the PMO backbone is not charged. Thus the binding between PMO and mRNA is free of electrostatic repulsion between the backbones, and tends to be stronger than that between DNA and mRNA. Since PMO is structurally very different from DNA, PMO wouldn't be recognized by the hepatic transporter(s) recognizing DNA or RNA. However, PMO doesn't readily penetrate mammalian cell membrane.

Peptide Nucleic Acid: Peptide nucleic acid (PNA) is a polypeptide with N-(2-aminoethyl)glycine as the unit backbone, and was discovered by Dr. Nielsen and colleagues. [Science vol 254, 1497-1500 (1991**)]** The chemical structure and abbreviated nomenclature of prototype PNA are illustrated by the drawing provided below.

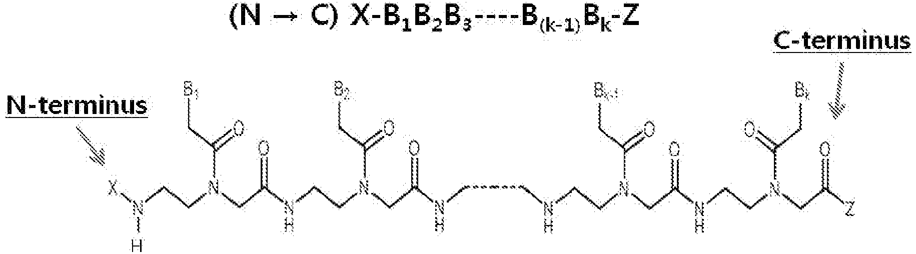

Like DNA and RNA, PNA selectively binds to complementary nucleic acid. [Nature (London) vol 365, 566-568 (1992**)]** In binding to complementary nucleic acid, the N-terminus of PNA is regarded as equivalent to the "5'-end" of DNA or RNA, and the C-terminus of PNA as equivalent to the "3'-end" of DNA or RNA.

Like PMO, the PNA backbone is not charged. Thus the binding between PNA and RNA tends to be stronger than that between DNA and RNA. Since PNA is markedly different from DNA in the chemical structure, PNA wouldn't be recognized by the hepatic transporter(s) recognizing DNA, and would show a tissue distribution profile different from that of DNA or PTO. However, PNA also poorly penetrates mammalian cell membrane. (Adv. Drug Delivery Rev. vol 55, 267-280, 2003**)**

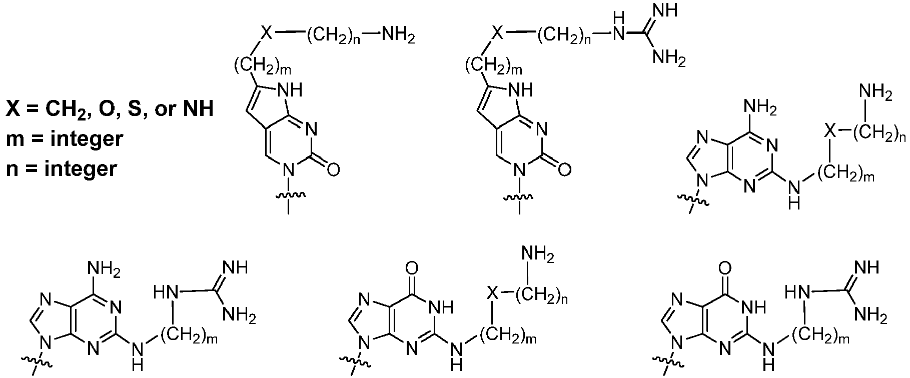

Modified Nucleobases to Improve Membrane Permeability of PNA: PNA was made highly permeable to mammalian cell membrane by introducing modified nucleobases with a cationic lipid or its equivalent covalently attached thereto. The chemical structures of such modified nucleobases are provided above. Such modified nucleobases of cytosine, adenine, and guanine were found to predictably and complementarily hybridize with guanine, thymine, and cytosine, respectively. [PCT Appl. No. PCT/KR2009/001256; EP2268607; US8680253]

Incorporation of such modified nucleobases onto PNA simulates situations of lipofection. By lipofection, oligonucleotide molecules are wrapped with cationic lipid molecules such as lipofectamine, and such lipofectamine/oligonucleotide complexes tend to penetrate membrane rather easily as compared to naked oligonucleotide molecules.

In addition to good membrane permeability, those PNA derivatives were found to show ultra-strong affinity for complementary nucleic acid. For example, introduction of 4 to 5 modified nucleobases onto 11- to 13-mer PNA derivatives readily yielded a Tₘ gain of 20°C or higher upon duplex formation with complementary DNA. Such PNA derivatives are highly sensitive to a single base mismatch. A single base mismatch resulted in a loss of 11 to 22°C in melting temperature (Tₘ) depending on the type of modified base as well as PNA sequence.

Small Interfering RNA (siRNA): Small interfering RNA (siRNA) refers to a double stranded RNA of 20-25 base pairs. [Microbiol. Mol. Biol. Rev. vol 67(4), 657-685 (2003**)]** The antisense strand of siRNA somehow interacts with proteins to form the "RNA-induced Silencing Complex" (RISC). Then the RISC binds to a certain portion of mRNA complementary to the antisense strand of siRNA. The mRNA complexed with RISC undergoes cleavage. Thus siRNA catalytically induces the cleavage of its target mRNA, and inhibits the protein expression by the mRNA. The RISC does not always bind to the full complementary sequence within its target mRNA, which raises concerns relating to off-target effects of a siRNA therapy. [Nature Rev. Drug Discov. vol 9, 57-67 (2010**)]** Like other classes of oligonucleotide with DNA or RNA backbone, siRNA possesses poor cell permeability and therefore tends to show poor in vitro or in vivo therapeutic activity unless properly formulated or chemically modified to show good membrane permeability.

SCN9A siRNA: A prior art disclosed siRNAs targeting a 19-mer sequence [(5' → 3') GAUUAUGGCUACACGAGCU] within exon 8 of the human SCN9A mRNA. [US8183221] Upon an intrathecal infusion, the siRNAs were claimed to show therapeutic activity in animal models of neuropathic pain and inflammatory pain. The siRNAs were said to down-regulate Naᵥ1.7 expression in rat DRG cells.

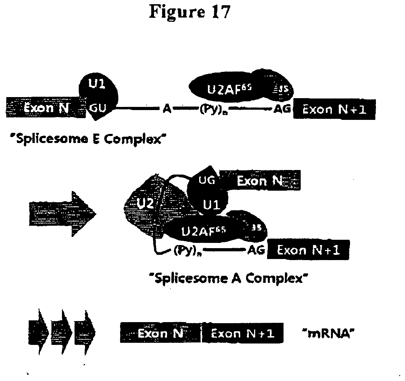

Splicing: DNA is transcribed to produce pre-mRNA (pre-messenger ribonucleic acid) in the nucleus. Pre-mRNA is then processed into mRNA following deletion of introns by a series of complex reactions collectively called "splicing" as schematically summarized in the diagram in figure 17. [Ann. Rev. Biochem. 72(1), 291-336 (2003); Nature Rev. Mol. Cell Biol. 6(5), 386-398 (2005); Nature Rev. Mol. Cell Biol. 15(2), 108-121 (2014)]

Splicing is initiated by forming "splicesome E complex" (i.e. early splicesome complex) between pre-mRNA and splicing adapter factors. In "splicesome E complex", U1 binds to the junction of exon N and intron N, and U2AF³⁵ binds to the junction of intron N and exon (N+1). Thus the junctions of exon/intron or intron/exon are critical to the formation of the early splicesome complex. "Splicesome E complex" evolves into "splicesome A complex" upon additional complexation with U2. The "splicesome A complex" undergoes a series of complex reactions to delete or splice out the intron to adjoin the neighboring exons.

Antisense Inhibition of Splicing: In the nucleus, ASO may tightly bind to a certain position within a pre-mRNA, and can interfere with the splicing process of the pre-mRNA into mRNA, producing an mRNA or mRNAs lacking the target exon. Such mRNA(s) is called "splice variant(s)", encodes protein(s) shorter than the protein encoded by the full-length mRNA.

In principle, splicing can be interrupted by inhibiting the formation of "splicesome E complex". If an ASO tightly binds to a junction of (5' → 3') exon-intron, i.e. "5' splice site", the ASO blocks the complex formation between pre-mRNA and factor U1, and therefore the formation of "splicesome E complex". Likewise, "splicesome E complex" cannot be formed if an ASO tightly binds to a junction of (5' → 3') intron-exon, i.e. "3' splice site".

KR20110087436A describes an antisense oligonucleic acid suppressing the synthesis of the sodium ion channel Naᵥ1.7.

Antisense Inhibition of SCN9A Pre-mRNA Splicing: To date, there are no reported cases of SCN9A ASOs inducing an alternative splicing of SCN9A pre-mRNA.

### Brief Description of Drawings

**Figures 1(A)****-(C).** Examples of natural and unnatural nucleobases selectable for the peptide nucleic acid derivative of **Formula I.**
**Figures 2(A)****-(E).** Examples of substituents selectable for the peptide nucleic acid derivative of **Formula I.**
**Figure 3****.** Chemical structures for PNA monomers with natural or modified nucleobase.
**Figure 4****.** Chemical structures for abbreviations of N- or C-terminus substituents.
**Figure 5(A)****.** Chemical structure for "(N → C) Fethoc-TA(5)A-A(5)TA(5)-CGC(1O2)-AA(5)A-A(5)A-NH₂".
**Figure 5(B)****.** Chemical structure for "(N → C) Fethoc-AG(5)C-A(5)CT-TA(5)C-GC(1O2)A-A(5)AA(2O2)-A-Lys-NH₂".
**Figure 6****.** Chemical structures for Fmoc-PNA monomers employed to synthesize the PNA derivatives of this invention.
**Figures 7(A)** and **(B)** shows C₁₈-reverse phase HPLC chromatograms of "ASO 4" before and after HPLC purification, respectively.
**Figure 8****.** ESI-TOF mass spectrum for "ASO 4" after purification by C₁₈-reverse phase chromatography.
**Figure 9(A)****.** Electrophoretic analysis of the SCN9A nested PCR products of PC3 cells treated with "ASO 9" at 0 (negative control), 10, 100 or 1,000 zM.
**Figure 9(B)****.** Sanger sequencing data for the PCR product bands assigned to the skipping of "exon 4" (top) and "exons 4-5" (bottom), respectively.
**Figure 10(A)****.** SCN9A nested qPCR data obtained with PC3 cells treated with "ASO 9" at 0 (negative control), 10, 100 or 1,000 zM. (error bar by standard error)
**Figure 10(B)****.** CoroNa assay data obtained in PC3 cells treated with "ASO 9" at 0 (negative control), 100 or 1,000 zM.
**Figure 11(A)**. SCN9A nested qPCR data in PC3 cells treated with "ASO 4" at 0 (negative control), 10, 100 or 1,000 zM. (error bar by standard error)
**Figure 11(B)**. SCN9A nested qPCR data in PC3 cells treated with "ASO 5" at 0 (negative control), 10, 100 or 1,000 zM. (error bar by standard error)
**Figure 11(C)**. SCN9A nested qPCR data in PC3 cells treated with "ASO 1" at 0 (negative control), 10, 100 or 1,000 zM. (error bar by standard error)
**Figure 11(D)**. SCN9A nested qPCR data in PC3 cells treated with "ASO 6" at 0 (negative control), 10 or 100 zM. (error bar by standard error)
**Figure 11(E)**. SCN9A nested qPCR data in PC3 cells treated with "ASO 10" at 0 (negative control), 10 or 100 zM. (error bar by standard error)
**Figure 12(A)****.** Traces of average intracellular fluorescence by CoroNa assay in PC3 cells following a 30 hour incubation with "ASO 10" at 0 (negative control), 100 or 1,000 zM.
**Figure 12(B)****.** Traces of average intracellular fluorescence by CoroNa assay in PC3 cells following a 30 hour incubation with "ASO 6" at 0 (negative control), 100 or 1,000 zM.
**Figure 12(C)****.** Traces of average intracellular fluorescence by CoroNa assay in PC3 cells following a 30 hour incubation with "ASO 4" at 0 (negative control), 100 or 1,000 zM.
**Figure 12(D)****.** Traces of average intracellular fluorescence by CoroNa assay in PC3 cells following a 30 hour incubation with "ASO 5" at 0 (negative control), 100 or 1,000 zM.
**Figure 13****.** Reversal of the allodynia induced with SNL (L5 ligation with L6 cut) in SD rats subcutaenously administered with "ASO 1" at 0 (negative control), 3 or 10 pmole/Kg. (error bar by standard error)
**Figure 14****.** Reversal of the allodynia induced by DPNP in rats subcutaenously adminstered with "ASO 5" at 0 (negative cotrol), 0.01, 0.1, 1 or 10 pmole/Kg, or orally with pregabalin 30 mg/Kg (positive control), (error bar by standard error)
**Figure 15****.** Reversal of the allodynia induced by DPNP in rats subcutaneously administered with "ASO 9" or "ASO 10" 100 pmole/Kg, or vehicle only. (error bar by standard error)
**Figure 16****.** Reversal of the allodynia induced by SNL (L5/L6 ligation) in SD rats subcutaneously receiving "ASO 9" at 0 (negative control) or 100 pmole/Kg, TID (3X per day).
**Figure 17****:** Pre-mRNA is processed into mRNA following deletion of introns by a series of complex reactions collectively called "splicing".

### Summary of Invention

The present invention provides a peptide nucleic acid derivative represented by **Formula I,** or a pharmaceutically acceptable salt thereof: 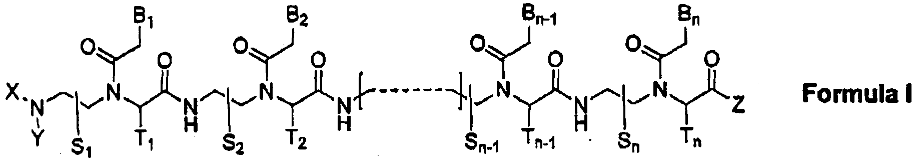 wherein,
n is an integer between 10 and 21:
the compound of **Formula I** possesses at least a 10-mer complementary overlap with the 14-mer RNA sequence of [(5' → 3') UUUUUGCGUAAGUA] within the human SCN9A pre-mRNA;
the compound of **Formula I** is fully complementary to the target pre-mRNA sequence, or partially complementary to the target pre-mRNA sequence with one or two mismatches;
S₁, S₂, ..., Sₙ₋₁, Sₙ, T₁, T₂, ..., Tₙ₋₁, and Tₙ independently represent deuterido, hydrido, substituted or non-substituted alkyl, or substituted or non-substituted aryl radical;
X and Y independently represent hydrido [H], formyl [H-C(=O)-]. aminocarbonyl [NH₂-C(=O)-], substituted or non-substituted alkyl, substituted or non-substituted aryl, substituted or non-substituted alkylacyl, substituted or non-substituted arylacyl, substituted or non-substituted alkyloxycarbonyl, substituted or non-substituted aryloxycarbonyl, substituted or non-substituted alkylaminocarbonyl, substituted or non-substituted arylaminocarbonyl, substituted or non-substituted alkylsulfonyl, or substituted or non-substituted arylsulfonyl radical;
Z represents hydroxy, substituted or non-substituted alkyloxy, substituted or non-substituted aryloxy, substituted or non-substituted amino, substituted or non-substituted alkyl, or substituted or non-substituted aryl radical;
B₁, B₂, ..., Bₙ₋₁, and Bₙ are independently selected from natural nucleobases including adenine, thymine, guanine, cytosine and uracil, and unnatural nucleobases; and,
at least four of B₁, B₂, ..., Bₙ₋₁, and Bₙ are independently selected from unnatural nucleobases with a substituted or non-substituted amino radical covalently linked to the nucleobase moiety.

The compound of **Formula I** induces alternative splicing of the human SCN9A pre-mRNA, yields SCN9A mRNA splice variant(s) lacking "exon 4", and is useful to treat pains, or conditions involving Naᵥ1.7 activity.

### Description of Invention

The present invention provides a peptide nucleic acid derivative represented by **Formula I,** or a pharmaceutically acceptable salt thereof: 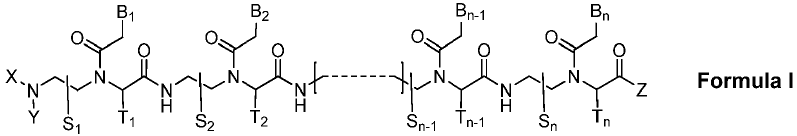 wherein,
n is an integer between 10 and 21;
the compound of **Formula I** possesses at least a 10-mer complementary overlap with the 14-mer RNA sequence of [(5' → 3') UUUUUGCGUAAGUA] within the human SCN9A pre-mRNA;
the compound of **Formula I** is fully complementary to the target pre-mRNA sequence, or partially complementary to the target pre-mRNA sequence with one or two mismatches;
S₁, S₂, ..., Sₙ₋₁, Sn, T₁, T₂, ..., Tn-i, and Tₙ independently represent deuterido, hydrido, substituted or non-substituted alkyl, or substituted or non-substituted aryl radical;
X and Y independently represent hydrido [H], formyl [H-C(=O)-], aminocarbonyl [NH₂-C(=O)-], substituted or non-substituted alkyl, substituted or non-substituted aryl, substituted or non-substituted alkylacyl, substituted or non-substituted arylacyl, substituted or non-substituted alkyloxycarbonyl, substituted or non-substituted aryloxycarbonyl, substituted or non-substituted alkylaminocarbonyl, substituted or non-substituted arylaminocarbonyl, substituted or non-substituted alkylsulfonyl, or substituted or non-substituted arylsulfonyl radical;
Z represents hydroxy, substituted or non-substituted alkyloxy, substituted or non-substituted aryloxy, substituted or non-substituted amino, substituted or non-substituted alkyl, or substituted or non-substituted aryl radical;
B₁, B₂, ..., Bₙ₋₁, and Bₙ are independently selected from natural nucleobases including adenine, thymine, guanine, cytosine and uracil, and unnatural nucleobases; and,
at least four of B₁, B₂, ..., Bₙ₋₁, and Bₙ are independently selected from unnatural nucleobases with a substituted or non-substituted amino radical covalently linked to the nucleobase moiety.

The compound of **Formula I** induces alternative splicing of the human SCN9A pre-mRNA, yields SCN9A mRNA splice variant(s) lacking "exon 4", and is useful to treat pains, or conditions involving Naᵥ1.7 activity.

The description that "n is an integer between 10 and 21" literally states that n is an integer selectable from a group of integers of 11, 12, 13, 14, 15, 16, 17, 18, 19, and 20.

The compound of **Formula I** tightly binds to the 5' splice site of "exon 4" of the human SCN9A pre-mRNA transcribed from the human SCN9A gene of [NCBI Reference Sequence: NC_000002.12]. The 40-mer SCN9A pre-mRNA sequence consisting of a 20-mer from "exon 4" and a 20-mer from "intron 4" reads [(5' → 3') UUUGUCGUCAUUGUUUUUGC-GUAAGUACUUUCAGCUUUUU], although the exon and intron number may vary depending on SCN9A mRNA transcripts. Provision of the 40-mer pre-mRNA sequence is to unequivocally define the target 5' splice within the human SCN9A pre-mRNA.

The 40-mer pre-mRNA sequence may be alternatively expressed as [(5' → 3') UUUGUCGUCAUUGUUUUUGC | guaaguacuuucagcuuuuu], wherein the exon and intron sequences are denoted with "capital" and "small" letters, respectively, and the junction between the exon and the intron is marked with " | ". Thus the 14-mer pre-mRNA sequence of [(5' → 3') UUUUUGCGUAAGUA] adopted to describe the compound of **Formula I** in this invention may be alternatively expressed as [(5' → 3') UUUUUGC | guaagua].

The compound of **Formula I** tightly binds to the target 5' splice site of "exon 4" within the human SCN9A pre-mRNA, and interferes with the formation of "splicesome early complex" involving the compound's target exon. Since the compound of this invention sterically inhibits the formation of "splicesome early complex" involving "exon 4", the SCN9A "exon 4" is spliced out or deleted to yield an SCN9A mRNA splice variant or variants lacking "exon 4". Consequently the compound of this invention is said to induce the skipping of the SCN9A "exon 4". The resulting SCN9A mRNA splice variant(s) encodes Naᵥ1.7 protein(s) lacking the Naᵥ1.7 functional activity (i.e., sodium ion channel activity) expressed by the full-length Naᵥ1.7 protein.

The compound of **Formula I** tightly binds to the complementary DNA as exemplified in the prior art [PCT/KR2009/001256 (published as WO 2009/113828)]. The duplex between the PNA derivative of **Formula I** and its full-length complementary DNA or RNA shows a Tₘ value too high to be reliably determined in aqueous buffer. The buffer solution tends to boil off during a Tₘ measurement. The PNA compound of **Formula I** still yields high Tₘ values with complementary DNAs of shorter length, for example, 10-mer.

Owing to the high binding affinity, the PNA derivative of this invention potently induces the skipping of SCN9A "exon 4" in cells even with a complementary overlap of as small as 11-mer with the 5' splice site of "exon 4".

The compound of **Formula I** possesses a very strong affinity for the target SCN9A pre-mRNA sequence with full complementarity. Even in case the compound of **Formula I** has one or two mismatches with the target SCN9A pre-mRNA sequence, the PNA compound may still tightly bind to the target pre-mRNA sequence and interrupts the splicing process since the affinity between the compound of **Formula I** and the target SCN9A pre-mRNA sequence is strong enough despite the mismatch(es). For example, a 14-mer PNA derivative of **Formula I** possesses only a 12-mer complementary overlap with the 14-mer SCN9A pre-mRNA sequence of [(5' → 3') UUUUUGC | guaagua] in this invention, and induces the skipping of the SCN9A "exon 4" despite the two mismatches with the 14-mer sequence. Nevertheless, it would not be desired to have too many mismatches with the target pre-mRNA sequence in order to avoid a cross reactivity with pre-mRNA sequences from other gene(s).

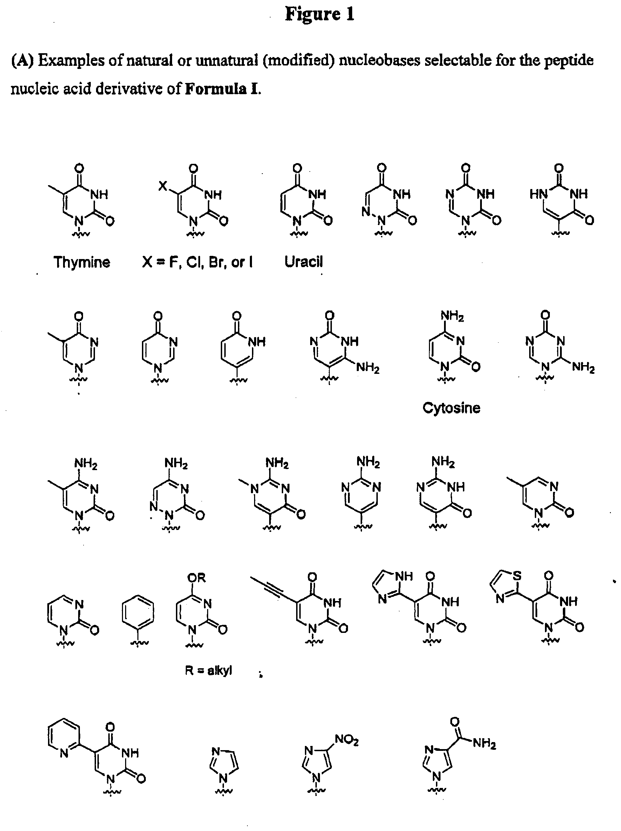

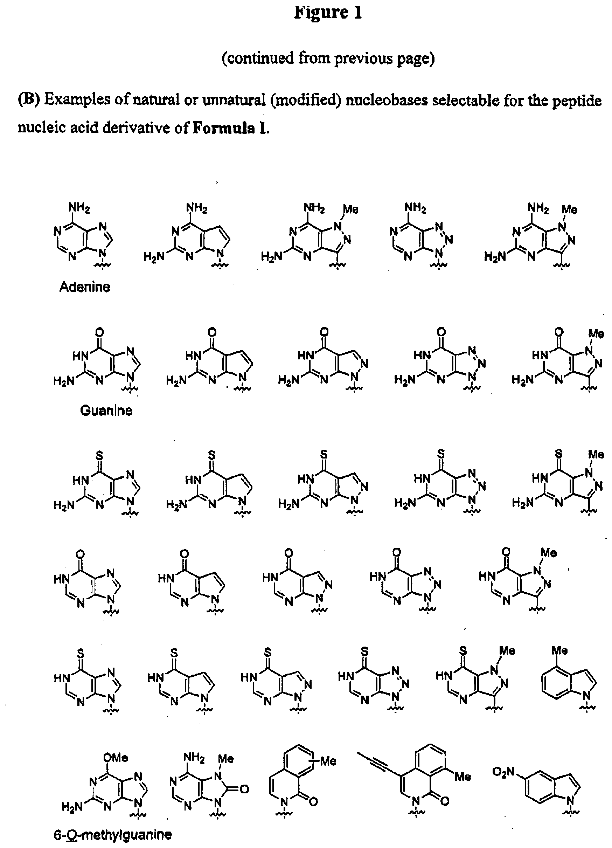

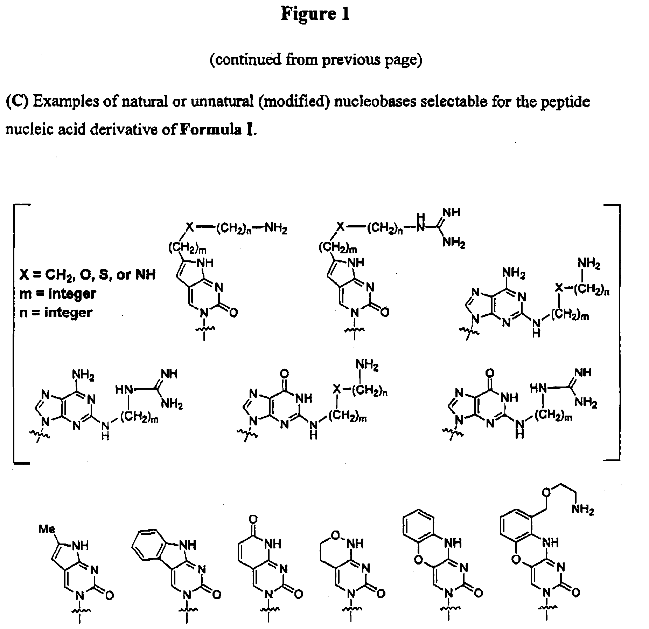

The chemical structures of natural or unnatural nucleobases in the PNA derivative of **Formula I** are exemplified in Figures 1(A)-(C). Natural (conventionally expressed as "naturally occurring") or unnatural (conventionally expressed as "naturally non-occurring") nucleobases of this invention comprise but are not limited to the nucleobases provided in Figures 1(A)-(C). Provision of such unnatural nucleobases is to illustrate the diversity of nucleobases allowable for the compound of **Formula I,** and therefore should not be interpreted to limit the scope of the present invention. A skilled person in the field may easily figure out that variations of unnatural nucleobases are possible for specific positions within the PNA compound of **Formula I** as long as such variations meet the conditions of complementarity with the target pre-mRNA sequence.

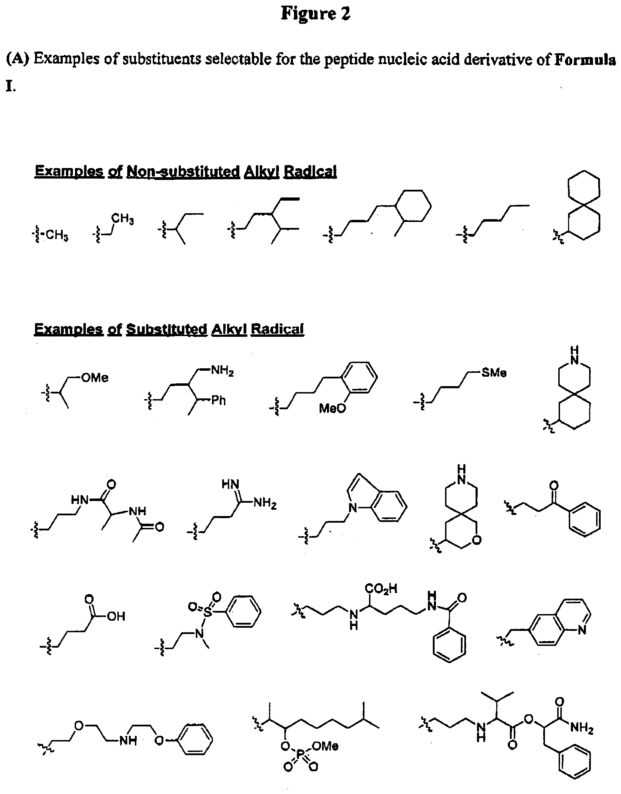

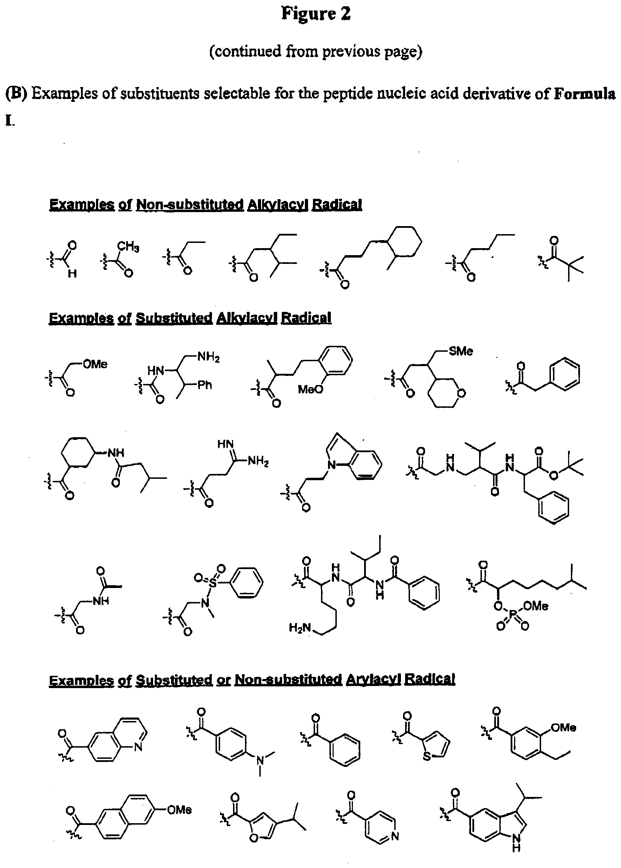

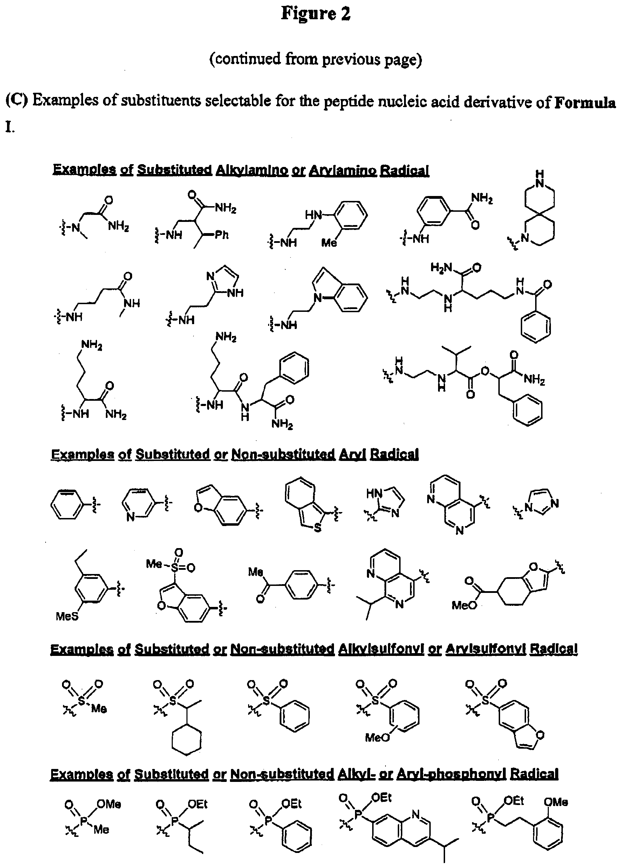

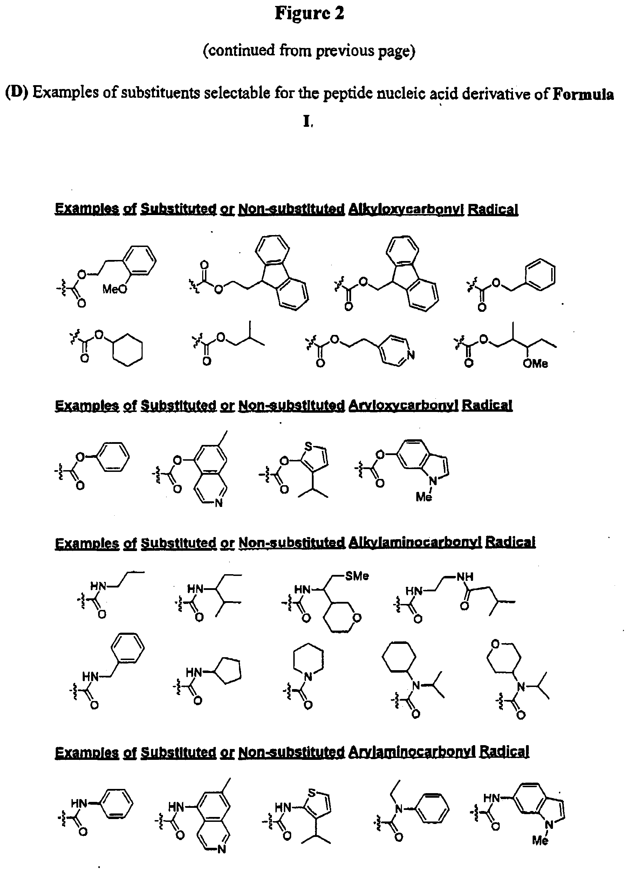

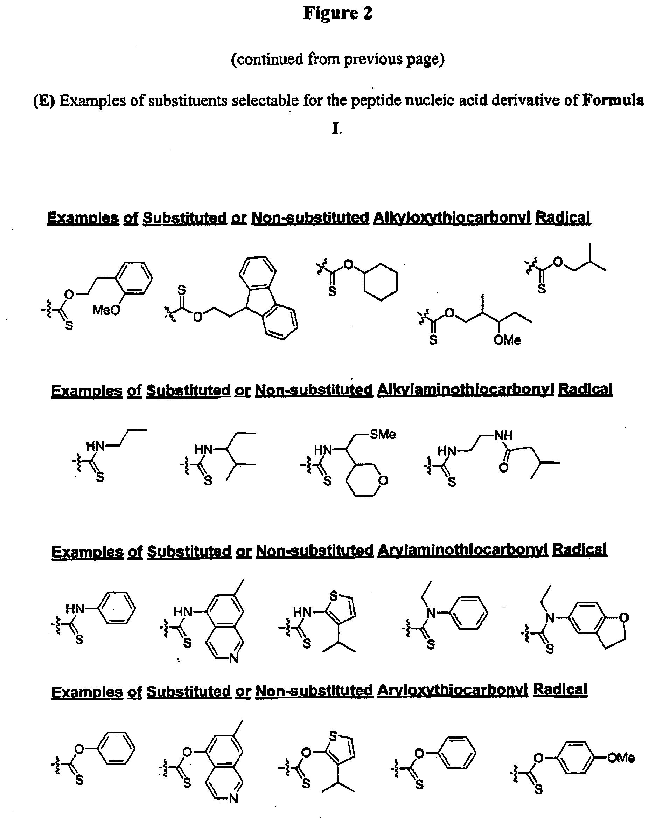

The substituents adopted to describe the PNA derivative of **Formula I** are exemplified in Figures 2(A)-(E). Figure 2(A) provides examples for substituted or non-substituted alkyl radicals. Substituted or non-substituted alkylacyl and substituted or non-substituted alkylacyl arylacyl radicals are exemplified in Figure 2(B). Figure 2(C) illustrates examples for substituted or non-substituted alkylamino, substituted or non-substituted arylamino, substituted or non-substituted aryl, substituted or non-substituted alkylsulfonyl or arylsulfonyl, and substituted or non-substituted alkylphosphonyl or arylphosphonyl radicals. Figure 2(D) provides examples for substituted or non-substituted alkyloxycarbonyl or aryloxycarbonyl, substituted or non-substituted alkyl aminocarbonyl or arylaminocarbonyl radicals. In Figure 2(E) are provided examples for substituted or non-substituted alkylaminothiocarbonyl, substituted or non-substituted arylaminothiocarbonyl, substituted or non-substituted alkyloxythiocarbonyl, and substituted or non-substituted aryloxythiocarbonyl radicals. Provision of such exemplary substituents is to illustrate the diversity of substituents allowable for the compound of **Formula I,** and therefore should not be interpreted to limit the scope of the present invention. A skilled person in the field may easily figure out that the PNA oligonucleotide sequence is the overriding factor for the sequence specific binding of a PNA oligonucleotide to the target pre-mRNA sequence over substituents in the N-terminus or C-terminus.

The PNA compound of **Formula I** possesses good cell permeability and can be readily delivered into cell if treated as "naked" oligonucleotide as exemplified in the prior art [PCT/KR2009/001256]. Thus the compound of this invention induces the skipping of "exon 4" in the SCN9A pre-mRNA to yield SCN9A mRNA splice variant(s) lacking SCN9A "exon 4" in cells treated with the compound of **Formula I** as "naked" oligonucleotide. Cells treated with the compound of **Formula I** as "naked oligonucleotide" express a lower level of the full length SCN9A mRNA, and therefore show a lower Naᵥ1.7 functional activity than cells without the compound treatment.

The compound of **Formula I** does not require an invasive formulation to increase systemic delivery to target tissue for the intended therapeutic or biological activity. Usually the compound of **Formula I** is dissolved in PBS (phosphate buffered saline) or saline, and systemically administered to elicit the desired therapeutic (i.e. analgesic) or biological activity in target cells (mostly neuronal cells). The compound of this invention does not need to be heavily or invasively formulated to elicit the systemic therapeutic activity.

The compound of **Formula I** inhibits Naᵥ1.7 expression in neuronal cells or tissues upon systemic administration as "naked oligonucleotide". Thus the compound is useful to safely treat pains, or disorders involving excessive expression of Naᵥ1.7.

The PNA derivative of **Formula I** may be used as combined with a pharmaceutically acceptable acid or base including but not limited to sodium hydroxide, potassium hydroxide, hydrochloric acid, methanesulfonic acid, citric acid, trifluoroacetic acid, and so on.

The PNA compound of **Formula I** or a pharmaceutically acceptable salt thereof can be administered to a subject in combination with a pharmaceutically acceptable adjuvant including but not limited to citric acid, hydrochloric acid, tartaric acid, stearic acid, polyethyleneglycol, polypropyleneglycol, ethanol, isopropanol, sodium bicarbonate, distilled water, preservative(s), and so on.

The compound of the present invention can be systemically administered to a subject at a therapeutically or biologically effective dose of 1 nmole/Kg or less, which would vary depending on the dosing schedule, conditions or situations of the subject, and so on.

Preferred is a PNA derivative of **Formula I,** or a pharmaceutically acceptable salt thereof:
wherein,
   n is an integer between 10 and 21;
   the compound of **Formula I** possesses at least a 10-mer complementary overlap with the 14-mer RNA sequence of [(5' → 3') UUUUUGCGUAAGUA] within the human SCN9A pre-mRNA;
   the compound of **Formula I** is fully complementary to the target pre-mRNA sequence, or partially complementary to the target pre-mRNA sequence with one or two mismatches;
   S₁, S₂, ..., Sₙ₋₁, Sn, T₁, T₂, ..., Tn-i, and Tₙ independently represent hydrido radical;
   X and Y independently represent hydrido [H], formyl [H-C(=O)-], aminocarbonyl [NH₂-C(=O)-], substituted or non-substituted alkyl, substituted or non-substituted aryl, substituted or non-substituted alkylacyl, substituted or non-substituted arylacyl, substituted or non-substituted alkyloxycarbonyl, substituted or non-substituted aryloxycarbonyl, substituted or non-substituted alkylaminocarbonyl, substituted or non-substituted arylaminocarbonyl, substituted or non-substituted alkylsulfonyl, or substituted or non-substituted arylsulfonyl radical;
   Z represents hydroxy, substituted or non-substituted alkyloxy, substituted or non-substituted aryloxy, substituted or non-substituted amino, substituted or non-substituted alkyl, or substituted or non-substituted aryl radical;
   B₁, B₂, ..., Bₙ₋₁, and Bₙ are independently selected from natural nucleobases including adenine, thymine, guanine, cytosine and uracil, and unnatural nucleobases;
   at least three of B₁, B₂, ..., Bₙ₋₁, and Bₙ are independently selected from unnatural nucleobases represented by **Formula II, Formula III,** or **Formula IV:** 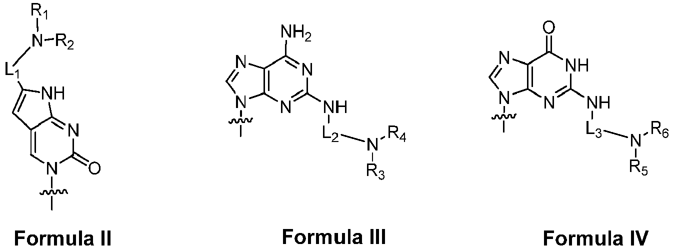
wherein,
   R₁, R₂, R₃, R₄, R₅ and R₆ are independently selected from hydrido, and substituted or non-substituted alkyl radical; and,
   Li, L₂ and L₃ are a covalent linker represented by **Formula V** connecting the basic amino group to the nucleobase moiety responsible for nucleobase pairing: 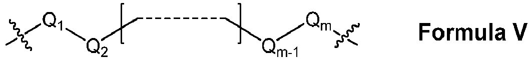
wherein,
   Qi and Qₘ are substituted or non-substituted methylene (-CH₂-) radical, and Qₘ is directly linked to the basic amino group;
   Q₂, Q₃, ..., and Qₘ₋₁ are independently selected from substituted or non-substituted methylene, oxygen (-O-), sulfur (-S-), and substituted or non-substituted amino radical [-N(H)-, or -N(substituent)-]; and,
   m is an integer between 1 and 16.

Of interest is a PNA oligomer of **Formula I,** or a pharmaceutically acceptable salt thereof:
wherein,
n is an integer between 12 and 20;
the compound of **Formula I** possesses at least a 10-mer complementary overlap with the 14-mer RNA sequence of [(5' → 3') UUUUUGCGUAAGUA] within the human SCN9A pre-mRNA;
the compound of **Formula I** is fully complementary to the target pre-mRNA sequence, or partially complementary to the target pre-mRNA sequence with one or two mismatches;
S₁, S₂, ..., Sₙ₋₁, Sn, T₁, T₂, ..., Tn-i, and Tₙ are hydrido radical;
X and Y independently represent hydrido [H], aminocarbonyl [NH₂-C(=O)-], substituted or non-substituted alkyl, substituted or non-substituted aryl, substituted or non-substituted alkylacyl, substituted or non-substituted arylacyl, substituted or non-substituted alkyloxycarbonyl, substituted or non-substituted alkylaminocarbonyl, or substituted or non-substituted arylsulfonyl radical;
Z represents substituted or non-substituted amino radical;
B₁, B₂, ..., Bₙ₋₁, and Bₙ are independently selected from natural nucleobases including adenine, thymine, guanine and cytosine, and unnatural nucleobases;
at least four of B₁, B₂, ..., Bₙ₋₁, and Bₙ are independently selected from unnatural nucleobases represented by **Formula II, Formula III,** or **Formula IV;**
R₁, R₂, R₃, R₄, R₅ and R₆ are independently selected from hydrido, and substituted or non-substituted alkyl radical;
Qi and Qₘ are substituted or non-substituted methylene radical, and Qₘ is directly linked to the basic amino group;
Q₂, Q₃, ..., and Qₘ₋₁ are independently selected from substituted or non-substituted methylene, oxygen, and amino radical; and,
m is an integer between 1 and 11.

Of particular interest is a PNA derivative of **Formula I,** or a pharmaceutically acceptable salt thereof:
wherein,
n is an integer between 12 and 19;
the compound of **Formula I** possesses at least a 10-mer complementary overlap with the 14-mer RNA sequence of [(5' → 3') UUUUUGCGUAAGUA] within the human SCN9A pre-mRNA;
the compound of **Formula I** is fully complementary to the target pre-mRNA sequence;
S₁, S₂, ..., Sₙ₋₁, Sn, T₁, T₂, ..., Tn-i, and Tₙ are hydrido radical;
X and Y independently represent hydrido [H], substituted or non-substituted alkylacyl, substituted or non-substituted arylacyl, substituted or non-substituted alkyloxycarbonyl, or substituted or non-substituted alkylaminocarbonyl radical;
Z represents substituted or non-substituted amino radical;
B₁, B₂, ..., Bₙ₋₁, and Bₙ are independently selected from natural nucleobases including adenine, thymine, guanine and cytosine, and unnatural nucleobases;
at least four of B₁, B₂, ..., Bₙ₋₁, and Bₙ are independently selected from unnatural nucleobases represented by **Formula II, Formula III,** or **Formula IV;**
R₁, R₂, R₃, R₄, R₅ and R₆ are independently selected from hydrido, and substituted or non-substituted alkyl radical;
Qi and Qₘ are methylene radical, and Qₘ is directly linked to the basic amino group;
Q₂, Q₃, ..., and Qₘ₋₁ are independently selected from methylene, and oxygen radical; and,
m is an integer between 1 and 10.

Of high interest is a PNA oligomer of **Formula I,** or a pharmaceutically acceptable salt thereof:
wherein,
n is an integer between 12 and 18;
the compound of **Formula** I possesses at least a 10-mer complementary overlap with the 14-mer RNA sequence of [(5' → 3') UUUUUGCGUAAGUA] within the human SCN9A pre-mRNA;
the compound of **Formula I** is fully complementary to the target pre-mRNA sequence;
S₁, S₂, ..., Sₙ₋₁, Sn, T₁, T₂, ..., Tn-i, and Tₙ are hydrido radical;
X and Y independently represent hydrido [H], substituted or non-substituted alkylacyl, substituted or non-substituted arylacyl, or substituted or non-substituted alkyloxycarbonyl radical;
Z represents substituted or non-substituted amino radical;
B₁, B₂, ..., Bₙ₋₁, and Bₙ are independently selected from natural nucleobases including adenine, thymine, guanine and cytosine, and unnatural nucleobases;
at least five of B₁, B₂, ..., Bₙ₋₁, and Bₙ are independently selected from unnatural nucleobases represented by **Formula II, Formula III,** or **Formula IV;**
R₁, R₃, and R₅ are hydrido radical, and R₂, R₄, and R₆ independently represent hydrido, or substituted or non-substituted alkyl radical;
Qi and Qₘ are methylene radical, and Qₘ is directly linked to the basic amino group;
Q₂, Q₃, ..., and Qₘ₋₁ are independently selected from methylene, and oxygen radical; and,
m is an integer between 1 and 10.

Of higher interest is a PNA derivative of **Formula I,** or a pharmaceutically acceptable salt thereof:
wherein,
n is an integer between 12 and 16;
the compound of **Formula I** possesses at least a 10-mer complementary overlap with the 14-mer RNA sequence of [(5' → 3') UUUUUGCGUAAGUA] within the human SCN9A pre-mRNA;
the compound of **Formula I** is fully complementary to the target pre-mRNA sequence;
S₁, S₂, ..., Sₙ₋₁, Sn, T₁, T₂, ..., Tn-i, and Tₙ are hydrido radical;
X and Y independently represent hydrido [H], substituted or non-substituted alkylacyl, substituted or non-substituted arylacyl, or substituted or non-substituted alkyloxycarbonyl radical;
Z represents substituted or non-substituted amino radical;
B₁, B₂, ..., Bₙ₋₁, and Bₙ are independently selected from natural nucleobases including adenine, thymine, guanine and cytosine, and unnatural nucleobases;
at least five of B₁, B₂, ..., Bₙ₋₁, and Bₙ are independently selected from unnatural nucleobases represented by **Formula II, Formula III,** or **Formula IV;**
R₁, R₂ , R₃, R₄, R₅, and R₆ are hydrido radical;
Qi and Qₘ are methylene radical, and Qₘ is directly linked to the basic amino group;
Q₂, Q₃, ..., and Qₘ₋₁ are independently selected from methylene, and oxygen radical; and,
m is an integer between 1 and 10.

Of highest interest is a PNA derivative of **Formula I,** or a pharmaceutically acceptable salt thereof:
wherein,
n is an integer between 12 and 16;
the compound of **Formula I** possesses at least a 10-mer complementary overlap with the 14-mer RNA sequence of [(5' → 3') UUUUUGCGUAAGUA] within the human SCN9A pre-mRNA;
the compound of **Formula I** is fully complementary to the target pre-mRNA sequence;
S₁, S₂, ..., Sₙ₋₁, Sn, T₁, T₂, ..., Tn-i, and Tₙ are hydrido radical;
X is hydrido radical;
Y represents substituted or non-substituted alkylacyl, substituted or non-substituted arylacyl, or substituted or non-substituted alkyloxycarbonyl radical;
Z represents substituted or non-substituted amino radical;
B₁, B₂, ..., Bₙ₋₁, and Bₙ are independently selected from natural nucleobases including adenine, thymine, guanine and cytosine, and unnatural nucleobases;
at least five of B₁, B₂, ..., Bₙ₋₁, and Bₙ are independently selected from unnatural nucleobases represented by **Formula II, Formula III,** or **Formula IV;**
R₁, R₂ , R₃, R₄, R₅, and R₆ are hydrido radical;
L₁ represents -(CH₂)₂-O-(CH₂)₂-, -CH₂-O-(CH₂)₂-, -CH₂-O-(CH₂)₃-, -CH₂-O-(CH₂)₄-, or -CH₂-O-(CH₂)₅- with the right end being directly linked to the basic amino group; and,
L₂ and L₃ are independently selected from -(CH₂)₂-O-(CH₂)₂-, -(CH₂)₃-O-(CH₂)₂-, - (CH₂)₂-O-(CH₂)₃-, -(CH₂)₂-, -(CH₂)₃-, -(CH₂)₄-, -(CH₂)₅-, -(CH₂)₆-, -(CH₂)₇-, and -(CH₂)₈-with the right end being directly linked to the basic amino group.

Of specific interest is a PNA derivative of **Formula I** which is selected from the group of compounds provided below, or a pharmaceutically acceptable salt thereof:
(N → C) Fmoc-TA(5)A-A(5)TA(5)-CGC(1O2)-AA(5)A-A(5)A-NH₂;
(N → C) Fethoc-TA(5)A-A(5)TA(5)-CGC(1O2)-AA(5)A-A(5)A-NH₂;
(N → C) Piv-TA(5)A-A(5)TA(5)-CGC(1O2)-AA(5)A-A(5)A-NH₂;
(N → C) FAM-HEX-TA(5)A-A(5)TA(5)-CGC(1O2)-AA(5)A-A(5)A-NH₂;
(N → C) Acetyl-TA(5)A-A(5)TA(5)-CGC(1O2)-AA(5)A-A(5)A-NH₂;
(N → C) Fethoc-Lys-TA(5)A-A(5)TA(5)-CGC(1O2)-AA(5)A-A(5)A-NH₂;
(N → C) H-TA(5)A-A(5)TA(5)-CGC(1O2)-AA(5)A-A(5)A-NH₂;
(N → C) Me-TA(5)A-A(5)TA(5)-CGC(1O2)-AA(5)A-A(5)A-NH₂;
(N → C) Benzyl-TA(5)A-A(5)TA(5)-CGC(1O2)-AA(5)A-A(5)A-NH₂;
(N → C) Fethoc-TA(5)A-A(5)TA(5)-CGC(1O2)-AA(5)A-A(5)A-Lys-NH₂;
(N → C) Fmoc-TA(5)A-A(5)TA(5)-CGC(1O2)-AA(5)A-A(5)AC-A(5)A-NH₂;
(N → C) Fethoc-TA(5)C-GC(1O2)A-A(5)AA(5)-ACA(5)-A-NH₂;
(N → C) Fethoc-TA(6)C-GC(1O2)A-A(6)AA(6)-ACA(6)-A-NH₂;
(N → C) Fethoc-AC(1O2)T-TA(5)C-G(6)CA-A(5)AA(5)-AC(102)A-A(5)-NH₂;
(N → C) Fethoc-AG(5)T-A(5)CT-TA(5)C-GC(1O2)A-A(5)AA(5)-ACA(5)-A-NH₂;
(N → C) Fethoc-AG(5)T-A(5)CT-TA(5)C-GC(1O2)A-A(5)AA(5)-A-NH₂;
(N → C) Fethoc-AG(5)T-A(5)CT-TA(5)C-GC(1O2)A-A(5)AA(2O2)-A-NH₂;
(N → C) Fethoc-Val-AG(5)T-A(5)CT-TA(5)C-GC(1O2)A-A(5)AA(2O2)-A-NH₂;
(N → C) Fethoc-Gly-AG(5)T-A(5)CT-TA(5)C-GC(1O2)A-A(5)AA(2O2)-A-NH₂;
(N → C) Fethoc-AG(5)T-A(5)CT-TA(5)C-GC(1O2)A-A(5)AA(2O2)-A-Lys-NH₂;
(N → C) Piv-AG(5)T-A(5)CT-TA(5)C-GC(1O2)A-A(5)AA(5)-A-NH₂;
(N → C) Fethoc-Lys-AG(5)T-A(5)CT-TA(5)C-GC(1O2)A-A(5)A-NH₂;
(N → C) Piv-Leu-AG(5)T-A(5)CT-TA(5)C-GC(1O2)A-A(5)AA(2O2)-A-NH₂;
(N → C) Fethoc-A(5)GT-A(5)CT-TA(5)C-G(6)CA(5)-A-NH₂;
(N → C) Fethoc-Lys-A(5)TC(1O3)-A(5)CT-TA(5)C-GC(1O2)A-A(5)A-NH₂;
(N → C) Fethoc-Gly-A(5)TC(1O3)-A(5)CT-TA(5)C-GC(1O2)A-A(5)A-Arg-NH₂;
(N → C) H-CTT-A(5)CG(3)-C(1O2)AA(5)-AA(5)A-C(1O3)AA(5)-NH₂;
(N → C) Fethoc-CTT-A(5)CG(6)-C(1O2)AA(5)-AA(5)A-C(1O2)AA(5)-NH₂;
(N → C) Fethoc-CTT-A(5)CG(6)-C(1O2)TA(5)-AA(5)T-C(1O2)AA(5)-NH₂;
(N → C) Benzoyl-CTT-A(5)CG(2O2)-C(1O2)AA(5)-AA(5)A-C(1O5)AA(5)-NH₂;
(N → C) n-Propyl-CTT-A(5)CG(2O3)-C(1O2)AA(3)-AA(5)A-C(2O2)AA(5)-NH₂;
(N → C) p-Toluenesulfonyl-CTT-A(5)CG(6)-C(1O2)AA(8)-AA(5)A-C(1O2)AA(5)-NH₂;
(N → C) [N-(2-Phenylethyl)amino]carbonyl-CTT-A(5)CG(6)-C(1O2)AA(2O2)-AA(5)A-C(1O2)A A(5)-NH₂;
(N → C) Fethoc-Lys-Leu-CTT-A(5)CG(6)-C(1O2)AA(4)-AA(5)A-C(1O2)AA(5)-Lys-NH₂;
(N → C) N-Phenyl-N-Me-CTT-A(5)CG(6)-C(1O2)AA(5)-AA(5)A-C(1O2)AA(5)-Lys-NH₂;
(N → C) Fethoc-AA(5)G-TA(5)C-TTA(5)-CG(6)C-A(5)A-NH₂; and,
(N → C) Fethoc-AA(5)G-TA(5)C-TTA(5)-CG(6)C-A(5)A-Lys-NH₂:

wherein,
   A, G, T, and C are PNA monomers with a natural nucleobase of adenine, guanine, thymine, and cytosine, respectively;
   C(pOq), A(p), A(pOq), G(p), and G(pOq) are PNA monomers with an unnatural nucleobase represented by **Formula VI, Formula VII, Formula VIII, Formula IX,** and **Formula X,** respectively; 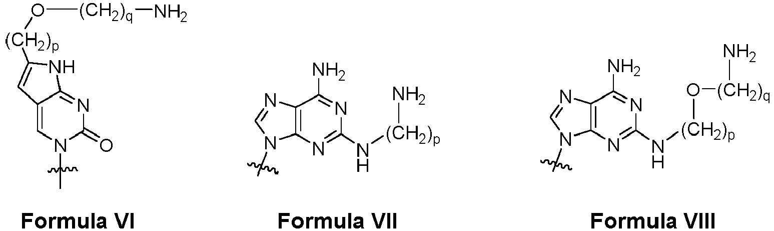 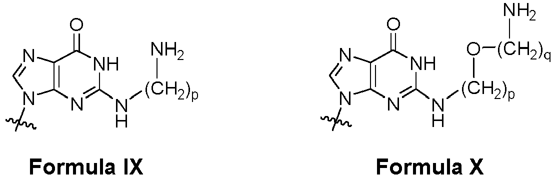
wherein,
   p and q are integers; and,
   the abbreviations for the N- and C-terminus substituents are as specifically described as follows: "Fmoc-" is the abbreviation for "[(9-fluorenyl)methyloxy]carbonyl-"; "Fethoc-" for "[2-(9-fluorenyl)ethyl-1-oxy]carbonyl"; "Ac-" for "acetyl-"; "Benzoyl-" for "benzenecabonyl-"; "Piv-" for "pivalyl-"; "Me-" for "methyl-"; "n-Propyl-" for "1-(n-propyl)-"; "H-" for "hydrido-" group; "p-Toluenesulfonyl" for "(4-methylbenzene)-1-sulfonyl-"; "-Lys-" for amino acid residue "lysine"; "-Val-" for amino acid residue "valine"; "-Leu-" for amino acid residue "leucine"; "-Arg-" for amino acid residue "arginine"; "-Gly-" for amino acid residue "glycine"; "[N-(2-Phenylethyl)amino]carbonyl-" for "[N-1-(2-phenylethyl)amino]carbonyl-"; "Benzyl-" for "1-(phenyl)methyl-"; "Phenyl-" for "phenyl-"; "Me-" for "methyl-"; "-HEX-" for "6-amino-1-hexanoyl-", "FAM-" for "5, or 6-fluorescein-carbonyl- (isomeric mixture)" ,and "-NH₂" for non-subsituted "-amino" group.

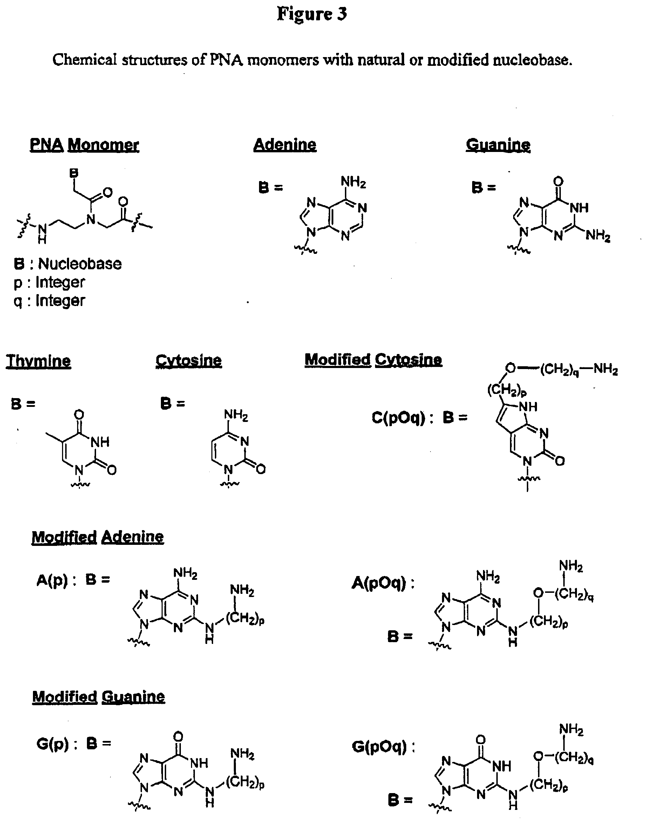

Figure 3 collectively provides the chemical structures for the PNA monomers abbreviated as A, G, T, C, C(pOq), A(p), A(pOq), G(p), and G(pOq). As discussed in the prior art [PCT/KR2009/001256], C(pOq) is regarded as a modified PNA monomer corresponding to "cytosine" due to its preferred hybridization to "guanine". A(p) and A(pOq) are taken as modified PNA monomers acting as "adenine" for their tight affinity for "thymine". Likewise G(p) and G(pOq) are considered to be modified PNA monomers equivalent to "guanine" owing to their productive base pairing with "cytosine".

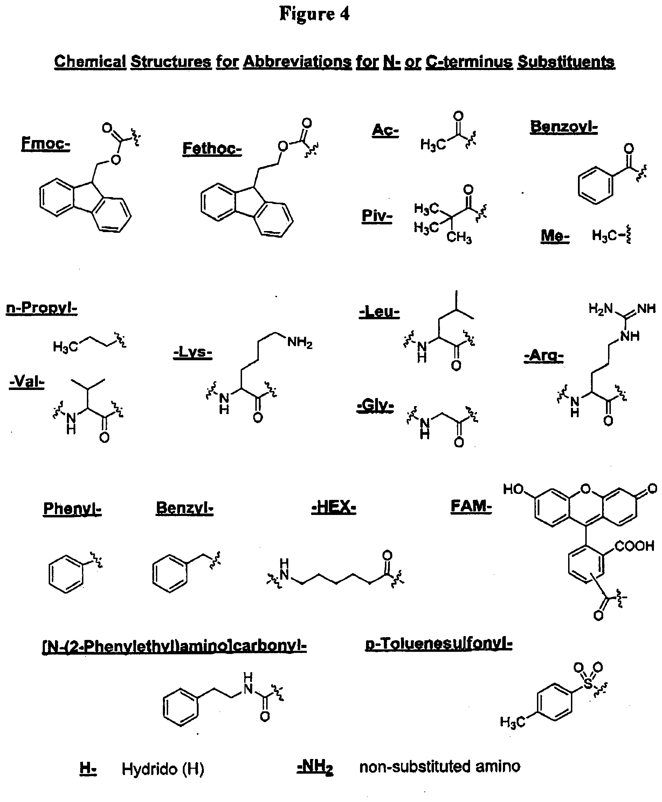

Figure 4 unequivocally illustrates the chemical structures for a variety of abbreviations for substituents used for diversifying the N-terminus or C-terminus of the PNA derivative of **Formula I** in this invention.

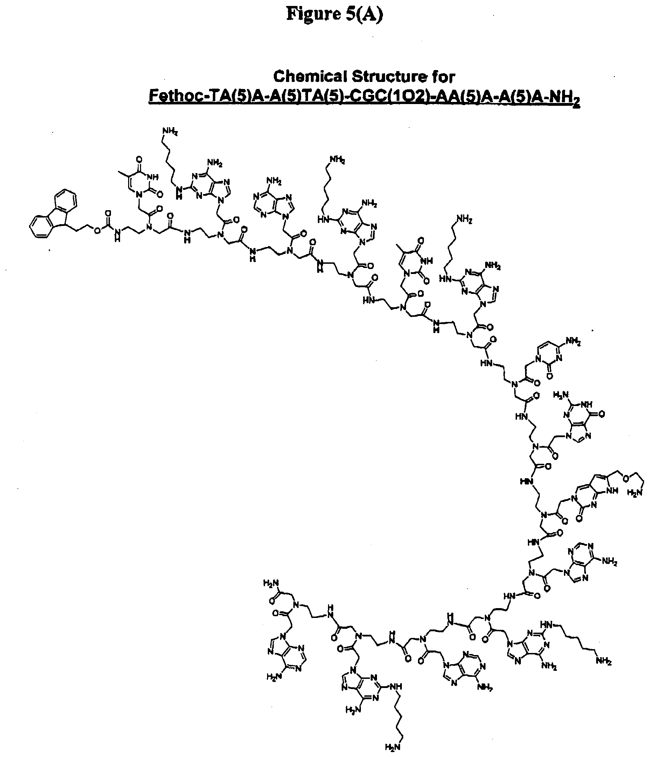

In order to illustrate the abbreviations for the PNA derivatives in this invention, the chemical structure for a 14-mer PNA derivative abbreviated as "(N → C) Fethoc-TA(5)A-A(5)TA(5)-CGC(1O2)-AA(5)A-A(5)A-NH₂" is provided in Figure 5(A). The 14-mer PNA sequence is equivalent to the DNA sequence of "(5' → 3') TAA-ATA-CGC-AAA-AA" for complementary binding to the SCN9A pre-mRNA. The 14-mer PNA possesses a 12-mer complementary overlap within a 20-mer sequence of [(5' → 3') UUGUUUUUGC | guaaguacuu] spanning the 5' splice site involving "exon 4" within the human SCN9A pre-mRNA with the complementary base pairings marked "bold" and "underlined" as in [(5' → 3') UUG**UUUUUGC** | **gua**"aa"**ua**cuu] along with the two mismatches in "intron 4" marked with a quote notation (" "). Despite the two mismatches in "intron 5", the 14-mer PNA meets the complementary overlap criteria for the compound of **Formula I** in this invention, i.e. the criteria provided below:
"the compound of **Formula I** possesses at least a 10-mer complementary overlap with the 14-mer RNA sequence of [(5' → 3') UUUUUGCGUAAGUA] within the human SCN9A pre-mRNA, and the compound of **Formula I** is fully complementary to the target pre-mRNA sequence, or partially complementary to the target pre-mRNA sequence with one or two mismatches."

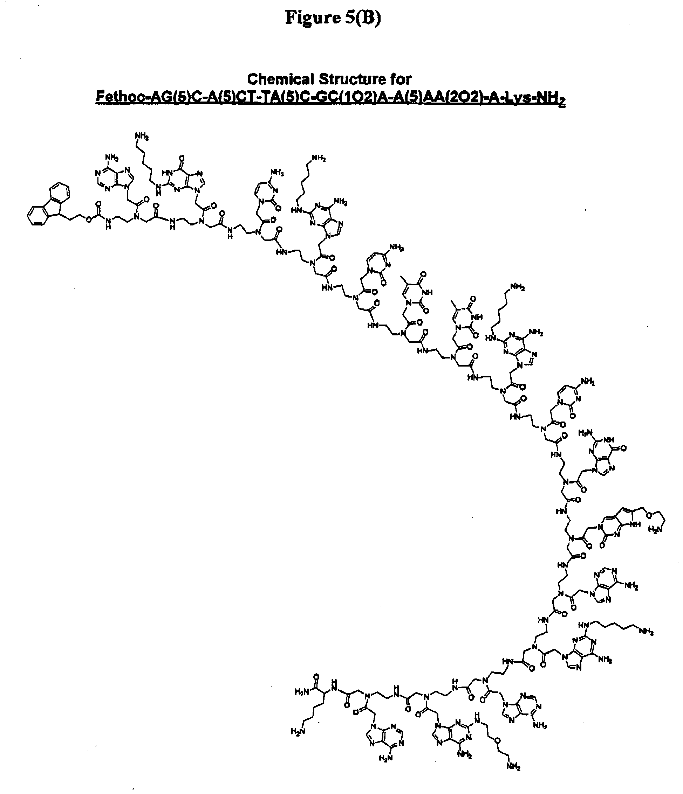

As another illustration, the chemical structure for a 16-mer PNA derivative abbreviated as "(N → C) Fethoc-AG(5)C-A(5)CT-TA(5)C-GC(1O2)A-A(5)AA(2O2)-A-Lys-NH₂" is provided in Figure 5(B). The 16-mer PNA sequence is equivalent to the DNA sequence of "(5' → 3') AGC-ACT-TAC-GCA-AAA-A" for complementary binding to the SCN9A pre-mRNA. The 16-mer PNA has a 15-mer complementary overlap with the 20-mer pre-mRNA sequence of [(5' → 3') UUGUUUUUGC | guaaguacuu] within the human SCN9A pre-mRNA with the compelementary base pairings marked "bold" and "underlined" in [(5' → 3') UUG**UUUUUGC** | **guaagu**"a"**cu**u] along with the single mismatch in "intron 4" marked with a quote notation (" "). This 16-mer PNA meets the complementary overlap criteria for the compound of **Formula** I in this invention despite the single mismatch in "intron 5".

A 16-mer PNA sequence of "(N → C) Fethoc-AC(1O2)T-TA(5)C-G(6)CA-A(5)AA(5)-AC(1O2)A-A(5)-NH₂" is equivalent to the DNA sequence of "(5' → 3') ACT-TAC-GCA-AAA-ACA-A" for complementary binding to the SCN9A pre-mRNA. This 16-mer PNA possesses full (i.e. 16-mer) complementary binding to the 20-mer SCN9A pre-mRNA sequence of [(5' → 3') UUGUUUUUGC | guaaguacuu] with the complementary base pairings as marked "bold" and "underlined" in [(5' → 3') **UUGUUUUUGC** | **guaagu**acuu]. This 16-mer PNA meets the complementary overlap criteria for the compound of **Formula I** in this invention.

A 17-mer PNA sequence of "(N → C) Fethoc-TG(6)T-TA(5)A-A(5)TA(5)-CGC(1O2)-AA(5)A-A(5)A-NH₂" is equivalent to the DNA sequence of "(5' → 3') TGT-TAA-ATA-CGC-AAA-AA" for complementary binding to the SCN9A pre-mRNA. This 17-mer PNA has a 12-mer complementary overlap the 20-mer SCN9A pre-mRNA sequence of [(5' → 3') UUGUUUUUGC | guaaguacuu] with the complementary base pairings marked "bold" and "underlined" in [(5' → 3') UUG**UUUUUGC** | **gua**"ag"**ua**"cuu"] along with the five mismatches in "intron 4" as marked with quote notations (" "). This 17-mer PNA doesn't meet the complementary overlap criteria for the compound of **Formula I** in this invention due to the five mismatches in "intron 5", even though this 17-mer PNA possesses a 12-mer complementary overlap like the above-mentioned 14-mer PNA. Having too many mismatches for the oligomer length as with this 17-mer PNA potentially may elicit cross reactivity with pre-mRNA(s) other than the SCN9A pre-mRNA, and therefore needs to be avoided for safety concerns.

### Detailed Description of Invention

### General Procedures for Preparation of PNA Oligomers

PNA oligomers were synthesized by solid phase peptide synthesis (SPPS) based on Fmoc-chemistry according to the method disclosed in the prior art [US6,133,444; WO96/40685] with minor but due modifications. The solid support employed in this study was H-Rink Amide-ChemMatrix purchased from PCAS BioMatrix Inc. (Quebec, Canada). Fmoc-PNA monomers with a modified nucleobase were synthesized as described in the prior art [PCT/KR 2009/001256] or with minor modifications. Such Fmoc-PNA monomers with a modified nucleobase and Fmoc-PNA monomers with a naturally occurring nucleobase were used to synthesize the PNA derivatives of the present invention. PNA oligomers were purified by C₁₈-reverse phase HPLC (watre/acetonitrile or water/methanol with 0.1% TFA) and characterized by mass spectrometry including ESI/TOF/MS.

Scheme 1 illustrates a typical monomer elongation cycle adopted in the SPPS of this invention, and the synthetic details are provided as below. To a skilled person in the field, however, lots of minor variations are obviously possible in effectively running such SPPS reactions on an automatic peptide synthesizer or manual peptide synthesizer. Each reaction step in Scheme 1 is briefly provided as follows.

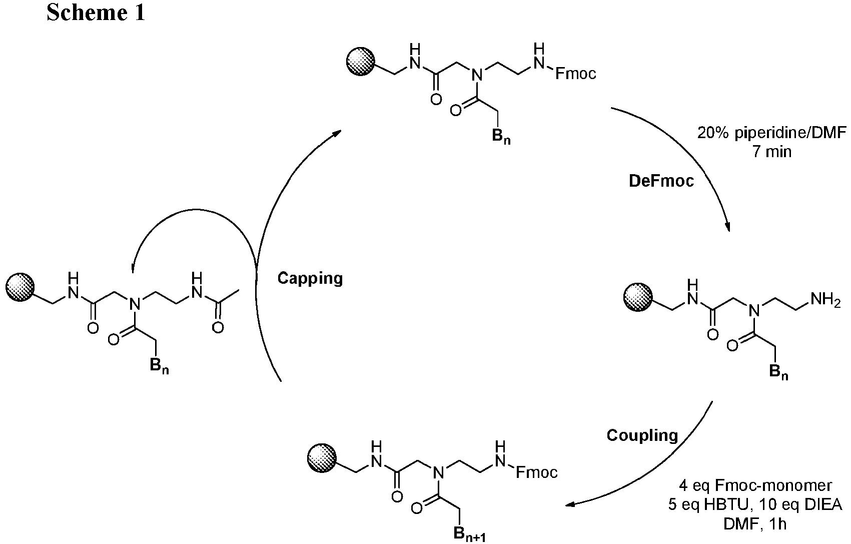

[Activation of H-Rink-ChemMatrix Resin] 0.01 mmol (ca 20 mg resin) of the ChemMatrix resin in 1.5 mL 20% piperidine/DMF was vortexed in a libra tube for 20 min, and the DeFmoc solution was filtered off. The resin was washed for 30 sec each in series with 1.5 mL methylene chloride (MC), 1.5 mL dimethylformamide (DMF), 1.5 mL MC, 1.5 mL DMF, and 1.5 mL MC. The resulting free amines on the solid support were subjected to coupling either with an Fmoc-PNA monomer or with an Fmoc-protected amino acid derivative.

[DeFmoc] The resin was vortexed in 1.5 mL 20% piperidine/DMF for 7 min, and the DeFmoc solution was filtered off. The resin was washed for 30 sec each in series with 1.5 mL MC, 1.5 mL DMF, 1.5 mL MC, 1.5 mL DMF, and 1.5 mL MC. The resulting free amines on the solid support were immediately subjected to coupling with an Fmoc-PNA monomer.

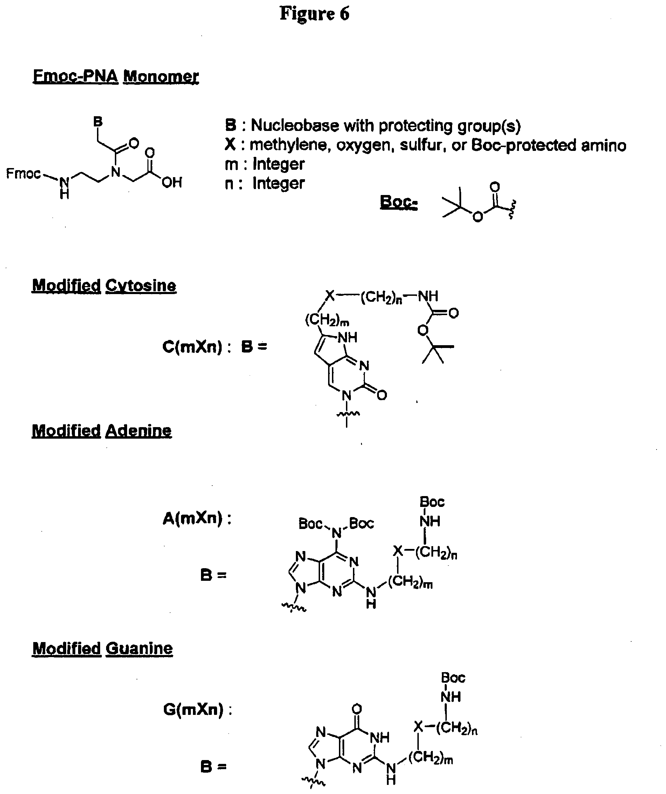

[Coupling with Fmoc-PNA Monomer] The free amines on the solid support were coupled with an Fmoc-PNA monomer as follows. 0.04 mmol of PNA monomer, 0.05 mmol HBTU, and 10 mmol DIEA were incubated for 2 min in 1 mL anhydrous DMF, and added to the resin with free amines. The resin solution was vortexed for 1 hour and the reaction medium was filtered off. Then the resin was washed for 30 sec each in series with 1.5 mL MC, 1.5 mL DMF, and 1.5 mL MC. The chemical structures of Fmoc-PNA monomers with a modified nucleobase used in this invention are provided in Figure 6. The Fmoc-PNA monomers with a modified nucleobase are provided in Figure 6 should be taken as examples, and therefore should not be taken to limit the scope of the present invention. A skilled person in the field may easily figure out a number of variations in Fmoc-PNA monomers to synthesize the PNA derivative of **Formula I.**

[Capping] Following the coupling reaction, the unreacted free amines were capped by shaking for 5 min in 1.5 mL capping solution (5% acetic anhydride and 6% 2,6-leutidine in DMF). Then the capping solution was filtered off and washed for 30 sec each in series with 1.5 mL MC, 1.5 mL DMF, and 1.5 mL MC.

[Introduction of "Fethoc-" Radical in N-Terminus] "Fethoc-" radical was introduced to the N-terminus by reacting the free amine on the resin with "Fethoc-OSu" under basic coupling conditions. The chemical structure of "Fethoc-OSu" [CAS No. 179337-69-0, C₂₀H₁₇NO₅, MW 351.36] is provided as follows.

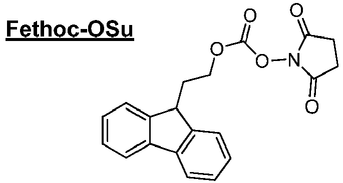

[Cleavage from Resin] PNA oligomers bound to the resin were cleaved from the resin by shaking for 3 hours in 1.5 mL cleavage solution (2.5% tri-isopropylsilane and 2.5% water in trifluoroacetic acid). The resin was filtered off and the filtrate was concentrated under reduced pressure. The resulting residue was triturated with diethylether and the resulting precipitate was collected by filtration for purification by reverse phase HPLC.

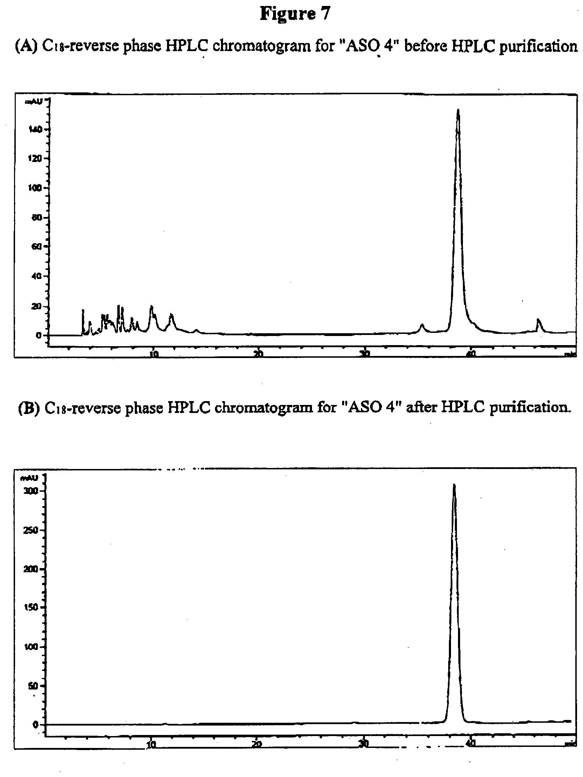

[HPLC Analysis and Purification] Following a cleavage from resin, the crude product of a PNA derivative was purified by C₁₈-reverse phase HPLC eluting water/acetonitrile or water/methanol (gradient method) containing 0.1% TFA. Figures 7(A) and 7(B) are exemplary HPLC chromatograms for "ASO 4" before and after HPLC purification, respectively. The oligomer sequence of "ASO 4" is as provided in Table 1.

### Synthetic Examples for PNA Derivatives of Formula I

PNA derivatives in this invention were prepared according to the synthetic procedures provided above or with minor modifications. Table 1 provides examples of SCN9A ASOs targeting the 5' splice site of the human SCN9A "exon 4" along with structural characterization data by mass spectrometry. Provision of the SCN9A ASOs as in Table 1 is to exemplify the PNA derivative of **Formula I,** and should not be interpreted to limit the scope of the present invention.

**Table 1. SCN9A ASOs targeting the 5' splice site of "exon 4" in the human SCN9A pre-mRNA along with structural characterization data by mass spectrometry.**

| PNA Example | PNA Sequence (N → C) | Exact Mass, m/z | |
|---|---|---|---|
| | | theor.^{a} | obs.^{b} |
| ASO 1 | Fmoc-TA(5)A-A(5)TA(5)-CGC(1O2)-AA(5)A-A(5)A-NH₂ | 4640.19 | 4640.88 |
| ASO 2 | FAM-HEX-TA(5)A-A(5)TA(5)-CGC(1O2)-AA(5)A-A(5)A- NH2 | 4887.24 | 4887.40 |
| ASO 3 | Fmoc-TA(5)A-A(5)TA(5)-CTC(1O2)-AA(5)A-A(5)A-NH₂ | 4613.17 | 4612.51 |
| ASO 4 | Fethoc-TA(5)A-A(5)TA(5)-CGC(1O2)-AA(5)A-A(5)A-NH₂ | 4652.20 | 4652.24 |
| ASO 5 | Fethoc-TG(6)T-TA(5)A-A(5)TA(5)-CGC(1O2)-AA(5)A- A(5)A-NH2 | 5574.61 | 5574.57 |
| ASO 6 | Fethoc-TA(5)A-C(1O2)TA(5)-CGA(5)-AA(5)A-A(5)A-NH₂ | 4652.20 | 4652.24 |
| ASO 7 | Fethoc-TA(5)C-GC(1O2)A-A(5)AA(5)-ACA(5)-A-NH₂ | 4261.98 | 4262.00 |
| ASO 8 | Fethoc-TA(6)C-GC(1O2)A-A(6)AA(6)-ACA(6)-A-NH₂ | 4318.05 | 4318.17 |
| ASO 9 | Fethoc-AC(1O2)T-TA(5)C-G(6)CA-A(5)AA(5)-AC(1O2)A- A(5)-NH2 | 5250.53 | 5250.46 |
| ASO 10 | Fmoc-TA(5)A-A(5)TA(5)-CGC(1O2)-AA(5)A-A(5)AC- A(5)A-NH2 | 5539.61 | 5539.57 |
| AOS 11 | Piv-TA(5)A-A(5)TA(5)-CGC(1O2)-AA(5)A-A(5)A-NH2 | 4500.17 | 4499.79 |
| ASO 12 | FAM-HEX-A(5)TA(5)-CGC(1O2)-AA(5)A-A(5)A-NH₂ | 3970.82 | 3974.17 |
| ASO 13 | Fmoc-TA(6)A-A(5)TA(6)-CGC(1O2)-AA(6)A-AA(6)C- A(6)-NH2 | 5334.57 | 5335.59 |
| ASO 14 | Fethoc-CTT-A(5)CG(6)-C(1O2)AA(5)-AA(5)A- C(1O2)AA(5)-NH2 | 4975.34 | 4975.34 |
| ASO 15 | H-CTT-A(5)CG(3)- C(1O2)AA(5)-AA(5)A-C(1O3)AA(5)- NH2 | 4711.22 | 4711.25 |
| ASO 16 | Benzoyl-CTT-A(5)CG(2O2)-C(1O2)AA(5)-AA(5)A- C(1O5)AA(5)-NH2 | 4873.30 | 4873.32 |
| ASO 17 | n-Propyl-CTT-A(5)CG(2O3)-C(1O2)AA(3)-AA(5)A- C(2O2)AA(5)-NH2 | 4769.27 | 4769.30 |
| ASO 18 | p-Toluenesulfonyl-CTT-A(5)CG(6)-C(1O2)AA(8)-AA(5)A- C(1O2)AA(5)-NH2 | 4935.32 | 4935.29 |
| ASO 19 | [N-(2-Phenylethyl)amino]carbonyl-CTT-A(5)CG(6)- C(1O2)AA(2O2)-AA(5)A-C(1O2)A A(5)-NH2 | 4888.31 | 4888.32 |
| ASO 20 | Fethoc-CTT-A(5)CG(6)-C(1O2)TA(5)-AA(5)T- C(1O2)AA(5)-NH2 | 4957.32 | 4957.32 |
| ASO 21 | Fethoc-Lys-Leu-CTT-A(5)CG(6)-C(1O2)AA(4)-AA(5)A- C(1O2)AA(5)-Lys-NH2 | 5330.60 | 5330.60 |
| ASO 22 | N-Phenyl-N-Me-CTT-A(5)CG(6)-C(1O2)AA(5)-AA(5)A- C(1O2)AA(5)-Lys-NH2 | 4957.40 | 4957.42 |

| | | | |
|---|---|---|---|
| ^{a)} theoretical exact mass, ^{b)} observed exact mass | | | |

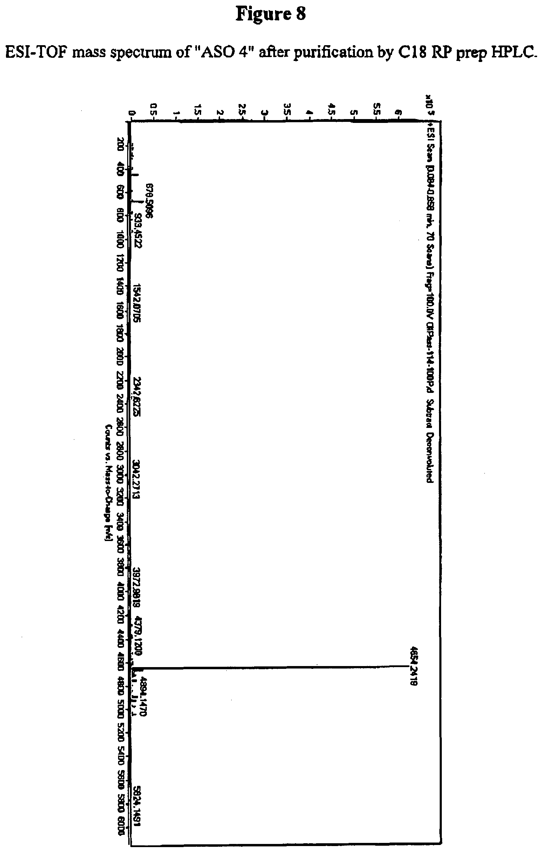

Figure 7(A) is a HPLC chromatogram obtained with a crude product of "ASO 4". The crude product was purified by C₁₈-reverse phase (RP) preparatory HPLC. Figure 7(B) is a HPLC chromatogram for a purified product of "ASO 4". The purity of "ASO 4" improved markedly by the preparatory HPLC purification. Figure 8 provides a ESI-TOF mass spectrum obtained with the purified product of "ASO 4". Provision of the analysis data for "ASO 4" is to illustrate how the PNA derivatives of **Formula I** were purified and identified in the present invention, and should not be interpreted to limit the scope of this invention.

### Binding Affinity with 10-mer Complementary DNA

PNA derivatives in Table 1 were evaluated for their binding affinity for 10-mer DNAs complementarily targeting either the N-terminal or the C-terminal. The binding affinity was assessed by Tₘ value for the duplex between PNA and 10-mer complementary DNA. The duplex between PNA derivatives in Table 1 and fully complementary DNAs show Tₘ values too high to be reliably determined in aqueous buffer solution, since the buffer solution tends to boil off during the Tₘ measurement.

Tₘ values were determined on a UV/Vis spectrophotometer as follows. A mixed solution of 4 µM PNA oligomer and 4 µM complementary 10-mer DNA in 4 mL aqueous buffer (pH 7.16, 10 mM sodium phosphate, 100 mM NaCl) in 15 mL polypropylene falcon tube was incubated at 90°C for a minute and slowly cooled down to ambient temperature. Then the solution was transferred into a 3 mL quartz UV cuvette equipped with an air-tight cap, and subjected to a Tₘ measurement at 260 nm on a UV/Visible spectrophotometer as described in the prior art [PCT/KR2009/001256] or with minor modifications. The 10-mer complementary DNAs for Tₘ measurement were purchased from Bioneer (www.bioneer.com, Dajeon, Republic of Korea) and used without further purification.

Observed Tₘ values of the PNA derivatives of **Formula I** are very high for a complementary binding to 10-mer DNA, and provided in Table 2. For example, "ASO 10" showed a Tₘ value of 74.0°C for the duplex with the 10-mer complementary DNA targeting the N-terminal 10-mer in the PNA as marked "bold" and "underlined" in [(N → C) Fmoc-**TA(5)A-A(5)TA(5)-CGC(1O2)-A**A(5)A-A(5)AC-A(5)A-NH₂]. In the meantime, "ASO 10" showed a Tₘ of 68.6°C for the duplex with the 10-mer complementary DNA targeting the C-terminal 10-mer in the PNA as marked "bold" and "underlined" in [(N → C) Fmoc-TA(5)A-A(5)TA(5)-C**GC(1O2)-AA(5)A-A(5)AC-A(5)A-NH₂].**

**Table 2. Tₘ values between PNAs in Table and 10-mer complementary DNA targeting either the N-terminal or the C-terminal of PNA.**

| PNA | Tₘ Value, °C | |
|---|---|---|
| | 10-mer DNA against N-Terminal | 10-mer DNA against C-Terminal |
| ASO 5 | 63.5 | 71.6 |
| ASO 9 | 65.0 | 64.6 |
| ASO 10 | 74.0 | 68.6 |
| ASO 14 | 76.0 | 77.0 |

### Examples for Biological Activities of PNA Derivatives of Formula I

PNA derivatives of **Formula I** were evaluated for their biological activities in vitro and in vivo. The biological examples provided below are provided as examples to illustrate the biological profiles of such PNA derivatives of **Formula I,** and therefore should not be interpreted to limit the scope of the current invention.

### Example 1. Exon Skipping Induced by "ASO 9" in PC3 Cells.

"ASO 9" complementarily binds to the 16-mer pre-mRNA sequence as marked "bald" and "underlined" in a 30-mer sequence of [(5' → 3') CGUCA**UUGUUUUUGC** | **guaagu**acuuucagc] spanning the junction of "exon 4" and "intron 4" within the human SCN9A pre-mRNA. "ASO 9" possesses a 10-mer overlap with "exon 4" and a 6-mer overlap with "intron 4". Thus "ASO 9" meets the complementary overlap criteria for the compound of **Formula I** in this invention

Given that PC3 cells are known to abundantly express the human SCN9A mRNA [Br. J. Pharmacol. vol 156, 420-431 (2009**)],** "ASO 9" was evaluated by SCN9A nested RT-PCR for its ability to induce the skipping of "exon 4" of the human SCN9A pre-mRNA in PC3 cells as described below.

[Cell Culture & ASO Treatment] PC3 cells (Cat. No. CRL-1435, ATCC) were grown in 60 mm culture dish containing 5 mL Ham's F-12K medium supplemented with 10% FBS, 1% streptomycin/penicillin, 1% L-glutamine, and 1% sodium pyruvate under 5% CO₂ atmosphere at 37°C. Cells were then treated with "ASO 9" at 0 (negative control), 10, 100 or 1,000 zM for 18 hours until an additional treatment with 100 µg/mL cyclohexamide for another 6 hours in order to freeze the ribosomal translation.

[RNA Extraction] Total RNA was extracted from cells using "Universal RNA Extraction Kit" (Cat. Number 9767, Takara) according to the manufacturer's instructions.

[cDNA Synthesis by One Step RT-PCR] 200 ng of RNA template was used in a 25 µL reverse transcription reaction using Super Script^{®} One-Step RT-PCR kit with Platinum^{®} Taq polymerase (Cat. Number 10928-042, Invitrogen) and a set of gene-specific primers [exon 2_forward: (5' → 3') CTTTCTCCTTTCAGTCCTCT, and exon 9_reverse: (5' → 3') CGTCT-GTTGGTAAAGGTTTT] according to the following cycle conditions: 50°C for 30 min and 94°C for 2 min, followed by 40 cycles of 30 sec at 94°C, 30 sec at 55°C, and 2 min at 72°C.

[Nested PCR Amplification] 1 µL of cDNA solution (diluted by 100 times) was subjected to a 20 µL PCR amplification by nested PCR (Cat. No. K2612, Bioneer) against a set of primers of [exon 3n_forward: (5' → 3') GGACCAAAAATGTCGAGTATTT, and exon 8_reverse: (5' → 3') GCTAAGAAGGCCCAGCTGAA], which was designed to probe the skipping of "exon 4". The employed cycle conditions were 95°C for 5 min followed by 35 cycles of 30 sec at 95°C, 30 sec at 50°C, and 1 min at 72°C. The sequence of "exon 3n_forward" targets the junction of "exon 3" and "exon 5" to probe the deletion of "exon 4".

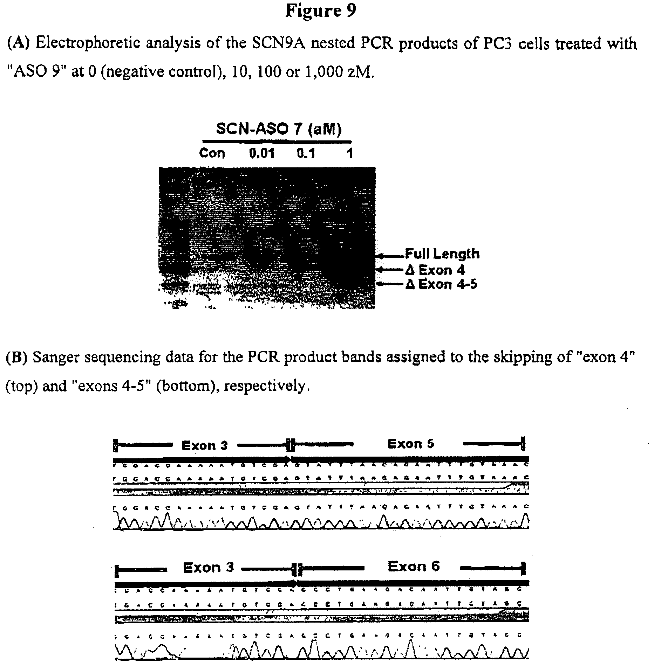

[Identification of Exon Skipping Products] The PCR products were subjected to electrophoretic separation on a 2% agarose gel. The bands of target size were collected and analyzed by Sanger Sequencing. The skipping of "exon 4" was conspicuously strong in PC3 cells treated with 1 aM "ASO 9", although the "exon 4" skipping was visible too at 10 and 100 zM [cf. Figure 9(A)]. The "exon 4" skipping band was unequivocally confirmed by Sanger sequencing as provided in Figure 9(B).

### Example 2. qPCR for SCN9A mRNA in PC3 Cells Treated with "ASO 9".

"ASO 9" was evaluated for its ability to induce changes in the expression level of the human SCN9A mRNA in PC3 cells by qPCR against exon-specific primers sets covering "exons 4-6" as follows.

[Cell Culture & ASO Treatment] PC3 cells grown in 60 mm culture dish containing 5 mL F-12K medium were incubated with "ASO 9" at 0 (negative control), 10, 100 or 1,000 zM for 24 hours. (2 culture dishes per ASO concentration)

[RNA Extraction] Total RNA was extracted using "MiniBEST Universal RNA Extraction Kit" (Cat. Number 9767, Takara) according to the manufacturer's instructions.

[cDNA Synthesis by One Step RT-PCR] 200 ng of RNA template was used for a 20 µL reverse transcription reaction using Super Script^{®} One-Step RT-PCR kit with Platinum^{®} Taq polymerase (Cat. Number 10928-042, Invitrogen) and against a set of exon-specific primers [exon 2_forward: (5' → 3') CTTTCTCCTTTCAGTCCTCT; and exon 9_reverse: (5' → 3') TTGCCTGGTTCTGTTCTT] according to the following cycle conditions: 50°C for 30 min and 94°C for 2 min, followed by 15 cycles of 15 sec at 94°C, 30 sec at 55°C, and 2 min at 72°C.

[Nested qPCR Amplification] The cDNA solutions were diluted by 50 times. 1 µL of each diluted cDNA solution was subjected to a 20 µL Real-Time PCR reaction against exon specific primers sets specified as follows: [exon 4_forward: (5' → 3') GTACACTTT-TACTGGAATATATAC; exon 4_reverse: (5' - 3') AATGACGACAAAATCCAGC; exon 5_forward: (5' → 3') GTATTTAACAGAATTTGTAAACCT; exon 5 reverse: (5' → 3') CTG-GGATTACAGAAATAGTTTTCA; exon 6 forward: (5' → 3') GAAGACAATTGTAGGGGC; exon 6 reverse: (5' → 3') GTCTTCTTCACTCTCTAGGG]. The PCR reactions were probed by SYBR Green (Takara, Japan) according to the following cycle conditions: 95°C for 30 sec followed by 40 cycles 5 sec at 95°C, and 30 sec at 60°C.

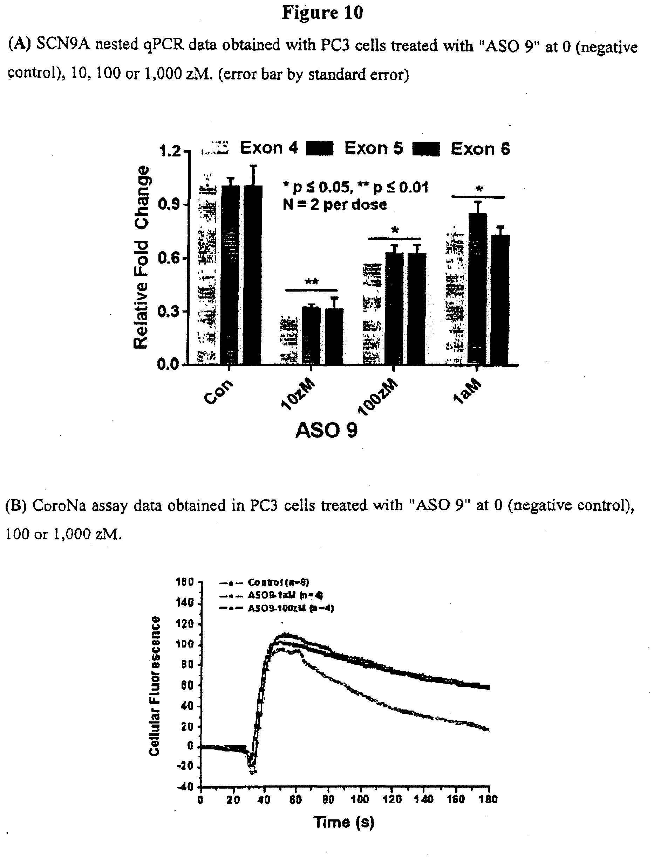

[qPCR Results] Each exon level of the ASO treated cells was normalized against the exon level of the negative control cells (i.e. without ASO treatment). Figure 10(A) summarizes the qPCR results. The expression levels of "exons 4-6" significantly decreased by ca 70%, 40% and 20 ~ 30% at 10, 100 and 1,000 zM, respectively. The dose response pattern could be an artifact possibly due to the "exon intron circular RNA (EIciRNA)" accumulated during the exon skipping by "ASO 9". [Nature Struc. Mol. Biol. vol 22(3), 256-264 (2015**)]**

### Example 3. Inhibition of Sodium Current in PC3 Cells Treated with "ASO 9".

Cellular sodium current is usually measured by patch clamp. As sodium ions enter cell, the intra-cellular sodium ion level increases. The intra-celluar sodium level can be probed using a sodium ion sensitive dye. "CoroNa Green" is a dye with a sodium ion chelator of crown ether type. Upon chelation of a sodium ion, "CoroNa Green" emits green fluorescence. "CoroNa Green" has been used to indirectly measure the intra-cellular sodium level. The sodium level measured by "CoroNa Green" was found to correlate well with the sodium ion current measured by sodium ion patch clamp. [Proc. Natl. Acad. Sci. USA vol 106(38), 16145-16150 (2009**)]**

PC3 cells are known to abundantly express the human SCN9A mRNA and sodium current as well, although there are other SCN subtypes simultaneously expressed. [Br. J. Pharmacol. vol 156, 420-431 (2009**)]** Thus a down-regulation of the (functionally active) SCN9A mRNA may lead to a considerable reduction of the sodium ion current in PC3 cells, if the sodium ion current by the Naᵥ1.7 sodium channel subtype occupies a marked portion of the total sodium ion current in PC3 cells. It is note that the SCN9A mRNA encodes the Naᵥ1.7 sodium channel subtype.

"ASO 9" was evaluated for its ability to down-regulate sodium ion current in PC3 cells using "CoroNa Green" as briefly described below.

[Cell Culture & ASO Treatment] PC3 cells were grown in 2 mL F-12K medium in 35 mm culture dish, and treated with "ASO 9" at 0 zM (negative control), 100 zM or 1 aM.

[CoroNa Assay] 30 hours later, the cells were washed with 2 mL HBSS (Hank's Balanced Salt Solution, Cat. Number 14025-092, Life Technologies), and then charged with 2 mL fresh HBSS. Then the cells were treated with 5 µM "CoroNa Green" (Cat. Number C36676, Life Technologies) at 37°C. 30 min later, the cells were washed 2 times with 2 mL HBSS, and charged with 2 mL fresh HBSS. The culture dish was mounted on an Olympus fluorescence microscope equipped with a digital video camera to continuously capture the green fluorescence images of the cells. The cells were acutely treated with 100 mM NaCl, and then the changes in fluorescence cellular images were digitally recorded over a peroid of 3 min. There were about 4 cells per frame. The fluorescence intensities from each individual cell were traced at a resolution of second. The traces of the intracellular fluorescence intensities from individual cells were overlaid and averaged at each time point. The average of the traces from the individual cells of each ASO concentration was plotted as provided in Figure 10(B) using ImageJ program (version 1.50i, NIH). The average fluorescence intensity trace was taken as the individual intra-cellular sodium concentration trace for the cells treated with "ASO 9" at 0 (negative control), 100 or 1,000 zM.

[CoroNa Assay Results] The observed traces of intra-cellular fluorescence intensity are summarized in Figure 10(B). The fluorescence intensity trace for the cells treated with 1,000 zM "ASO 9" runs lower than the trace for the cells without ASO treatment. The average fluorescence intensities at 100 sec were compared to estimate a sodium current change induced by ASO treatment. The average fluorescence intensity of the cells without ASO treatment was 81.86 (arbitrary unit) at 100 sec. In the meantime, the average fluorescence intensity of the cells treated with 1,000 zM "ASO 9" was 51.47 (arbitrary unit) at 100 sec. Thus, a 30 hour incubation with 1,000 zM "ASO 9" induced a significant reduction in the sodium channel activity by 37% (p-value = 0.035 by student's t-test) in PC3 cells. Considering that PC3 cells express various subtypes of voltage-gated sodium channel (VGSC), the 37% decrease is taken as marked for the inhibition of Naᵥ1.7 expression by "ASO 9". There was no marked decrease in the sodium current in PC3 cells treated with 100 zM "ASO 9".

### Example 4. qPCR Evaluation of SCN9A mRNA in PC3 Cells Treated with "ASO 4".

"ASO 4" is a 14-mer SCN9A ASO initially designed to complementarily target a 14-mer sequence spanning the junction of "exon 4" and "exon 5" in the human SCN9A mRNA. However, "ASO 4" happens to complementarily overlap with a 12-mer pre-mRNA sequence as marked "bald" and "underlined" in the 30-mer 5' splice site sequence of [(5' → 3') CGUCAUUG**UUUUUGC** | **gua**"ag"**ua**cuuucagc] spanning the junction of "exon 4" and "intron 4" within the human SCN9A pre-mRNA, although there are two mismatches with "intron 5" as marked with a quote sign (" "). "ASO 4" possesses a 7-mer overlap with "exon 4" and a 5-mer overlap with "intron 4". Thus "ASO 4" meets the complementary overlap criteria for the compound of **Formula I** in this invention.

"ASO 4" was evaluated for its ability to inhibit the expression of the human SCN9A mRNA by qPCR against exon-specific primers sets covering "exons 4-6" according to the procedures provided in "Example 2" unless noted otherwise.

[ASO Treatment] The concentration of "ASO 4" in culture dish was 0 (negative control), 10, 100 or 1,000 zM. (2 culture dishes per dose)

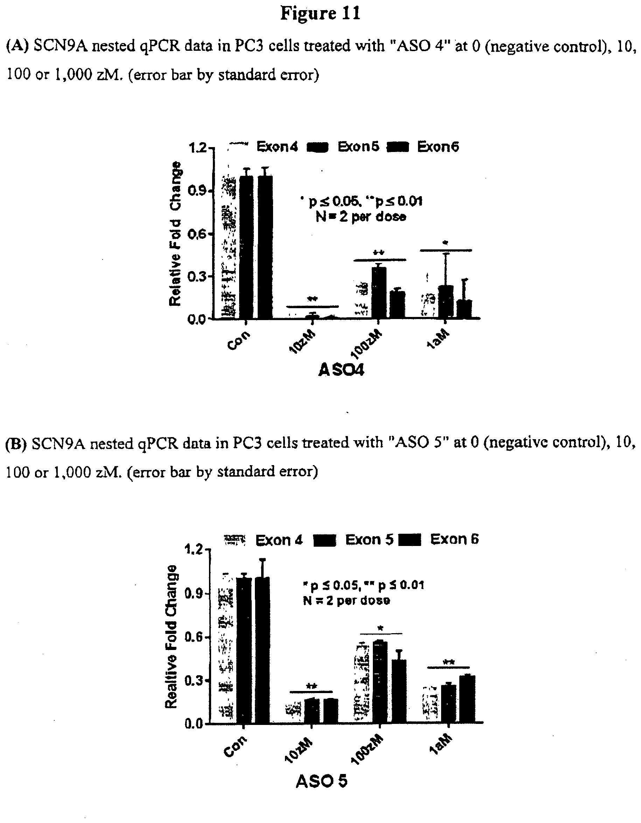

[qPCR Results] Figure 11(A) provides the qPCR results obtained with PC3 cells treated with "ASO 4". The expression levels of "exons 4-6" significantly decreased by > 70% in the PC3 cells treated with "ASO 4" at 10 to 1,000 zM for 24 hours.

### Example 5. qPCR Evaluation of SCN9A mRNA in PC3 Cells Treated with "ASO 5".

"ASO 5" is a 17-mer SCN9A ASO initially designed to complementarily target a 17-mer sequence spanning the junction of "exon 4" and "exon 5" in the human SCN9A mRNA. However, "ASO 5" happens to complementarily overlap with a 12-mer pre-mRNA sequence as marked "bald" and "underlined" in the 30-mer 5' splice site sequence of [(5' → 3') CGUCAUUG**UUUUUGC** | **gua**"ag"**ua**"cuu"ucagc] spanning the junction of "exon 4" and "intron 4" within the human SCN9A pre-mRNA, although there are five mismatches with "intron 5" as marked with a quote sign (" "). "ASO 5" possesses a 7-mer overlap with "exon 4" and a 5-mer overlap with "intron 4". Thus "ASO 5" does not meet the complementary overlap criteria for the compound of **Formula I** in this invention due to the 5 mismatches, although "ASO 5" and "ASO 4" possess the same degree of complementary overlap with the SCN9A pre-mRNA.

"ASO 5" was evaluated for its ability to inhibit the expression of the human SCN9A mRNA (full length) by qPCR against exon-specific primers sets covering "exons 4-6" according to the procedures provided in "Example 2" unless noted otherwise.

[qPCR Results] Figure 11(B) provides the qPCR results obtained with PC3 cells treated with "ASO 5". The expression levels of "exons 4-6" significantly decreased by ca 80%, 50% and 70% in the PC3 cells treated with "ASO 5" at 10 zM, 100 zM and 1 aM, respectively.

Even though "ASO 5" inhibited the expression of the full length SCN9A mRNA by qPCR, the five mismatches of "ASO 5" against the SCN9A pre-mRNA is rather too much and increases the propensity of cross reactivity with other pre-mRNA(s).

### Example 6. qPCR Evaluation of SCN9A mRNA in PC3 Cells Treated with "ASO 1".

"ASO 1" is a 14-mer SCN9A ASO possessing the same oligonucleotide sequence as "ASO 4", although the N-terminus substituent of "Fethoc-" radical in is replaced with "Fmoc" radical in "ASO 1". Thus "ASO 1" meets the complementary overlap criteria for the compound of **Formula I** in the present invention.

"ASO 1" was evaluated for its ability to inhibit the expression of the human SCN9A mRNA (full length) by qPCR against exon-specific primers sets covering "exons 4-6" according to the procedures provided in "Example 2" unless noted otherwise.

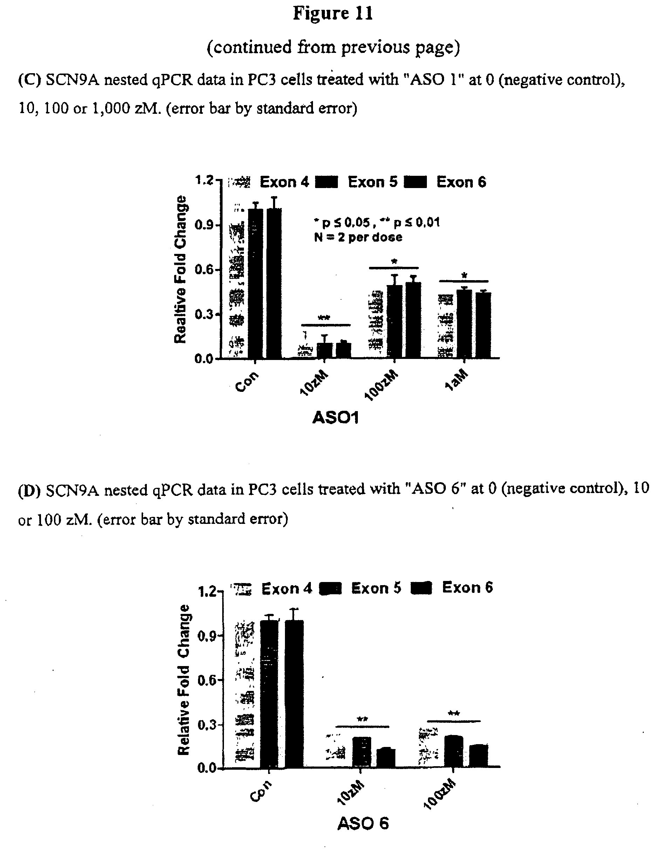

[qPCR Results] Figure 11(C) provides the qPCR results obtained with PC3 cells treated with "ASO 1". The expression levels of "exons 4-6" significantly (student's t-test) decreased by ca 85%, 50% and 60% in the PC3 cells treated with "ASO 1" at 10 zM, 100 zM and 1 aM, respectively.

### Example 7. qPCR Evaluation of SCN9A mRNA in PC3 Cells Treated with "ASO 6".

"ASO 6" is a 14-mer SCN9A ASO possessing a 11-mer complementary overlap with the SCN9A pre-mRNA as marked "bald" and "underlined" within the 30-mer 5' splice site sequence of [(5' → 3') CGUCAUUG**UUUUU"**G"**C** | **gua**"ag"**ua**cuuucagc] spanning the junction of "exon 4" and "intron 4" within the human SCN9A pre-mRNA. "ASO 6" possesses a 6-mer overlap with "exon 4" and a 5-mer overlap with "intron 4". Since "ASO 6" possesses 3 mismatches against the human SCN9A pre-mRNA, "ASO 6" does not meet the complementary overlap criteria for the compound of **Formula I** in this invention.

"ASO 6" was evaluated for its ability to inhibit the expression of the human SCN9A mRNA (full length) by qPCR against exon-specific primers sets covering "exons 4-6" according to the procedures provided in "Example 2" unless noted otherwise. It is noted that PC3 cells were incubated with "ASO 6" at 0 (negative control), 10 zM and 100 zM.

[qPCR Results] Figure 11(D) provides the qPCR results obtained with PC3 cells treated with "ASO 6". The expression levels of "exons 4-6" significantly (student's t-test) decreased by ca 80% in the PC3 cells treated with "ASO 6" at 10 zM and 100 zM.

Although "ASO 6" does not meet the complementary overlap criteria for the compound of **Formula I** in this invention due to the 3 mismatches, the qPCR data of "ASO 6" suggests that even an 11-mer complementary overlap with the SCN9A pre-mRNA would be still strong enough to induce the exon skipping in PC3 cells.

### Example 8. qPCR Evaluation of SCN9A mRNA in PC3 Cells Treated with "ASO 10".

"ASO 10" is a 17-mer SCN9A ASO initially designed to complementarily target a 17-mer sequence spanning the junction of "exon 4" and "exon 5" in the human SCN9A mRNA. Nevertheless, "ASO 10" happens to complementarily overlap with a 15-mer pre-mRNA sequence as marked "bald" and "underlined" in the 30-mer 5' splice site sequence of [(5' → 3') CGUCA**UUGUUUUUGC** | **gua**"ag"**ua**cuuucagc] spanning the junction of "exon 4" and "intron 4" within the human SCN9A pre-mRNA, although there are two mismatches with "intron 5" as marked with a quote sign (" "). "ASO 10" possesses a 10-mer overlap with "exon 4" and a 5-mer overlap with "intron 4". Thus "ASO 10" meets the complementary overlap criteria for the compound of **Formula I** in the present invention.

"ASO 10" was evaluated for its ability to inhibit the expression of the human SCN9A mRNA (full length) by qPCR against exon-specific primers sets covering "exons 4-6" according to the procedures described in "Example 2" unless noted otherwise. It is noted that PC3 cells were incubated with "ASO 10" at 0 (negative control), 10 zM and 100 zM.

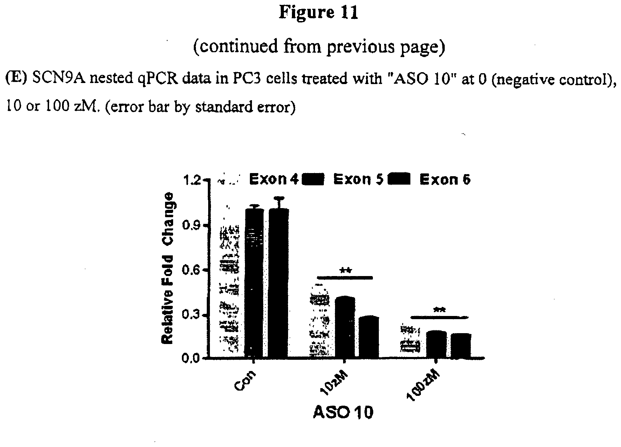

[qPCR Results] Figure 11(E) provides the qPCR results obtained with PC3 cells treated with "ASO 10". The expression levels of "exons 4-6" significantly (student's t-test) decreased by ca 60% and 80% in the PC3 cells treated with "ASO 10" at 10 zM and 100 zM, respectively.

### Example 9. Inhibition of Sodium Current in PC3 Cells Treated with "ASO 10".

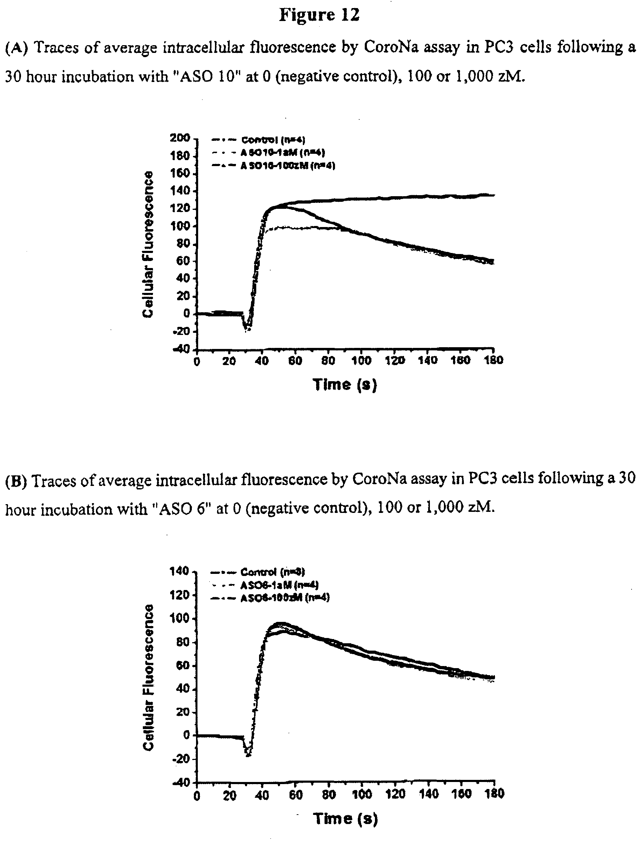

"ASO 10" was evaluated for its ability to inhibit the sodium current in PC3 cells using "CoroNa Green" according to the procedures described in "Example 3" unless noted otherwise. [CoroNa Assay Results] The observed traces of average cellular fluorescence intensity are provided in Figure 12(A). The average fluorescence intensity trace for the cells treated with 1,000 zM "ASO 10" ran lower than that for the cells without ASO treatment. The average cellular fluorescence intensity of the cells without ASO treatment was 130.3 (arbitrary unit) at 100 sec. In the meantime, the average cellular fluorescence intensity of the cells treated with 1,000 zM "ASO 10" was 89.7 (arbitrary unit) at 100 sec. Thus, a 30 hour incubation with 1,000 zM "ASO 10" is estimated to have significantly (p < 0.001) inhibited the sodium channel activity by 31% in PC3 cells. The decrease induced by 100 zM "ASO 10" was 30% (p < 0.001).

### Example 10. Inhibition of Sodium Current in PC3 Cells Treated with "ASO 6".

"ASO 6" was evaluated for its ability to down-regulate sodium current in PC3 cells using "CoroNa Green" according to the procedures provided in "Example 3" unless noted otherwise.

[CoroNa Assay Results] The observed traces of cellular fluorescence intensity are provided in Figure 12(B). The fluorescence intensity traces for the cells treated with 100 and 1,000 zM "ASO 6" were no different from the trace for the cells without ASO treatment. Thus, a 30 hour incubation with "ASO 6" failed to induce a notable decrease in the sodium channel activity in PC3 cells.

Although "ASO 6" inhibited the expression of the full length SCN9A mRNA as provided in "Example 7", "ASO 6" failed to inhibit the sodium current in PC3 cells. "ASO 6" may not tightly bind to the 5' splice site of "exon 4" enough to induce the exon skipping owing to the three mismatches with the target pre-mRNA sequence.

### Example 11. Inhibition of Sodium Current in PC3 Cells Treated with "ASO 4".

"ASO 4" was evaluated for its ability to down-regulate sodium current in PC3 cells using "CoroNa Green" according to the procedures provided in "Example 3" unless noted otherwise.

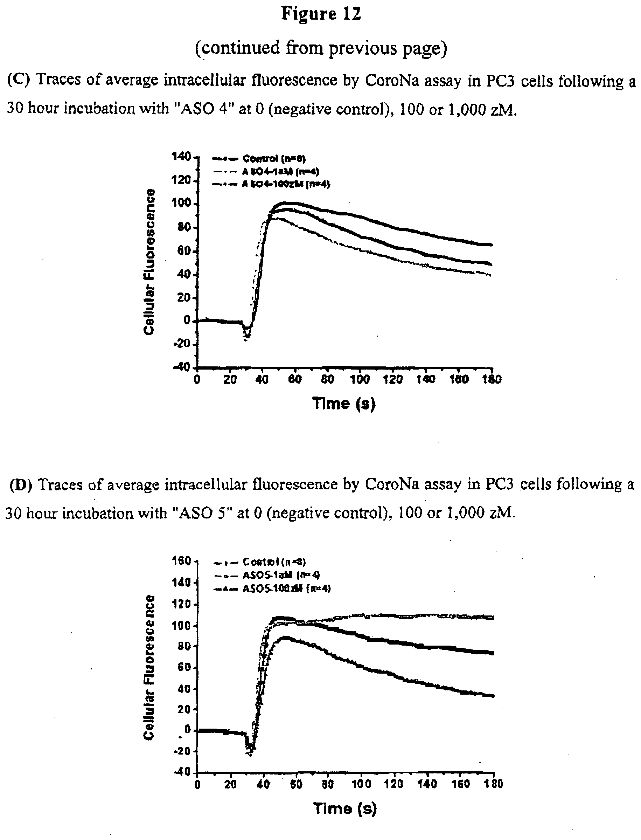

[CoroNa Assay Results] The observed traces of cellular fluorescence intensity are provided in Figure 12(C). The fluorescence intensity trace for the cells treated with 100 zM "ASO 4" ran lower than that for the cells without ASO treatment. The average cellular fluorescence intensity of the cells without ASO treatment (i.e. negative control) was 89.3 (arbitrary unit) at 100 sec. In the meantime, the average cellular fluorescence intensity of the cells treated with 1,000 zM "ASO 10" was 61.4 (arbitrary unit) at 100 sec. Thus, a 30 hour incubation with 1,000 zM "ASO 4" is estimated to have significantly (p < 0.01) decreased the sodium channel activity by 31% in PC3 cells. However, the decrease induced by 100 zM "ASO 4" was only 18% without statistical significance.

### Example 12. Inhibition of Sodium Current in PC3 Cells Treated with "ASO 5".

"ASO 5" was evaluated for its ability to inhibit sodium current in PC3 cells using "CoroNa Green" according to the procedures provided in "Example 3" unless noted otherwise.

[CoroNa Assay Results] The observed traces of cellular fluorescence intensity are summarized in Figure 12(D). The fluorescence intensity trace for the cells treated with 100 zM "ASO 5" ran lower than the trace for the cells without ASO treatment. The average cellular fluorescence intensity of the cells without ASO treatment was 90.6 (arbitrary unit) at 100 sec. In the meantime, the average cellular fluorescence intensity of the cells treated with 100 zM "ASO 5" was 60.8 (arbitrary unit) at 100 sec. Thus, a 30 hour incubation with 100 zM "ASO 5" is estimated to have significantly (p < 0.01) down-regulated the sodium channel activity by 33% in PC3 cells.

As the concentration of "ASO 5" was increased from 100 zM to 1,000 zM, however, the average cellular fluorescence intensity increased to 110.2 (arbitrary unit) at 100 sec. Thus, a 30 hour incubation with 1,000 zM "ASO 5" is estimated to have significantly (p < 0.05) increased the sodium channel activity by 22% in PC3 cells. The dose response pattern could be a result of transcription upregulation possibly due to the "exon intron circular RNA (EIciRNA)" accumulated during the exon skipping by "ASO 5". [Nature Struc. Mol. Biol. vol 22(3), 256-264 (2015**)]**

### Example 13. L5 Ligation and L5/L6 Ligation in Spinal Nerve Ligation.

Spinal nerve ligation (SNL) induces neuropathy in DRG (dorsal root ganglia) and has been widely used as a model for neuropathic pains. [Pain vol 50(3), 355-363 (1992**)]** Depending on how spinal nerve bundle(s) is ligated, however, there can be several variations of SNL. The degree and duration of neuropathy in DRG appears to vary depending on how nerve bundles are ligated. [Pain vol 43(2), 205-218 (1990**)]** Of the two SNL variations performed for this invention, "L5/L6 ligation" (i.e. Method B) is considered to induce neuropathy more severe and persisting longer than "L5 ligation with L6 cut" (i.e. Method A).

[Method A: L5 Ligation with L6 Cut] Male SD rats were anesthetized with zoletil/rompun. The L5 and L6 spinal nerve bundles (left side) were exposed and tightly ligated, and then the L6 nerve was cut. Finally the muscle and skin were closed and clipped according to due aseptic procedures.

[Method B: L5/L6 Ligation] Male SD rats were anesthetized with zoletil/rompun. The L5 and L6 spinal nerve bundles (left side) were exposed and tightly ligated. Then the muscle and skin were closed and clipped according to due aseptic procedures.

### Example 14. Allodynia Scoring by Von Frey.

[Method A: Electronic Von Frey] Allodynia was scored by von Frey method using an electronic von Frey anesthesiometer [37450 Dynamic Plantar Aesthesiometer, Ugo Basile; or, Model Number 2390, IITC Inc. Life Sciences] as briefly described as follows: After stabilizing for less than 30 minutes each animal in a plastic cage customized for von Frey scoring, the ligated hindpaw (ligated side, left usually) of each animal was subjected to von Frey scoring 6 times with an interval of a few minutes between two neighboring rounds of scoring. The first round scores were discarded since the animals were considered not fully acclimated during the first round of scoring. Of the five remaining scores, the highest and lowest scores were excluded as outliers. Then the average of the remaining three scores was taken as the von Frey score for the animal.

[Method B: Von Frey with Microfilaments (Touch Test^{®})] Allodynia was scored with a set of microfilaments (Touch Test^{®}) according to the "Up & Down" method. [J Neurosci. Methods vol 53(1), 55-63 (1994**)]**

### Example 15. Reversal of Allodynia Induced with SNL by "ASO 1".

"ASO 1" was evaluated for its ability to reverse the allodynia elicited by SNL in rats as described below.

[SNL Operation and Grouping] In Day 0, 30 male SD rats were subjected to SNL surgery of L5 ligation with L6 cut (Method A in "Example 13"). In Day 15, eighteen rats showing the lowest von Frey scores were selected and randomly assigned to three groups of the negative control (no ASO treatment), "ASO 1" 3 pmole/Kg, and "ASO 1" 10 pmole/Kg group (N = 6 per group).

[ASO Dosing and Von Frey Scoring] An aqueous stock solution of "ASO 1" was serially diluted to 3 nM and 10 nM "ASO 1" in PBS. Each diluted solution was subcutaneously administered to each rat of the ASO treatment groups at 1 mL/Kg in Days 16, 18, 20, 22, and 24. Von Frey scoring (by Electronic Von Frey: Method A in "Example 14") was carried out in Days 20, 22. 24, 27, and 29. ASO was administered after von Frey scoring in Days 20, 22, and 24. Von Frey scores were evaluated by student's t-test for statistical significance against the negative control group.

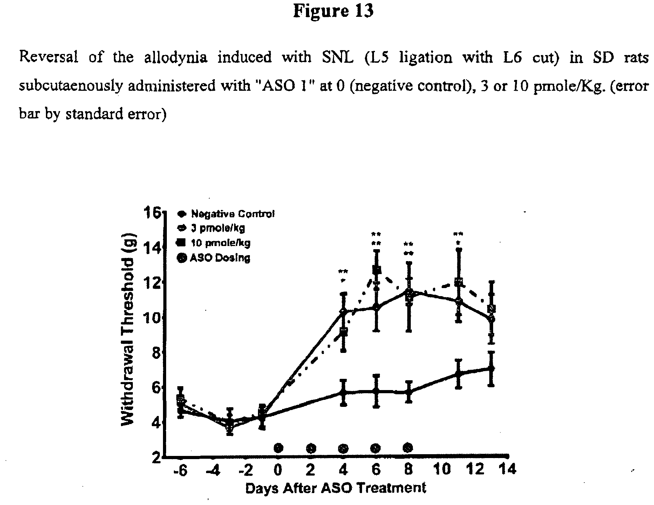

[Therapeutic Activity] Figure 13 provides the observed von Frey scores. The average von Frey scores of the ASO treatment groups were significantly higher than the negative control group in Days 20, 22, 24 and 27. The therapeutic activity persisted at least three days post the final ASO dosing in Day 24. Thus the allodynia induced with "L5 ligation" was significantly reversed in rats subcutaneous administered with "ASO 1" at 3 or 10 pmole/Kg.

### Example 16. Induction of Diabetes-induced Peripheral Neuropathic Pain (DPNP).

Peripheral neuropathic pain was induced in rats with type I diabetes as briefly described. Streptozotocin dissolved in citrate buffer (pH 6) was intra-peritoneally administered at 60 mg/Kg to male SD rats weighing ca 200 g. [J. Ethnopharmacol. vol 72(1-2), 69-76 (2000**)]** The degree of peripheral neuropathy was assessed by von Frey score. Animals showing low von Frey scores stably over days were selected for evaluation of the therapeutic activity of SCN9A ASOs.

### Example 17. Reversal of Allodynia by "ASO 5" in Rats with DPNP

"ASO 5" was evaluated for its ability to reverse the allodynia induced by DPNP in rats as described beolw.

[Induction of DPNP and Grouping] Type I diabetes was induced in Day 0 by an intraperitoneal administration of streptozotocin to male SD rats as described in "Example 16". In Day 10, rats with DPNP were randomly grouped based on the von Frey scores of individual animals in Day 10 by "Method B" in "Example 14". (Groups 1 ~ 6 and N = 8 per group) The six groups are "Group 1" for vehicle only (negative control), "Group 2" for pregabalin 30 mg/Kg, "Group 3" for "ASO 5" 0.01 pmole/Kg, "Group 4" for "ASO 5" 0.1 pmole/Kg, "Group 5" for "ASO 5" 1 pmole/Kg, and "Group 6" for "ASO 5" 10 pmole/Kg.

[ASO Treatment and von Frey Scoring] An aqueous stock solution of "ASO 5" was serially diluted to 0.01, 0.1, 1 and 10 nM "ASO 5" in DDW (deionized distilled water). "ASO 5" was subcutaneously administered in Days 11, 13, 15, and 17. Pregabalin 30 mg/Kg was orally administered in Days 11 and 17 as the positive control. Von Frey scoring was carried out 2 hours post dose in Days 11, 13, 15, and 17 by "Method B" described in "Example 14". Von Frey scoring was additionally performed in Day 20 to assess the duration of the therapeutic activity after the final dosing. Von Frey scores were evaluated for statistical significance by student's t-test against "Group 1" (vehicle only, negative control).

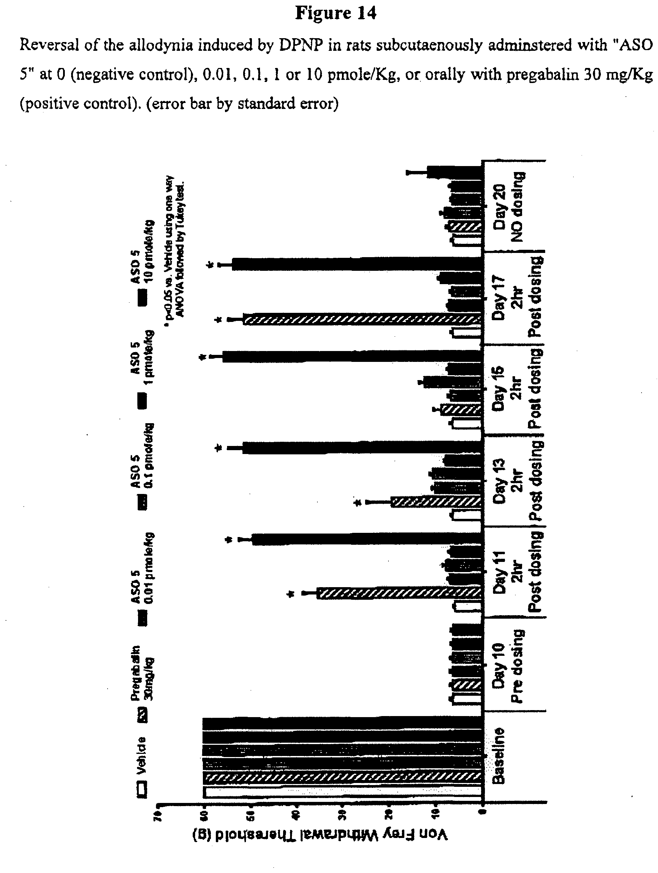

[Therapeutic Activity] The observed von Frey scores are summarized in Figure 14. Of the ASO treatment groups, the allodynia was markedly (~ 80%) and significantly reversed only in Group 6, i.e., "ASO 5" 10 pmole/Kg. It is interesting to note that the onset of the therapeutic activity was as fast as a few hours as observed in Day 11. The allodynia was comparably reversed in "Group 6" ("ASO 5" 10 pmole/Kg) and "Group 2" (pregabalin 30 mg/Kg) in Day 17. The therapeutic activity of "Group 6" washed out almost completely in Day 20 (three days after the final dose in Day 17).

### Example 18. Reversal of Allodynia by "ASO 9" and "ASO 10" in Rats with DPNP.

"ASO 9" and "ASO 10" were evaluated for their ability to reverse the allodynia induced by DPNP in rats according to the procedures described in "Example 17", unless noted otherwise.

[Grouping] In Day 10, rats with DPNP were randomly assigned to three groups of the negative control (vehicle/DDW only), "ASO 9" 100 pmole/Kg and "ASO 10" 100 pmole/Kg (N = 8 ~ 9 per group).

[ASO Treatment and von Frey Scoring] Rats were subcutaneously administered with "ASO 9", "ASO 10" or vehicle in Days 11, 13, 15, 17 and 19. Von Frey scoring was performed 2 hours post dose in Days 11, 13, 15, 17 and 19. Von Frey scoring was additionally performed in Days 21 and 23 to assess the duration of the therapeutic activity after the final dosing. Statistical significance of daily von Frey scores was assessed by student's t-test against the negative control group.

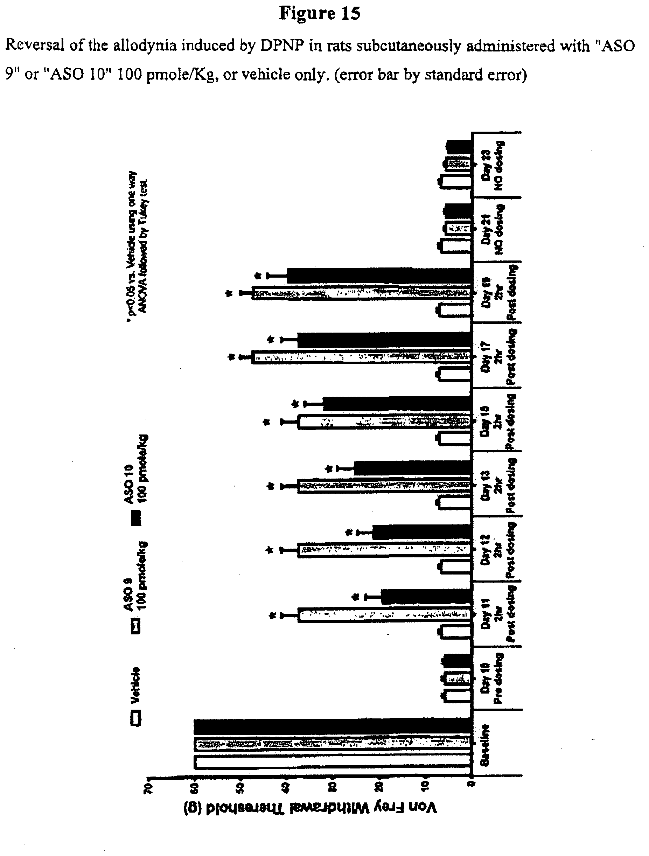

[Therapeutic Activity] The observed von Frey scores are summarized in Figure 15. The allodynia was markedly and significantly reversed by "ASO 9" and "ASO 10". "ASO 9" reversed the allodynia by ca 75% in Days 17 and 19. In case of "ASO 10", the allodynia was gradually reversed to ca 60% in Day 19. "ASO 9" possesses more complementary overlap with the SCN9A pre-mRNA than "ASO 10", which could explain the difference in the therapeutic efficacy of the two ASOs. The therapeutic activity of the ASOs completely washed out in Day 21, i.e. 2 days after the final dosing, suggesting a pharmacodynamic half-life shorter than a few days.

### Example 19. Reversal of Allodynia Induced with SNL by "ASO 9".

"ASO 9" was evaluated for its ability to reverse the allodynia elicited by SNL in rats as described below.

[SNL Operation and Grouping] In Day 0, male SD rats were subjected to SNL surgery of "L5/L6 ligation" ("Method B" in "Example 13"). In Day 21, 12 rats were selected based on the von Frey scores by electronic von Frey ("Method A" in "Example 14"), and randomly assigned to two groups of Negative Control (no ASO treatment) and "ASO 9" 100 pmole/Kg (N = 6 per group).

[ASO Dosing and Von Frey Scoring] An aqueous stock solution of "ASO 9" was diluted to 100 nM "ASO 9" in PBS. The ASO solution or PBS was subcutaneously administered to rats at 1 mL/Kg three times per day at 08:00H, 14:00H and 21:00H in Days 22, 23 and 24. Von Frey scoring was carried out at 20:00H in Days 22, 23 and 24 by "Method A" described in "Example 14". Von Frey scores were evaluated by student's t-test for statistical significance between the two groups.

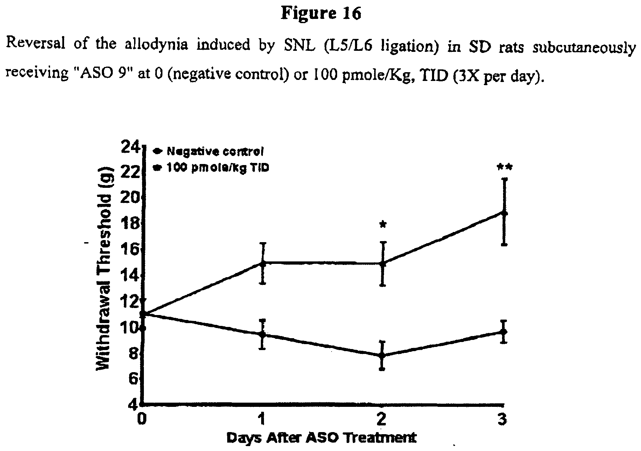

[Therapeutic Activity] The observed von Frey scores are summarized in Figure 16. The average von Frey scores of the ASO treatment group were significantly higher than the negative control group in Days 23 and 24. Thus subcutaneous TID administrations of "ASO 9" at 100 pmole/Kg significantly reversed the allodynia induced with SNL (L5/L6 ligation) in rats.

## Claims

1. A peptide nucleic acid derivative represented by **Formula I,** or a pharmaceutically acceptable salt thereof: 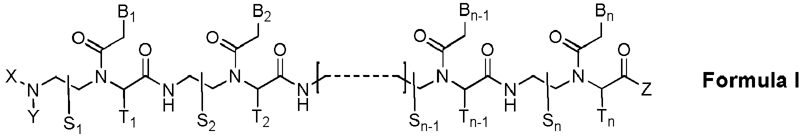 wherein,
n is an integer between 10 and 21;
the compound of **Formula I** possesses at least a 10-mer complementary overlap with the 14-mer RNA sequence of [(5' → 3') UUUUUGCGUAAGUA] within the human SCN9A pre-mRNA;
the compound of **Formula I** is fully complementary to the target pre-mRNA sequence, or partially complementary to the target pre-mRNA sequence with one or two mismatches;
S₁, S₂, ..., Sₙ₋₁, Sₙ, T₁, T₂, ..., Tₙ₋₁, and Tₙ independently represent deuterido, hydrido, substituted or non-substituted alkyl, or substituted or non-substituted aryl radical;
X and Y independently represent hydrido [H], formyl [H-C(=O)-], aminocarbonyl [NH₂-C(=O)-], substituted or non-substituted alkyl, substituted or non-substituted aryl, substituted or non-substituted alkylacyl, substituted or non-substituted arylacyl, substituted or non-substituted alkyloxycarbonyl, substituted or non-substituted aryloxycarbonyl, substituted or non-substituted alkylaminocarbonyl, substituted or non-substituted arylaminocarbonyl, substituted or non-substituted alkylsulfonyl, or substituted or non-substituted arylsulfonyl radical;
Z represents hydroxy, substituted or non-substituted alkyloxy, substituted or non-substituted aryloxy, substituted or non-substituted amino, substituted or non-substituted alkyl, or substituted or non-substituted aryl radical;
B₁, B₂, ..., Bₙ₋₁, and Bₙ are independently selected from natural nucleobases including adenine, thymine, guanine, cytosine and uracil, and unnatural nucleobases; and,
at least four of B₁, B₂, ..., Bₙ₋₁, and Bₙ are independently selected from unnatural nucleobases with a substituted or non-substituted amino radical covalently linked to the nucleobase moiety.

2. The peptide nucleic acid derivative according to claim 1, or a pharmaceutical salt thereof:
wherein,
n is an integer between 10 and 21;
the compound of **Formula I** possesses at least a 10-mer complementary overlap with the 14-mer RNA sequence of [(5' → 3') UUUUUGCGUAAGUA] within the human SCN9A pre-mRNA;
the compound of **Formula I** is fully complementary to the target pre-mRNA sequence, or partially complementary to the target pre-mRNA sequence with one or two mismatches;
S₁, S₂, ..., Sₙ₋₁, Sn, T₁, T₂, ..., Tₙ₋₁, and Tₙ independently represent hydrido radical;
X and Y independently represent hydrido [H], formyl [H-C(=O)-], aminocarbonyl [NH₂-C(=O)-], substituted or non-substituted alkyl, substituted or non-substituted aryl, substituted or non-substituted alkylacyl, substituted or non-substituted arylacyl, substituted or non-substituted alkyloxycarbonyl, substituted or non-substituted aryloxycarbonyl, substituted or non-substituted alkylaminocarbonyl, substituted or non-substituted arylaminocarbonyl, substituted or non-substituted alkylsulfonyl, or substituted or non-substituted arylsulfonyl radical;
Z represents hydroxy, substituted or non-substituted alkyloxy, substituted or non-substituted aryloxy, substituted or non-substituted amino, substituted or non-substituted alkyl, or substituted or non-substituted aryl radical;
B₁, B₂, ..., Bₙ₋₁, and Bₙ are independently selected from natural nucleobases including adenine, thymine, guanine, cytosine and uracil, and unnatural nucleobases;
at least three of B₁, B₂, ..., Bₙ₋₁, and Bₙ are independently selected from unnatural nucleobases represented by **Formula II, Formula III,** or **Formula IV:** 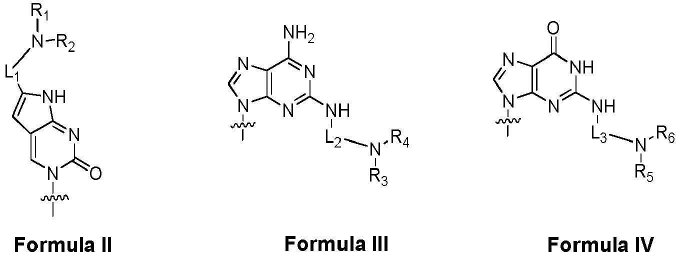
wherein,
R₁, R₂, R₃, R₄, R₅ and R₆ are independently selected from hydrido, and substituted or non-substituted alkyl radical; and,
L₁, L₂ and L₃ are a covalent linker represented by **Formula V** connecting the basic amino group to the nucleobase moiety responsible for nucleobase pairing: 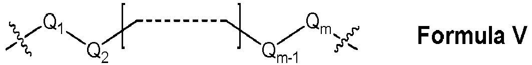
wherein,
Q₁ and Qₘ are substituted or non-substituted methylene (-CH₂-) radical, and Qₘ is directly linked to the basic amino group;
Q₂, Q₃, ..., and Qₘ₋₁ are independently selected from substituted or non-substituted methylene, oxygen (-O-), sulfur (-S-), and substituted or non-substituted amino radical [-N(H)-, or -N(substituent)-]; and,
m is an integer between 1 and 16.

3. The peptide nucleic acid derivative according to claim 1, or a pharmaceutical salt thereof:
wherein,
n is an integer between 12 and 20;
the compound of **Formula I** possesses at least a 10-mer complementary overlap with the 14-mer RNA sequence of [(5' → 3') UUUUUGCGUAAGUA] within the human SCN9A pre-mRNA;
the compound of **Formula I** is fully complementary to the target pre-mRNA sequence, or partially complementary to the target pre-mRNA sequence with one or two mismatches;
S₁, S₂, ..., Sₙ₋₁, Sn, T₁, T₂, ..., Tₙ₋₁, and Tn are hydrido radical;
X and Y independently represent hydrido [H], aminocarbonyl [NH₂-C(=O)-], substituted or non-substituted alkyl, substituted or non-substituted aryl, substituted or non-substituted alkylacyl, substituted or non-substituted arylacyl, substituted or non-substituted alkyloxycarbonyl, substituted or non-substituted alkylaminocarbonyl, or substituted or non-substituted arylsulfonyl radical;
Z represents substituted or non-substituted amino radical;
B₁, B₂, ..., Bₙ₋₁, and Bₙ are independently selected from natural nucleobases including adenine, thymine, guanine and cytosine, and unnatural nucleobases;
at least four of B₁, B₂, ..., Bₙ₋₁, and Bₙ are independently selected from unnatural nucleobases represented by **Formula II**, **Formula III**, or **Formula IV**;
R₁, R₂, R₃, R₄, R₅ and R₆ are independently selected from hydrido, and substituted or non-substituted alkyl radical;
Q₁ and Qₘ are substituted or non-substituted methylene radical, and Qₘ is directly linked to the basic amino group;
Q₂, Q₃, ..., and Qₘ₋₁ are independently selected from substituted or non-substituted methylene, oxygen, and amino radical; and,
m is an integer between 1 and 11.

4. The peptide nucleic acid derivative according to claim 1, or a pharmaceutical salt thereof:
wherein,
n is an integer between 12 and 19;
the compound of **Formula I** possesses at least a 10-mer complementary overlap with the 14-mer RNA sequence of [(5' → 3') UUUUUGCGUAAGUA] within the human SCN9A pre-mRNA;
the compound of **Formula I** is fully complementary to the target pre-mRNA sequence;
S₁, S₂, ..., Sₙ₋₁, Sn, T₁, T₂, ..., Tₙ₋₁, and Tn are hydrido radical;
X and Y independently represent hydrido [H], substituted or non-substituted alkylacyl, substituted or non-substituted arylacyl, substituted or non-substituted alkyloxycarbonyl, or substituted or non-substituted alkylaminocarbonyl radical;
Z represents substituted or non-substituted amino radical;
B₁, B₂, ..., Bₙ₋₁, and Bₙ are independently selected from natural nucleobases including adenine, thymine, guanine and cytosine, and unnatural nucleobases;
at least four of B₁, B₂, ..., Bₙ₋₁, and Bₙ are independently selected from unnatural nucleobases represented by **Formula II**, **Formula III**, or **Formula IV**;
R₁, R₂, R₃, R₄, R₅ and R₆ are independently selected from hydrido, and substituted or non-substituted alkyl radical;
Q₁ and Qₘ are methylene radical, and Qₘ is directly linked to the basic amino group;
Q₂, Q₃, ..., and Qₘ₋₁ are independently selected from methylene, and oxygen radical; and,
m is an integer between 1 and 10.

5. The peptide nucleic acid derivative according to claim 1, or a pharmaceutical salt thereof:
wherein,
n is an integer between 12 and 18;
the compound of **Formula I** possesses at least a 10-mer complementary overlap with the 14-mer RNA sequence of [(5' → 3') UUUUUGCGUAAGUA] within the human SCN9A pre-mRNA;
the compound of **Formula I** is fully complementary to the target pre-mRNA sequence;
S₁, S₂, ..., Sₙ₋₁, Sn, T₁, T₂, ..., Tₙ₋₁, and Tn are hydrido radical;
X and Y independently represent hydrido [H], substituted or non-substituted alkylacyl, substituted or non-substituted arylacyl, or substituted or non-substituted alkyloxycarbonyl radical;
Z represents substituted or non-substituted amino radical;
B₁, B₂, ..., Bₙ₋₁, and Bₙ are independently selected from natural nucleobases including adenine, thymine, guanine and cytosine, and unnatural nucleobases;
at least five of B₁, B₂, ..., Bₙ₋₁, and Bₙ are independently selected from unnatural nucleobases represented by **Formula II**, **Formula III**, or **Formula IV**;
R₁, R₃, and R₅ are hydrido radical, and R₂, R₄, and R₆ independently represent hydrido, or substituted or non-substituted alkyl radical;
Q₁ and Qₘ are methylene radical, and Qₘ is directly linked to the basic amino group;
Q₂, Q₃, ..., and Qₘ₋₁ are independently selected from methylene, and oxygen radical; and,
m is an integer between 1 and 10.

6. The peptide nucleic acid derivative according to claim 1, or a pharmaceutical salt thereof:
wherein,
n is an integer between 12 and 16;
the compound of **Formula I** possesses at least a 10-mer complementary overlap with the 14-mer RNA sequence of [(5' → 3') UUUUUGCGUAAGUA] within the human SCN9A pre-mRNA;
the compound of **Formula I** is fully complementary to the target pre-mRNA sequence;
S₁, S₂, ..., Sₙ₋₁, Sn, T₁, T₂, ..., Tₙ₋₁, and Tn are hydrido radical;
X and Y independently represent hydrido [H], substituted or non-substituted alkylacyl, substituted or non-substituted arylacyl, or substituted or non-substituted alkyloxycarbonyl radical;
Z represents substituted or non-substituted amino radical;
B₁, B₂, ..., Bₙ₋₁, and Bₙ are independently selected from natural nucleobases including adenine, thymine, guanine and cytosine, and unnatural nucleobases;
at least five of B₁, B₂, ..., Bₙ₋₁, and Bₙ are independently selected from unnatural nucleobases represented by **Formula II**, **Formula III**, or **Formula IV**;
R₁, R₂ , R₃, R₄, R₅, and R₆ are hydrido radical;
Q₁ and Qₘ are methylene radical, and Qₘ is directly linked to the basic amino group;
Q₂, Q₃, ..., and Qₘ₋₁ are independently selected from methylene, and oxygen radical; and,
m is an integer between 1 and 10.

7. The peptide nucleic acid derivative according to claim 1, or a pharmaceutical salt thereof:
wherein,
n is an integer between 12 and 16;
the compound of **Formula I** possesses at least a 10-mer complementary overlap with the 14-mer RNA sequence of [(5' → 3') UUUUUGCGUAAGUA] within the human SCN9A pre-mRNA;
the compound of **Formula I** is fully complementary to the target pre-mRNA sequence;
S₁, S₂, ..., Sₙ₋₁, Sn, T₁, T₂, ..., Tₙ₋₁, and Tn are hydrido radical;
X is hydrido radical;
Y represents substituted or non-substituted alkylacyl, substituted or non-substituted arylacyl, or substituted or non-substituted alkyloxycarbonyl radical;
Z represents substituted or non-substituted amino radical;
B₁, B₂, ..., Bₙ₋₁, and Bₙ are independently selected from natural nucleobases including adenine, thymine, guanine and cytosine, and unnatural nucleobases;
at least five of B₁, B₂, ..., Bₙ₋₁, and Bₙ are independently selected from unnatural nucleobases represented by **Formula II**, **Formula III**, or **Formula IV**;
R₁, R₂ , R₃, R₄, R₅, and R₆ are hydrido radical;
L₁ represents -(CH₂)₂-O-(CH₂)₂-, -CH₂-O-(CH₂)₂-, -CH₂-O-(CH₂)₃-, -CH₂-O-(CH₂)₄-, or -CH₂-O-(CH₂)₅- with the right end being directly linked to the basic amino group; and,
L₂ and L₃ are independently selected from -(CH₂)₂-O-(CH₂)₂-, -(CH₂)₃-O-(CH₂)₂-,-(CH₂)₂-O-(CH₂)₃-, -(CH₂)₂-, -(CH₂)₃-, -(CH₂)₄-, -(CH₂)₅-, -(CH₂)₆-, -(CH₂)₇-, and -(CH₂)₈- with the right end being directly linked to the basic amino group.

8. The peptide nucleic acid derivative according to claim 1, which is selected from the group of peptide nucleic acid derivatives provided below, or a pharmaceutically acceptable salt thereof:
(N → C) Fmoc-TA(5)A-A(5)TA(5)-CGC(1O2)-AA(5)A-A(5)A-NH₂;
(N → C) Fethoc-TA(5)A-A(5)TA(5)-CGC(1O2)-AA(5)A-A(5)A-NH₂;
(N → C) Piv-TA(5)A-A(5)TA(5)-CGC(1O2)-AA(5)A-A(5)A-NH₂;
(N → C) FAM-HEX-TA(5)A-A(5)TA(5)-CGC(1O2)-AA(5)A-A(5)A-NH₂;
(N → C) Acetyl-TA(5)A-A(5)TA(5)-CGC(1O2)-AA(5)A-A(5)A-NH₂;
(N → C) Fethoc-Lys-TA(5)A-A(5)TA(5)-CGC(1O2)-AA(5)A-A(5)A-NH₂;
(N → C) H-TA(5)A-A(5)TA(5)-CGC(1O2)-AA(5)A-A(5)A-NH₂;
(N → C) Me-TA(5)A-A(5)TA(5)-CGC(1O2)-AA(5)A-A(5)A-NH₂;
(N → C) Benzyl-TA(5)A-A(5)TA(5)-CGC(1O2)-AA(5)A-A(5)A-NH₂;
(N → C) Fethoc-TA(5)A-A(5)TA(5)-CGC(1O2)-AA(5)A-A(5)A-Lys-NH₂;
(N → C) Fmoc-TA(5)A-A(5)TA(5)-CGC(1O2)-AA(5)A-A(5)AC-A(5)A-NH₂;
(N → C) Fethoc-TA(5)C-GC(1O2)A-A(5)AA(5)-ACA(5)-A-NH₂;
(N → C) Fethoc-TA(6)C-GC(1O2)A-A(6)AA(6)-ACA(6)-A-NH₂;
(N → C) Fethoc-AC(1O2)T-TA(5)C-G(6)CA-A(5)AA(5)-AC(1O2)A-A(5)-NH₂;
(N → C) Fethoc-AG(5)T-A(5)CT-TA(5)C-GC(1O2)A-A(5)AA(5)-ACA(5)-A-NH₂;
(N → C) Fethoc-AG(5)T-A(5)CT-TA(5)C-GC(1O2)A-A(5)AA(5)-A-NH₂;
(N → C) Fethoc-AG(5)T-A(5)CT-TA(5)C-GC(1O2)A-A(5)AA(2O2)-A-NH₂;
(N → C) Fethoc-Val-AG(5)T-A(5)CT-TA(5)C-GC(1O2)A-A(5)AA(2O2)-A-NH₂;
(N → C) Fethoc-Gly-AG(5)T-A(5)CT-TA(5)C-GC(1O2)A-A(5)AA(2O2)-A-NH₂;
(N → C) Fethoc-AG(5)T-A(5)CT-TA(5)C-GC(1O2)A-A(5)AA(2O2)-A-Lys-NH₂;
(N → C) Piv-AG(5)T-A(5)CT-TA(5)C-GC(1O2)A-A(5)AA(5)-A-NH₂;
(N → C) Fethoc-Lys-AG(5)T-A(5)CT-TA(5)C-GC(1O2)A-A(5)A-NH₂;
(N → C) Piv-Leu-AG(5)T-A(5)CT-TA(5)C-GC(1O2)A-A(5)AA(2O2)-A-NH₂;
(N → C) Fethoc-A(5)GT-A(5)CT-TA(5)C-G(6)CA(5)-A-NH₂;
(N → C) Fethoc-Lys-A(5)TC(1O3)-A(5)CT-TA(5)C-GC(1O2)A-A(5)A-NH₂;
(N → C) Fethoc-Gly-A(5)TC(1O3)-A(5)CT-TA(5)C-GC(1O2)A-A(5)A-Arg-NH₂;
(N → C) H-CTT-A(5)CG(3)- C(1O2)AA(5)-AA(5)A-C(1O3)AA(5)-NH₂;
(N → C) Fethoc-CTT-A(5)CG(6)-C(1O2)AA(5)-AA(5)A-C(1O2)AA(5)-NH₂;
(N → C) Fethoc-CTT-A(5)CG(6)-C(1O2)TA(5)-AA(5)T-C(1O2)AA(5)-NH₂;
(N → C) Benzoyl-CTT-A(5)CG(2O2)-C(1O2)AA(5)-AA(5)A-C(1O5)AA(5)-NH₂;
(N → C) n-Propyl-CTT-A(5)CG(2O3)-C(1O2)AA(3)-AA(5)A-C(2O2)AA(5)-NH₂;
(N → C) p-Toluenesulfonyl-CTT-A(5)CG(6)-C(1O2)AA(8)-AA(5)A-C(1O2)AA(5)-NH₂;
(N → C) [N-(2-Phenylethyl)amino]carbonyl-CTT-A(5)CG(6)-C(1O2)AA(2O2)-AA(5)A-C(1O2)A A(5)-NH₂;
(N → C) Fethoc-Lys-Leu-CTT-A(5)CG(6)-C(1O2)AA(4)-AA(5)A-C(1O2)AA(5)-Lys-NH₂;
(N → C) N-Phenyl-N-Me-CTT-A(5)CG(6)-C(1O2)AA(5)-AA(5)A-C(1O2)AA(5)-Lys-NH₂;
(N → C) Fethoc-AA(5)G-TA(5)C-TTA(5)-CG(6)C-A(5)A-NH₂; and,
(N → C) Fethoc-AA(5)G-TA(5)C-TTA(5)-CG(6)C-A(5)A-Lys-NH₂:
wherein,
A, G, T, and C are PNA monomers with a natural nucleobase of adenine, guanine, thymine, and cytosine, respectively;
C(pOq), A(p), A(pOq), G(p), and G(pOq) are PNA monomers with an unnatural nucleobase represented by **Formula VI**, **Formula VII**, **Formula VIII**, **Formula IX**, and **Formula X**, respectively: 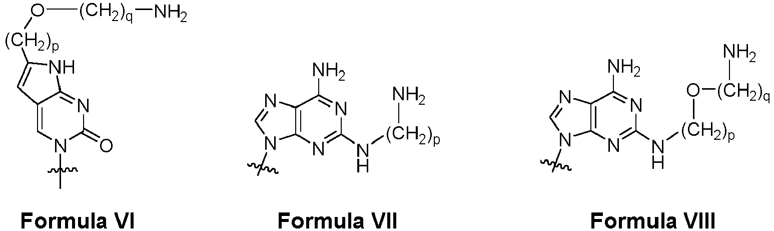 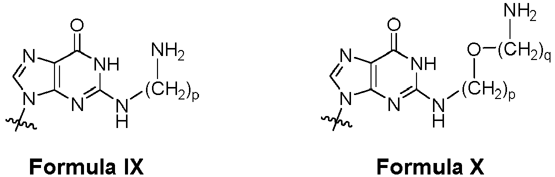
wherein,
p and q are integers; and,
the abbreviations for the N- and C-terminus substituents are as specifically described as follows: "Fmoc-" is the abbreviation for "[(9-fluorenyl)methyloxy]carbonyl-"; "Fethoc-" for "[2-(9-fluorenyl)ethyl-1-oxy]carbonyl"; "Ac-" for "acetyl-"; "Benzoyl-" for "benzenecabonyl-"; "Piv-" for "pivalyl-"; "Me-" for "methyl-"; "n-Propyl-" for "1-(n-propyl)-"; "H-" for "hydrido-" group; "p-Toluenesulfonyl" for "(4-methylbenzene)-1-sulfonyl-"; "-Lys-" for amino acid residue "lysine"; "-Val-" for amino acid residue "valine"; "-Leu-" for amino acid residue "leucine"; "-Arg-" for amino acid residue "arginine"; "-Gly-" for amino acid residue "glycine"; "[N-(2-Phenylethyl)amino]carbonyl-" for "[N-1-(2-phenylethyl)amino]carbonyl-"; "Benzyl-" for "1-(phenyl)methyl-"; "Phenyl-" for "phenyl-"; "Me-" for "methyl-"; "-HEX-" for "6-amino-1-hexanoyl-", "FAM-" for "5, or 6-fluorescein-carbonyl- (isomeric mixture)" ,and "-NH₂" for non-subsituted "-amino" group.

9. The peptide nucleic acid derivative according to claim 8, which is selected from the group of peptide nucleic acid derivatives provided below, or a pharmaceutically acceptable salt thereof:
(N → C) Fmoc-TA(5)A-A(5)TA(5)-CGC(1O2)-AA(5)A-A(5)A-NH₂;
(N → C) Fethoc-TA(5)A-A(5)TA(5)-CGC(1O2)-AA(5)A-A(5)A-NH₂;
(N → C) Fethoc-AC(1O2)T-TA(5)C-G(6)CA-A(5)AA(5)-AC(1O2)A-A(5)-NH₂; and
(N → C) Fmoc-TA(5)A-A(5)TA(5)-CGC(1O2)-AA(5)A-A(5)AC-A(5)A-NH₂.

10. A peptide nucleic acid derivative according to any one of claims 1 to 9, for use in the treatment of pain.

11. A peptide nucleic acid derivative according to any one of claims 1 to 9, for use according to claim 10 wherein the pain is chronic pain.

12. A peptide nucleic acid derivative according to any one of claims 1 to 9, for use according to claim 10 wherein the pain is neuropathic pain.

13. A peptide nucleic acid derivative according to any one of claims 1 to 9, for use in therapy.

14. A method of inducing skipping of exon 4 in the SCN9A pre-mRNA to yield a SCN9A mRNA splice variant lacking SCN9A exon 4 in cells *in vitro* comprising contacting the cells with the peptide nucleic acid derivative of any one of claims 1-9.

15. The method of claim 14, wherein the cells express a lower level of a full length SCN9A mRNA and show a lower Naᵥ1.7 functional activity than cells without contacting with the peptide nucleic acid derivative.

## Patentansprüche

1. Peptidnukleinsäurederivat, dargestellt durch **Formel I**, oder ein pharmazeutisch annehmbares Salz davon: 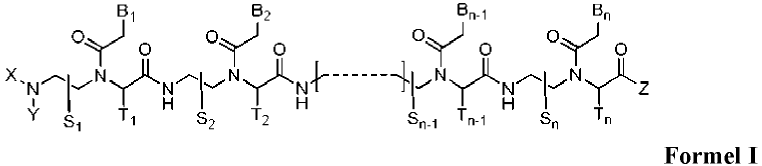 wobei
n eine ganze Zahl zwischen 10 und 21 ist;
die Verbindung der **Formel I** zumindest eine komplementäre 10-mer-Überlappung mit der 14-mer-RNA-Sequenz von [(5' → 3') UUUUUGCGUAAGUA] innerhalb der menschlichen SCN9A-prä-mRNA besitzt;
die Verbindung der **Formel I** vollständig komplementär zu der prä-mRNA-Zielsequenz oder teilweise komplementär zu der prä-mRNA-Zielsequenz mit einer oder zwei Fehlpaarungen ist;
S₁, S₂, ..., Sₙ₋₁, Sn, T₁, T₂, ..., Tₙ₋₁ und Tn unabhängig Deuterido-, Hydrido-, substituiertes oder nicht substituiertes Alkyl- oder substituiertes oder nicht substituiertes Aryl-Radikal darstellen;
X und Y unabhängig Hydrido- [H], Formyl- [H-C(=O)-], Aminocarbonyl- [NH₂-C(=O)-], substituiertes oder nicht substituiertes Alkyl-, substituiertes oder nicht substituiertes Aryl-, substituiertes oder nicht substituiertes Alkylacyl-, substituiertes oder nicht substituiertes Arylacyl-, substituiertes oder nicht substituiertes Alkyloxycarbonyl-, substituiertes oder nicht substituiertes Aryloxycarbonyl-, substituiertes oder nicht substituiertes Alkylaminocarbonyl-, substituiertes oder nicht substituiertes Arylaminocarbonyl-, substituiertes oder nicht substituiertes Alkylsulfonyl- oder substituiertes oder nicht substituiertes Arylsulfonyl-Radikal darstellen;
Z Hydroxy-, substituiertes oder nicht substituiertes Alkyloxy-, substituiertes oder nicht substituiertes Aryloxy-, substituiertes oder nicht substituiertes Amino-, substituiertes oder nicht substituiertes Alkyl- oder substituiertes oder nicht substituiertes Aryl-Radikal darstellt;
B₁, B₂, ..., Bₙ₋₁ und Bₙ unabhängig aus natürlichen Nukleobasen, die Adenin, Thymin, Guanin, Cytosin und Uracil beinhalten, und unnatürlichen Nukleobasen ausgewählt sind; und
zumindest vier von B₁, B₂, ..., Bₙ₋₁ und Bₙ unabhängig aus unnatürlichen Nukleobasen mit einem substituierten oder nicht substituierten Amino-Radikal ausgewählt sind, das kovalent mit der Nukleobaseneinheit verknüpft ist.

2. Peptidnukleinsäurederivat nach Anspruch 1 oder ein pharmazeutisches Salz davon:
wobei
n eine ganze Zahl zwischen 10 und 21 ist;
die Verbindung der **Formel I** zumindest eine komplementäre 10-mer-Überlappung mit der 14-mer-RNA-Sequenz von [(5' → 3') UUUUUGCGUAAGUA] innerhalb der menschlichen SCN9A-prä-mRNA besitzt;
die Verbindung der **Formel I** vollständig komplementär zu der prä-mRNA-Zielsequenz oder teilweise komplementär zu der prä-mRNA-Zielsequenz mit einer oder zwei Fehlpaarungen ist;
S₁, S₂, ..., Sₙ₋₁, Sn, T₁, T₂, ..., Tₙ₋₁ und Tn unabhängig Hydrido-Radikal darstellen;
X und Y unabhängig Hydrido- [H], Formyl- [H-C(=O)-], Aminocarbonyl- [NH₂-C(=O)-], substituiertes oder nicht substituiertes Alkyl-, substituiertes oder nicht substituiertes Aryl-, substituiertes oder nicht substituiertes Alkylacyl-, substituiertes oder nicht substituiertes Arylacyl-, substituiertes oder nicht substituiertes Alkyloxycarbonyl-, substituiertes oder nicht substituiertes Aryloxycarbonyl-, substituiertes oder nicht substituiertes Alkylaminocarbonyl-, substituiertes oder nicht substituiertes Arylaminocarbonyl-, substituiertes oder nicht substituiertes Alkylsulfonyl- oder substituiertes oder nicht substituiertes Arylsulfonyl-Radikal darstellen;
Z Hydroxy-, substituiertes oder nicht substituiertes Alkyloxy-, substituiertes oder nicht substituiertes Aryloxy-, substituiertes oder nicht substituiertes Amino-, substituiertes oder nicht substituiertes Alkyl- oder substituiertes oder nicht substituiertes Aryl-Radikal darstellt;
B₁, B₂, ..., Bₙ₋₁ und Bₙ unabhängig aus natürlichen Nukleobasen, die Adenin, Thymin, Guanin, Cytosin und Uracil beinhalten, und unnatürlichen Nukleobasen ausgewählt sind;
zumindest drei von B₁, B₂, ..., Bₙ₋₁, and Bₙ unabhängig aus unnatürlichen Nukleobasen ausgewählt sind, die durch **Formel II**, **Formel III** oder **Formel IV** dargestellt sind: 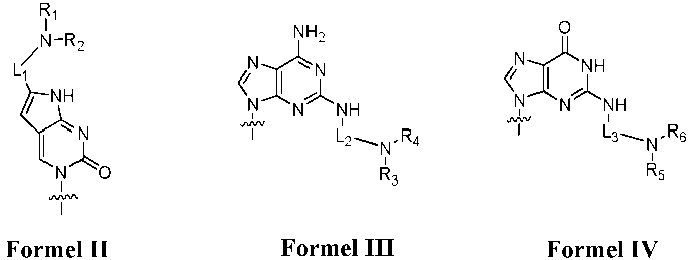
wobei
R₁, R₂, R₃, R₄, R₅ und R₆ unabhängig aus Hydrido- und substituiertem oder nicht substituiertem Alkyl-Radikal ausgewählt sind; und
L₁, L₂ und L₃ ein kovalenter Linker sind, der durch **Formel V** dargestellt ist, der die basische Aminogruppe mit der Nukleobaseneinheit verbindet, die für Nukleobasenpaarung verantwortlich ist: 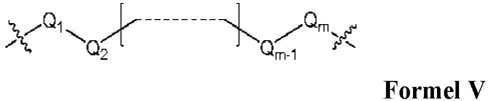
wobei
Q₁ und Qₘ substituiertes oder nicht substituiertes Methylen-(-CH₂-)Radikal sind, und Qₘ direkt mit der basischen Aminogruppe verknüpft ist;
Q₂, Q₃, ..., und Qₘ₋₁ unabhängig aus substituiertem oder nicht substituiertem Methylen-, Sauerstoff- (-O-), Schwefel- (-S-) und substituiertem oder nicht substituiertem Amino-Radikal [-N(H)- oder -N(Substituent)-] ausgewählt sind; und
m eine ganze Zahl zwischen 1 und 16 ist.

3. Peptidnukleinsäurederivat nach Anspruch 1 oder ein pharmazeutisches Salz davon:
wobei
n eine ganze Zahl zwischen 12 und 20 ist;
die Verbindung der **Formel I** zumindest eine komplementäre 10-mer-Überlappung mit der 14-mer-RNA-Sequenz von [(5' → 3') UUUUUGCGUAAGUA] innerhalb der menschlichen SCN9A-prä-mRNA besitzt;
die Verbindung der **Formel I** vollständig komplementär zu der prä-mRNA-Zielsequenz oder teilweise komplementär zu der prä-mRNA-Zielsequenz mit einer oder zwei Fehlpaarungen ist;
S₁, S₂, ..., Sₙ₋₁, Sn, T₁, T₂, ..., Tₙ₋₁ und Tₙ Hydrido-Radikal sind;
X und Y unabhängig Hydrido- [H], Aminocarbonyl- [NH₂-C(=O)-], substituiertes oder nicht substituiertes Alkyl-, substituiertes oder nicht substituiertes Aryl-, substituiertes oder nicht substituiertes Alkylacyl-, substituiertes oder nicht substituiertes Arylacyl-, substituiertes oder nicht substituiertes Alkyloxycarbonyl-, substituiertes oder nicht substituiertes Alkylaminocarbonyl- oder substituiertes oder nicht substituiertes Arylsulfonyl-Radikal darstellen;
Z substituiertes oder nicht substituiertes Amino-Radikal darstellt;
B₁, B₂, ..., Bₙ₋₁ und Bₙ unabhängig aus natürlichen Nukleobasen, die Adenin, Thymin, Guanin und Cytosin beinhalten, und unnatürlichen Nukleobasen ausgewählt sind;
zumindest vier von B₁, B₂, ..., Bₙ₋₁ und Bₙ unabhängig aus unnatürlichen Nukleobasen ausgewählt sind, die durch **Formel II**, **Formel III** oder **Formel IV** dargestellt sind;
R₁, R₂, R₃, R₄, R₅ und R₆ unabhängig aus Hydrido- und substituiertem oder nicht substituiertem Alkyl-Radikal ausgewählt sind;
Q₁ und Qₘ substituiertes oder nicht substituiertes Methylen-Radikal sind, und Qₘ direkt mit der basischen Aminogruppe verknüpft ist;
Q₂, Q₃, ..., und Qₘ₋₁ unabhängig aus substituiertem oder nicht substituiertem Methylen-, Sauerstoff- und Amino-Radikal ausgewählt sind; und
m eine ganze Zahl zwischen 1 und 11 ist.

4. Peptidnukleinsäurederivat nach Anspruch 1 oder ein pharmazeutisches Salz davon:
wobei
n eine ganze Zahl zwischen 12 und 19 ist;
die Verbindung der **Formel I** zumindest eine komplementäre 10-mer-Überlappung mit der 14-mer-RNA-Sequenz von [(5' → 3') UUUUUGCGUAAGUA] innerhalb der menschlichen SCN9A-prä-mRNA besitzt;
die Verbindung der **Formel I** vollständig komplementär zu der prä-mRNA-Zielsequenz ist;
S₁, S₂, ..., Sₙ₋₁, Sn, T₁, T₂, ..., Tₙ₋₁ und Tn Hydrido-Radikal sind;
X und Y unabhängig Hydrido- [H], substituiertes oder nicht substituiertes Alkylacyl-, substituiertes oder nicht substituiertes Arylacyl-, substituiertes oder nicht substituiertes Alkyloxycarbonyl- oder substituiertes oder nicht substituiertes Alkylaminocarbonyl-Radikal darstellen;
Z substituiertes oder nicht substituiertes Amino-Radikal darstellt;
B₁, B₂, ..., Bₙ₋₁ und Bₙ unabhängig aus natürlichen Nukleobasen, die Adenin, Thymin, Guanin und Cytosin beinhalten, und unnatürlichen Nukleobasen ausgewählt sind;
zumindest vier von B₁, B₂, ..., Bₙ₋₁ und Bₙ unabhängig aus unnatürlichen Nukleobasen ausgewählt sind, die durch **Formel II**, **Formel III** oder **Formel IV** dargestellt sind;
R₁, R₂, R₃, R₄, R₅ und R₆ unabhängig aus Hydrido- und substituiertem oder nicht substituiertem Alkyl-Radikal ausgewählt sind;
Q₁ und Qₘ Methylen-Radikal sind, und Qₘ direkt mit der basischen Aminogruppe verknüpft ist;
Q₂, Q₃, ..., und Qₘ₋₁ unabhängig aus Methylen- und Sauerstoff-Radikal ausgewählt sind; und
m eine ganze Zahl zwischen 1 und 10 ist.

5. Peptidnukleinsäurederivat nach Anspruch 1 oder ein pharmazeutisches Salz davon:
wobei
n eine ganze Zahl zwischen 12 und 18 ist;
die Verbindung der **Formel I** zumindest eine komplementäre 10-mer-Überlappung mit der 14-mer-RNA-Sequenz von [(5' → 3') UUUUUGCGUAAGUA] innerhalb der menschlichen SCN9A-prä-mRNA besitzt;
die Verbindung der **Formel I** vollständig komplementär zu der prä-mRNA-Zielsequenz ist;
S₁, S₂, ..., Sₙ₋₁, Sn, T₁, T₂, ..., Tₙ₋₁ und Tn Hydrido-Radikal sind;
X und Y unabhängig Hydrido- [H], substituiertes oder nicht substituiertes Alkylacyl-, substituiertes oder nicht substituiertes Arylacyl- oder substituiertes oder nicht substituiertes Alkyloxycarbonyl-Radikal darstellen;
Z substituiertes oder nicht substituiertes Amino-Radikal darstellt;
B₁, B₂, ..., Bₙ₋₁ und Bₙ unabhängig aus natürlichen Nukleobasen, die Adenin, Thymin, Guanin und Cytosin beinhalten, und unnatürlichen Nukleobasen ausgewählt sind;
zumindest fünf von B₁, B₂, ..., Bₙ₋₁ und Bₙ unabhängig aus unnatürlichen Nukleobasen ausgewählt sind, die durch **Formel II**, **Formel III** oder **Formel IV** dargestellt sind;
R₁, R₃ und R₅ Hydrido-Radikal sind, und R₂, R₄ und R₆ unabhängig Hydrido- oder substituiertes oder nicht substituiertes Alkyl-Radikal darstellen;
Q₁ und Qₘ Methylen-Radikal sind, und Qₘ direkt mit der basischen Aminogruppe verknüpft ist;
Q₂, Q₃, ..., und Qₘ₋₁ unabhängig aus Methylen- und Sauerstoff-Radikal ausgewählt sind; und
m eine ganze Zahl zwischen 1 und 10 ist.

6. Peptidnukleinsäurederivat nach Anspruch 1 oder ein pharmazeutisches Salz davon:
wobei
n eine ganze Zahl zwischen 12 und 16 ist;
die Verbindung der **Formel I** zumindest eine komplementäre 10-mer-Überlappung mit der 14-mer-RNA-Sequenz von [(5' → 3') UUUUUGCGUAAGUA] innerhalb der menschlichen SCN9A-prä-mRNA besitzt;
die Verbindung der **Formel I** vollständig komplementär zu der prä-mRNA-Zielsequenz ist;
S₁, S₂, Sₙ₋₁, Sn, T₁, T₂, Tₙ₋₁ und Tn Hydrido-Radikal sind;
X und Y unabhängig Hydrido- [H], substituiertes oder nicht substituiertes Alkylacyl-, substituiertes oder nicht substituiertes Arylacyl- oder substituiertes oder nicht substituiertes Alkyloxycarbonyl-Radikal darstellen;
Z substituiertes oder nicht substituiertes Amino-Radikal darstellt;
B₁, B₂, ..., Bₙ₋₁ und Bₙ unabhängig aus natürlichen Nukleobasen, die Adenin, Thymin, Guanin und Cytosin beinhalten, und unnatürlichen Nukleobasen ausgewählt sind;
zumindest fünf von B₁, B₂, ..., Bₙ₋₁ und Bₙ unabhängig aus unnatürlichen Nukleobasen ausgewählt sind, die durch **Formel II**, **Formel III** oder **Formel IV** dargestellt sind;
R₁, R₂, R₃, R₄, R₅ und R₆ Hydrido-Radikal sind;
Q₁ und Qₘ Methylen-Radikal sind, und Qₘ direkt mit der basischen Aminogruppe verknüpft ist;
Q₂, Q₃, ..., und Qₘ₋₁ unabhängig aus Methylen- und Sauerstoff-Radikal ausgewählt sind; und
m eine ganze Zahl zwischen 1 und 10 ist.

7. Peptidnukleinsäurederivat nach Anspruch 1 oder ein pharmazeutisches Salz davon:
wobei
n eine ganze Zahl zwischen 12 und 16 ist;
die Verbindung der **Formel I** zumindest eine komplementäre 10-mer-Überlappung mit der 14-mer-RNA-Sequenz von [(5' → 3') UUUUUGCGUAAGUA] innerhalb der menschlichen SCN9A-prä-mRNA besitzt;
die Verbindung der **Formel I** vollständig komplementär zu der prä-mRNA-Zielsequenz ist;
S₁, S₂, ..., Sₙ₋₁, Sn, T₁, T₂, ..., Tₙ₋₁ und Tn Hydrido-Radikal sind;
X Hydrido-Radikal ist;
Y substituiertes oder nicht substituiertes Alkylacyl-, substituiertes oder nicht substituiertes Arylacyl- oder substituiertes oder nicht substituiertes Alkyloxycarbonyl-Radikal darstellt;
Z substituiertes oder nicht substituiertes Amino-Radikal darstellt;
B₁, B₂, ..., Bₙ₋₁ und Bₙ unabhängig aus natürlichen Nukleobasen, die Adenin, Thymin, Guanin und Cytosin beinhalten, und unnatürlichen Nukleobasen ausgewählt sind;
zumindest fünf von B₁, B₂, ..., Bₙ₋₁ und Bₙ unabhängig aus unnatürlichen Nukleobasen ausgewählt sind, die durch **Formel II**, **Formel III** oder **Formel IV** dargestellt sind;
R₁, R₂, R₃, R₄, R₅ und R₆ Hydrido-Radikal sind;
L₁ -(CH₂)₂-O-(CH₂)₂-, -CH₂-O-(CH₂)₂-, -CH₂-O-(CH₂)₃-, -CH₂-O-(CH₂)₄- oder -CH₂-O-(CH₂)₅- darstellt, wobei das rechte Ende direkt mit der basischen Aminogruppe verknüpft ist; und
L₂ und L₃ unabhängig aus -(CH₂)₂-O-(CH₂)₂-, -(CH₂)₃-O-(CH₂)₂-, -(CH₂)₂-O-(CH₂)₃-,-(CH₂)₂-, -(CH₂)₃-, -(CH₂)₄-, -(CH₂)₅-, -(CH₂)₆-, -(CH₂)₇- und -(CH₂)₈- ausgewählt sind, wobei das rechte Ende direkt mit der basischen Aminogruppe verknüpft ist.

8. Peptidnukleinsäurederivat nach Anspruch 1, das aus der Gruppe aus Peptidnukleinsäurederivaten ausgewählt ist, die nachfolgend bereitgestellt ist, oder einem pharmazeutisch annehmbaren Salz davon:
(N → C) Fmoc-TA(5)A-A(5)TA(5)-CGC(1O2)-AA(5)A-A(5)A-NH₂;
(N → C) Fethoc-TA(5)A-A(5)TA(5)-CGC(1O2)-AA(5)A-A(5)A-NH₂;
(N → C) Piv-TA(5)A-A(5)TA(5)-CGC(1O2)-AA(5)A-A(5)A-NH₂;
(N → C) FAM-HEX-TA(5)A-A(5)TA(5)-CGC(1O2)-AA(5)A-A(5)A-NH₂;
(N → C) Acetyl-TA(5)A-A(5)TA(5)-CGC(1O2)-AA(5)A-A(5)A-NH₂;
(N → C) Fethoc-Lys-TA(5)A-A(5)TA(5)-CGC(1O2)-AA(5)A-A(5)A-NH₂;
(N → C) H-TA(5)A-A(5)TA(5)-CGC(1O2)-AA(5)A-A(5)A-NH₂;
(N → C) Me-TA(5)A-A(5)TA(5)-CGC(1O2)-AA(5)A-A(5)A-NH₂;
(N → C) Benzyl-TA(5)A-A(5)TA(5)-CGC(1O2)-AA(5)A-A(5)A-NH₂;
(N → C) Fethoc-TA(5)A-A(5)TA(5)-CGC(1O2)-AA(5)A-A(5)A-Lys-NH₂;
(N → C) Fmoc-TA(5)A-A(5)TA(5)-CGC(1O2)-AA(5)A-A(5)AC-A(5)A-NH₂;
(N → C) Fethoc-TA(5)C-GC(1O2)A-A(5)AA(5)-ACA(5)-A-NH₂;
(N → C) Fethoc-TA(6)C-GC(1O2)A-A(6)AA(6)-ACA(6)-A-NH₂;
(N → C) Fethoc-AC(1O2)T-TA(5)C-G(6)CA-A(5)AA(5)-AC(1O2)A-A(5)-NH₂;
(N → C) Fethoc-AG(5)T-A(5)CT-TA(5)C-GC(1O2)A-A(5)AA(5)-ACA(5)-A-NH₂;
(N → C) Fethoc-AG(5)T-A(5)CT-TA(5)C-GC(1O2)A-A(5)AA(5)-A-NH₂;
(N → C) Fethoc-AG(5)T-A(5)CT-TA(5)C-GC(1O2)A-A(5)AA(2O2)-A-NH₂;
(N → C) Fethoc-Val-AG(5)T-A(5)CT-TA(5)C-GC(1O2)A-A(5)AA(2O2)-A-NH₂;
(N → C) Fethoc-Gly-AG(5)T-A(5)CT-TA(5)C-GC(1O2)A-A(5)AA(2O2)-A-NH₂;
(N → C) Fethoc-AG(5)T-A(5)CT-TA(5)C-GC(1O2)A-A(5)AA(2O2)-A-Lys-NH₂;
(N → C) Piv-AG(5)T-A(5)CT-TA(5)C-GC(1O2)A-A(5)AA(5)-A-NH₂;
(N → C) Fethoc-Lys-AG(5)T-A(5)CT-TA(5)C-GC(1O2)A-A(5)A-NH₂;
(N → C) Piv-Leu-AG(5)T-A(5)CT-TA(5)C-GC(1O2)A-A(5)AA(2O2)-A-NH₂;
(N → C) Fethoc-A(5)GT-A(5)CT-TA(5)C-G(6)CA(5)-A-NH₂;
(N → C) Fethoc-Lys-A(5)TC(1O3)-A(5)CT-TA(5)C-GC(1O2)A-A(5)A-NH₂;
(N → C) Fethoc-Gly-A(5)TC(1O3)-A(5)CT-TA(5)C-GC(1O2)A-A(5)A-Arg-NH₂;
(N → C) H-CTT-A(5)CG(3)-C(1O2)AA(5)-AA(5)A-C(1O3)AA(5)-NH₂;
(N → C) Fethoc-CTT-A(5)CG(6)-C(1O2)AA(5)-AA(5)A-C(1O2)AA(5)-NH₂;
(N → C) Fethoc-CTT-A(S)CG(6)-C(1O2)TA(S)-AA(S)T-C(1O2)AA(S)-NH₂
(N → C) Benzoyl-CTT-A(5)CG(2O2)-C(1O2)AA(5)-AA(5)A-C(1O5)AA(5)-NH₂;
(N → C) n-Propyl-CTT-A(5)CG(2O3)-C(1O2)AA(3)-AA(5)A-C(2O2)AA(5)-NH₂;
(N → C) p-Toluolsulfonyl-CTT-A(5)CG(6)-C(1O2)AA(8)-AA(5)A-C(1O2)AA(5)-NH₂;
(N → C) [N-(2-Phenylethyl)amino]carbonyl-CTT-A(5)CG(6)-C(1O2)AA(2O2)-AA(5)A-C(1O2)A A(5)-NH₂;
(N → C) Fethoc-Lys-Leu-CTT-A(5)CG(6)-C(1O2)AA(4)-AA(5)A-C(1O2)AA(5)-Lys-NH₂;
(N → C) N-Phenyl-N-Me-CTT-A(5)CG(6)-C(1O2)AA(5)-AA(5)A-C(1O2)AA(5)-Lys-NH₂;
(N → C) Fethoc-AA(5)G-TA(5)C-TTA(5)-CG(6)C-A(5)A-NH₂; und
(N → C) Fethoc-AA(5)G-TA(5)C-TTA(5)-CG(6)C-A(5)A-Lys-NH₂:
wobei
A, G, T und C jeweils PNA-Monomere mit einer natürlichen Nukleobase von Adenin, Guanin, Thymin und Cytosin sind;
C(pOq), A(p), A(pOq), G(p) und G(pOq) PNA-Monomere mit einer unnatürlichen Nukleobase sind, die jeweils durch **Formel VI, Formel VII, Formel VIII, Formel** IX und Formel X dargestellt sind: 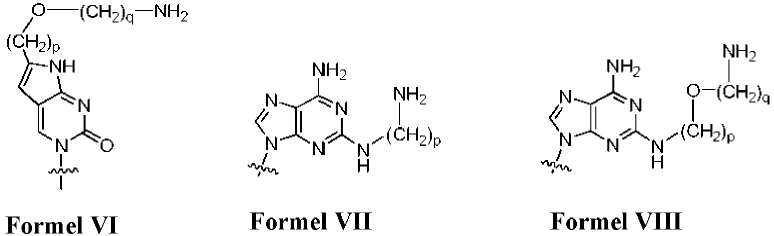 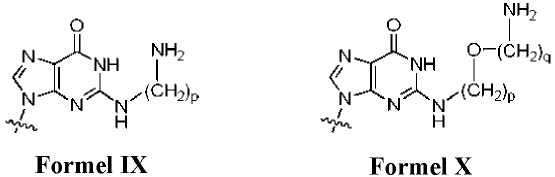
wobei
p und q ganze Zahlen sind; und
die Abkürzungen für die Substituenten am N- und C-Terminus wie folgt spezifisch beschrieben sind: "Fmoc-" ist die Abkürzung für "[(9-Fluorenyl)methyloxy]carbonyl-"; "Fethoc-" für "[2-(9-Fluorenyl)ethyl-1-oxy]carbonyl"; "Ac-" für "Acetyl-"; "Benzoyl-" für "Benzolcabonyl-"; "Piv-" für "Pivalyl-"; "Me-" für "Methyl-"; "n-Propyl-" für "1-(n-Propyl)-"; "H-" für "Hydrido-"Gruppe; "p-Toluolsulfonyl" für "(4-Methylbenzol)-1-sulfonyl-"; "-Lys-" für Aminosäurerest "Lysin"; "-Val-" für Aminosäurerest "Valin"; "-Leu-" für Aminosäurerest "Leucin"; "-Arg-" für Aminosäurerest "Arginin"; "-Gly-" für Aminosäurerest "Glycin"; "[N-(2-Phenylethyl)amino]carbonyl-" für "[N-1-(2-Phenylethyl)amino]carbonyl-"; "Benzyl-" für "1-(Phenyl)methyl-"; "Phenyl-" für "Phenyl-"; "Me-" für "Methyl-"; "-HEX-" für "6-Amino-1-hexanoyl-", "FAM-" für "5- oder 6-Fluorescein-carbonyl- (Isomerengemisch)" und "-NH₂" für nicht substituierte "-Amino"Gruppe.

9. Peptidnukleinsäurederivat nach Anspruch 8, das aus der Gruppe von Peptidnukleinsäurederivaten ausgewählt ist, die nachfolgend bereitgestellt ist, oder einem pharmazeutisch annehmbaren Salz davon:
(N → C) Fmoc-TA(5)A-A(5)TA(5)-CGC(1O2)-AA(5)A-A(5)A-NH₂;
(N → C) Fethoc-TA(5)A-A(5)TA(5)-CGC(1O2)-AA(5)A-A(5)A-NH₂;
(N → C) Fethoc-AC(1O2)T-TA(5)C-G(6)CA-A(5)AA(5)-AC(1O2)A-A(5)-NH₂; und
(N → C) Fmoc-TA(5)A-A(5)TA(5)-CGC(1O2)-AA(5)A-A(5)AC-A(5)A-NH₂.

10. Peptidnukleinsäurederivat nach einem der Ansprüche 1 bis 9 zur Verwendung bei der Behandlung von Schmerzen.

11. Peptidnukleinsäurederivat nach einem der Ansprüche 1 bis 9 zur Verwendung nach Anspruch 10, wobei die Schmerzen chronische Schmerzen sind.

12. Peptidnukleinsäurederivat nach einem der Ansprüche 1 bis 9 zur Verwendung nach Anspruch 10, wobei die Schmerzen neuropathische Schmerzen sind.

13. Peptidnukleinsäurederivat nach einem der Ansprüche 1 bis 9 zur Verwendung in der Therapie.

14. Verfahren zum Induzieren von Überspringen von Exon 4 in der SCN9A-prä-mRNA, um eine SCN9A-mRNA-Spleißvariante zu erhalten, der SCN9A-Exon 4 in Zellen fehlt, *in vitro,* umfassend das Kontaktieren der Zellen mit dem Peptidnukleinsäurederivat nach einem der Ansprüche 1-9.

15. Verfahren nach Anspruch 14, wobei die Zellen einen geringeren Spiegel einer SCN9A-mRNA voller Länge exprimieren und eine geringere funktionelle Naᵥ1.7-Aktivität zeigen als Zellen ohne Kontakt mit dem Peptidnukleinsäurederivat.

## Revendications

1. Dérivé d'acide nucléique peptidique représenté par la **formule I**, ou sel pharmaceutiquement acceptable de celui-ci : 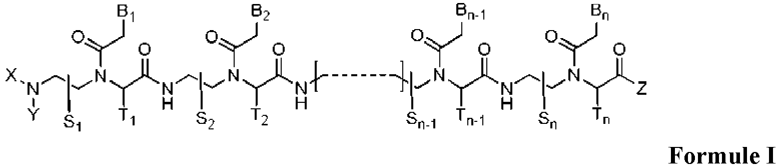 dans laquelle,
n représente un entier compris entre 10 et 21 ;
le composé de **formule I** possède au moins un chevauchement complémentaire 10-mère avec la séquence d'ARN 14-mère de [(5' → 3') UUUUUGCGUAAGUA] au sein du pré-ARNm de SCN9A humain ;
le composé de **formule I** est totalement complémentaire à la séquence de pré-ARNm cible, ou partiellement complémentaire à la séquence de pré-ARNm cible avec un ou deux mésappariements ;
S₁, S₂, ..., Sₙ₋₁, Sn, T₁, T₂, ..., Tₙ₋₁ et Tn représentent indépendamment un radical deutérido, hydrido, alkyle substitué ou non substitué, ou aryle substitué ou non substitué ;
X et Y représentent indépendamment un radical hydrido [H], formyle [H-C(=O)-], aminocarbonyle [NH₂-C(=O)-], alkyle substitué ou non substitué, aryle substitué ou non substitué, alkylacyle substitué ou non substitué, arylacyle substitué ou non substitué, alkyloxycarbonyle substitué ou non substitué, aryloxycarbonyle substitué ou non substitué, alkylaminocarbonyle substitué ou non substitué, arylaminocarbonyle substitué ou non substitué, alkylsulfonyle substitué ou non substitué, ou arylsulfonyle substitué ou non substitué ;
Z représente un radical hydroxy, alkyloxy substitué ou non substitué, aryloxy substitué ou non substitué, amino substitué ou non substitué, alkyle substitué ou non substitué, ou aryle substitué ou non substitué ;
B₁, B₂, ..., Bₙ₋₁ et Bₙ sont indépendamment choisis parmi les nucléobases naturelles comprenant l'adénine, la thymine, la guanine, la cytosine et l'uracile et les nucléobases non naturelles ; et,
au moins quatre des groupes B₁, B₂, ..., Bₙ₋₁ et Bₙ sont indépendamment choisis parmi les nucléobases non naturelles avec un radical amino substitué ou non substitué lié par covalence au groupement nucléobase.

2. Dérivé d'acide nucléique peptidique selon la revendication 1, ou sel pharmaceutiquement acceptable de celui-ci :
n représentant un entier compris entre 10 et 21 ;
le composé de **formule I** possédant au moins un chevauchement complémentaire 10-mère avec la séquence d'ARN 14-mère de [(5' → 3') UUUUUGCGUAAGUA] au sein du pré-ARNm de SCN9A humain ;
le composé de **formule I** étant totalement complémentaire à la séquence de pré-ARNm cible, ou partiellement complémentaire à la séquence de pré-ARNm cible avec un ou deux mésappariements ;
S₁, S₂, ..., Sₙ₋₁, Sn, T₁, T₂, ..., Tₙ₋₁ et Tn représentant indépendamment un radical hydrido ;
X et Y représentant indépendamment un radical hydrido [H], formyle [H-C(=O)-], aminocarbonyle [NH₂-C(=O)-], alkyle substitué ou non substitué, aryle substitué ou non substitué, alkylacyle substitué ou non substitué, arylacyle substitué ou non substitué, alkyloxycarbonyle substitué ou non substitué, aryloxycarbonyle substitué ou non substitué, alkylaminocarbonyle substitué ou non substitué, arylaminocarbonyle substitué ou non substitué, alkylsulfonyle substitué ou non substitué, ou arylsulfonyle substitué ou non substitué ;
Z représentant un radical hydroxy, alkyloxy substitué ou non substitué, aryloxy substitué ou non substitué, amino substitué ou non substitué, alkyle substitué ou non substitué, ou aryle substitué ou non substitué ;
B₁, B₂, ..., Bₙ₋₁ et Bₙ étant indépendamment choisis parmi les nucléobases naturelles comprenant l'adénine, la thymine, la guanine, la cytosine et l'uracile et les nucléobases non naturelles ;
au moins trois des groupes B₁, B₂, ..., Bₙ₋₁ et Bₙ étant indépendamment choisis parmi les nucléobases non naturelles représentées par **la formule II, la formule III** ou **la formule IV** : 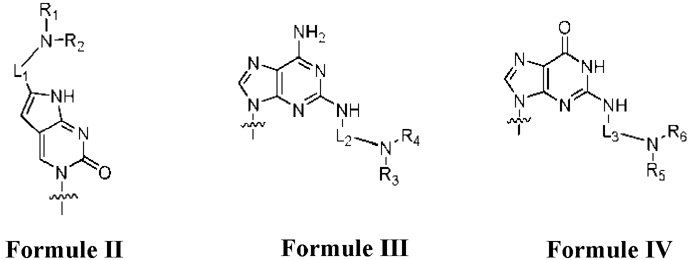
dans lesquelles,
R₁, R₂, R₃, R₄, R₅ et R₆ sont indépendamment choisis parmi le radical hydrido et alkyle substitué ou non substitué ; et,
L₁, L₂ et L₃ représentent un lieur covalent représenté par la **formule V** reliant le groupe amino basique au groupement nucléobase responsable de l'appariement des nucléobases : 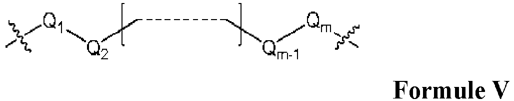
dans laquelle,
Q₁ et Qₘ représentent un radical méthylène substitué ou non substitué (-CH₂-) et Qₘ est directement lié au groupe amino basique ;
Q₂, Q₃, ..., et Qₘ₋₁ sont indépendamment choisis parmi un radical méthylène substitué ou non substitué, oxygène (-O-), soufre (-S-) et amino substitué ou non substitué [-N(H)- ou - N(substituant)-] ; et
m représente un entier compris entre 1 et 16.

3. Dérivé d'acide nucléique peptidique selon la revendication 1, ou sel pharmaceutiquement acceptable de celui-ci :
n représentant un entier compris entre 12 et 20 ;
le composé de **formule I** possédant au moins un chevauchement complémentaire 10-mère avec la séquence d'ARN 14-mère de [(5' → 3') UUUUUGCGUAAGUA] au sein du pré-ARNm de SCN9A humain ;
le composé de **formule I** étant totalement complémentaire à la séquence de pré-ARNm cible, ou partiellement complémentaire à la séquence de pré-ARNm cible avec un ou deux mésappariements ;
S₁, S₂, ..., Sₙ₋₁, Sn, T₁, T₂, ..., Tₙ₋₁ et Tn représentant un radical hydrido ;
X et Y représentant indépendamment un radical hydrido [H], aminocarbonyle [NH₂-C(=O)-], alkyle substitué ou non substitué, aryle substitué ou non substitué, alkylacyle substitué ou non substitué, arylacyle substitué ou non substitué, alkyloxycarbonyle substitué ou non substitué, alkylaminocarbonyle substitué ou non substitué, ou arylsulfonyle substitué ou non substitué ;
Z représentant un radical amino substitué ou non substitué ;
B₁, B₂, ..., Bₙ₋₁ et Bₙ étant indépendamment choisis parmi les nucléobases naturelles comprenant l'adénine, la thymine, la guanine et la cytosine et les nucléobases non naturelles ;
au moins quatre des groupes B₁, B₂, ..., Bₙ₋₁ et Bₙ étant indépendamment choisis parmi les nucléobases non naturelles représentées par la **formule II, la formule III** ou **la formule IV ;**
R₁, R₂, R₃, R₄, R₅ et R₆ étant indépendamment choisis parmi un radical hydrido et alkyle substitué ou non substitué ;
Q₁ et Qₘ représentant un radical méthylène substitué ou non substitué et Qₘ étant directement lié au groupe amino basique ;
Q₂, Q₃, ..., et Qₘ₋₁ étant indépendamment choisis parmi un radical méthylène substitué ou non substitué, oxygène et amino ; et
m représentant un entier compris entre 1 et 11.

4. Dérivé d'acide nucléique peptidique selon la revendication 1, ou sel pharmaceutiquement acceptable de celui-ci :
n représentant un entier compris entre 12 et 19 ;
le composé de **formule I** possédant au moins un chevauchement complémentaire 10-mère avec la séquence d'ARN 14-mère de [(5' → 3') UUUUUGCGUAAGUA] au sein du pré-ARNm de SCN9A humain ;
le composé de **formule I** étant totalement complémentaire à la séquence de pré-ARNm cible ;
S₁, S₂, ..., Sₙ₋₁, Sn, T₁, T₂, ..., Tₙ₋₁ et Tn représentant un radical hydrido ;
X et Y représentant indépendamment un radical hydrido [H], alkylacyle substitué ou non substitué, arylacyle substitué ou non substitué, alkyloxycarbonyle substitué ou non substitué, ou alkylaminocarbonyle substitué ou non substitué ;
Z représentant un radical amino substitué ou non substitué ;
B₁, B₂, ..., Bₙ₋₁ et Bₙ étant indépendamment choisis parmi les nucléobases naturelles comprenant l'adénine, la thymine, la guanine et la cytosine et les nucléobases non naturelles ;
au moins quatre des groupes B₁, B₂, ..., Bₙ₋₁ et Bₙ étant indépendamment choisis parmi les nucléobases non naturelles représentées par la **formule II, la formule III** ou **la formule IV ;**
R₁, R₂, R₃, R₄, R₅ et R₆ étant indépendamment choisis parmi un radical hydrido et alkyle substitué ou non substitué ;
Q₁ et Qₘ représentant un radical méthylène et Qₘ étant directement lié au groupe amino basique ;
Q₂, Q₃, ..., et Qₘ₋₁ étant indépendamment choisis parmi un radical méthylène et oxygène ; et
m représentant un entier compris entre 1 et 10.

5. Dérivé d'acide nucléique peptidique selon la revendication 1, ou sel pharmaceutiquement acceptable de celui-ci :
n représentant un entier compris entre 12 et 18 ;
le composé de **formule I** possédant au moins un chevauchement complémentaire 10-mère avec la séquence d'ARN 14-mère de [(5' → 3') UUUUUGCGUAAGUA] au sein du pré-ARNm de SCN9A humain ;
le composé de **formule I** étant totalement complémentaire à la séquence de pré-ARNm cible ;
S₁, S₂, ..., Sₙ₋₁, Sn, T₁, T₂, ..., Tₙ₋₁ et Tn représentant un radical hydrido ;
X et Y représentant indépendamment un radical hydrido [H], alkylacyle substitué ou non substitué, arylacyle substitué ou non substitué, ou alkyloxycarbonyle substitué ou non substitué ;
Z représentant un radical amino substitué ou non substitué ;
B₁, B₂, ..., Bₙ₋₁ et Bₙ étant indépendamment choisis parmi les nucléobases naturelles comprenant l'adénine, la thymine, la guanine et la cytosine et les nucléobases non naturelles ;
au moins cinq des groupes B₁, B₂, ..., Bₙ₋₁ et Bₙ étant indépendamment choisis parmi les nucléobases non naturelles représentées par la **formule II, la formule III** ou **la formule IV ;**
R₁, R₃ et R₅ représentant un radical hydrido et R₂, R₄ et R₅ représentant indépendamment un radical hydrido, ou alkyle substitué ou non substitué ;
Q₁ et Qₘ représentant un radical méthylène et Qₘ étant directement lié au groupe amino basique ;
Q₂, Q₃, ..., et Qₘ₋₁ étant indépendamment choisis parmi un radical méthylène et oxygène ; et,
m représentant un entier compris entre 1 et 10.

6. Dérivé d'acide nucléique peptidique selon la revendication 1, ou sel pharmaceutiquement acceptable de celui-ci :
n représentant un entier compris entre 12 et 16 ;
le composé de **formule I** possédant au moins un chevauchement complémentaire 10-mère avec la séquence d'ARN 14-mère de [(5' → 3') UUUUUGCGUAAGUA] au sein du pré-ARNm de SCN9A humain ;
le composé de **formule I** étant totalement complémentaire à la séquence de pré-ARNm cible ;
S₁, S₂, ..., Sₙ₋₁, Sn, T₁, T₂, ..., Tₙ₋₁ et Tn représentant un radical hydrido ;
X et Y représentant indépendamment un radical hydrido [H], alkylacyle substitué ou non substitué, arylacyle substitué ou non substitué, ou alkyloxycarbonyle substitué ou non substitué ;
Z représentant un radical amino substitué ou non substitué ;
B₁, B₂, ..., Bₙ₋₁ et Bₙ étant indépendamment choisis parmi les nucléobases naturelles comprenant l'adénine, la thymine, la guanine et la cytosine et les nucléobases non naturelles ;
au moins cinq des groupes B₁, B₂, ..., Bₙ₋₁ et Bₙ étant indépendamment choisis parmi les nucléobases non naturelles représentées par la **formule II, la formule III** ou **la formule IV ;**
R₁, R₂, R₃, R₄, R₅ et R₆ représentant un radical hydrido ;
Q₁ et Qₘ représentant un radical méthylène et Qₘ étant directement lié au groupe amino basique ;
Q₂, Q₃, ..., et Qₘ₋₁ étant indépendamment choisis parmi un radical méthylène et oxygène ; et,
m représentant un entier compris entre 1 et 10.

7. Dérivé d'acide nucléique peptidique selon la revendication 1, ou sel pharmaceutiquement acceptable de celui-ci :
n représentant un entier compris entre 12 et 16 ;
le composé de **formule I** possédant au moins un chevauchement complémentaire 10-mère avec la séquence d'ARN 14-mère de [(5' → 3') UUUUUGCGUAAGUA] au sein du pré-ARNm de SCN9A humain ;
le composé de **formule I** étant totalement complémentaire à la séquence de pré-ARNm cible ;
S₁, S₂, ..., Sₙ₋₁, Sn, T₁, T₂, ..., Tₙ₋₁ et Tn représentant un radical hydrido ;
X représentant un radical hydrido ;
Y représentant un radical alkylacyle substitué ou non substitué, arylacyle substitué ou non substitué, ou alkyloxycarbonyle substitué ou non substitué ;
Z représentant un radical amino substitué ou non substitué ;
B₁, B₂, ..., Bₙ₋₁ et Bₙ étant indépendamment choisis parmi les nucléobases naturelles comprenant l'adénine, la thymine, la guanine et la cytosine et les nucléobases non naturelles ;
au moins cinq des groupes B₁, B₂, ..., Bₙ₋₁ et Bₙ étant indépendamment choisis parmi les nucléobases non naturelles représentées par la **formule II, la formule III** ou **la formule IV ;**
R₁, R₂, R₃, R₄, R₅ et R₆ représentant un radical hydrido ;
L₁ représentant -(CH₂)₂-O-(CH₂)₂-, -CH₂-O-(CH₂)₂-, -CH₂-O-(CH₂)₃-, -CH₂-O-(CH₂)₄-, ou -CH₂-O-(CH₂)₅-, l'extrémité à droite étant directement liée au groupe amino basique ; et,
L₂ et L₃ étant indépendamment choisis parmi -(CH₂)₂-O-(CH₂)₂-, -(CH₂)₃-O-(CH₂)₂-,-(CH₂)₂-O-(CH₂)₃-, -(CH₂)₂-, -(CH₂)₃-, -(CH₂)₄-, -(CH₂)₅-, -(CH₂)₆-, -(CH₂)₇- et -(CH₂)₈-, l'extrémité à droite étant directement liée au groupe amino basique.

8. Dérivé d'acide nucléique peptidique selon la revendication 1, qui est choisi dans le groupe des dérivés d'acide nucléique peptidique fournis ci-dessous, ou sel pharmaceutiquement acceptable de celui-ci :
(N → C) Fmoc-TA(5)A-A(5)TA(5)-CGC(1O2)-AA(5)A-A(5)A-NH₂ ;
(N → C) Féthoc-TA(5)A-A(5)TA(5)-CGC(1O2)-AA(5)A-A(5)A-NH₂ ;
(N → C) Piv-TA(5)A-A(5)TA(5)-CGC(1O2)-AA(5)A-A(5)A-NH₂ ;
(N → C) FAM-HEX-TA(5)A-A(5)TA(5)-CGC(1O2)-AA(5)A-A(5)A-NH₂ ;
(N → C) acétyl-TA(5)A-A(5)TA(5)-CGC(1O2)-AA(5)A-A(5)A-NH₂ ;
(N → C) Féthoc-Lys-TA(5)A-A(5)TA(5)-CGC(1O2)-AA(5)A-A(5)A-NH₂ ;
(N → C) H-TA(5)A-A(5)TA(5)-CGC(1O2)-AA(5)A-A(5)A-NH₂ ;
(N → C) Me-TA(5)A-A(5)TA(5)-CGC(1O2)-AA(5)A-A(5)A-NH₂ ;
(N → C) benzyl-TA(5)A-A(5)TA(5)-CGC(1O2)-AA(5)A-A(5)A-NH₂ ;
(N → C) Féthoc-TA(5)A-A(5)TA(5)-CGC(1O2)-AA(5)A-A(5)A-Lys-NH₂ ;
(N → C) Fmoc-TA(5)A-A(5)TA(5)-CGC(1O2)-AA(5)A-A(5)AC-A(5)A-NH₂ ;
(N → C) Féthoc-TA(5)C-GC(1O2)A-A(5)AA(5)-ACA(5)-A-NH₂ ;
(N → C) Féthoc-TA(6)C-GC(1O2)A-A(6)AA(6)-ACA(6)-A-NH₂ ;
(N → C) Féthoc-AC(1O2)T-TA(5)C-G(6)CA-A(5)AA(5)-AC(1O2)A-A(5)-NH₂ ;
(N → C) Féthoc-AG(5)T-A(5)CT-TA(5)C-GC(1O2)A-A(5)AA(5)-ACA(5)-A-NH₂ ;
(N → C) Féthoc-AG(5)T-A(5)CT-TA(5)C-GC(1O2)A-A(5)AA(5)-A-NH₂ ;
(N → C) Féthoc-AG(5)T-A(5)CT-TA(5)C-GC(1O2)A-A(5)AA(2O2)-A-NH₂ ;
(N → C) Féthoc-Val-AG(5)T-A(5)CT-TA(5)C-GC(1O2)A-A(5)AA(2O2)-A-NH₂ ;
(N → C) Féthoc-Gly-AG(5)T-A(5)CT-TA(5)C-GC(1O2)A-A(5)AA(2O2)-A-NH₂ ;
(N → C) Féthoc-AG(5)T-A(5)CT-TA(5)C-GC(1O2)A-A(5)AA(2O2)-A-Lys-NH₂ ;
(N → C) Piv-AG(5)T-A(5)CT-TA(5)C-GC(1O2)A-A(5)AA(5)-A-NH₂ ;
(N → C) Féthoc-Lys-AG(5)T-A(5)CT-TA(5)C-GC(1O2)A-A(5)A-NH₂ ;
(N → C) Piv-Leu-AG(5)T-A(5)CT-TA(5)C-GC(1O2)A-A(5)AA(2O2)-A-NH₂ ;
(N → C) Féthoc-A(5)GT-A(5)CT-TA(5)C-G(6)CA(5)-A-NH₂ ;
(N → C) Féthoc-Lys-A(5)TC(1O3)-A(5)CT-TA(5)C-GC(1O2)A-A(5)A-NH₂ ;
(N → C) Féthoc-Gly-A(5)TC(1O3)-A(5)CT-TA(5)C-GC(1O2)A-A(5)A-Arg-NH₂ ;
(N → C) H-CTT-A(5)CG(3)- C(1O2)AA(5)-AA(5)A-C(1O3)AA(5)-NH₂ ;
(N → C) Féthoc-CTT-A(5)CG(6)-C(1O2)AA(5)-AA(5)A-C(1O2)AA(5)-NH₂ ;
(N → C) Féthoc-CTT-A(5)CG(6)-C(1O2)TA(5)-AA(5)T-C(1O2)AA(5)-NH₂ ;
(N → C) Benzoyl-CTT-A(5)CG(2O2)-C(1O2)AA(5)-AA(5)A-C(1O5)AA(5)-NH₂ ;
(N → C) n-Propyl-CTT-A(5)CG(2O3)-C(1O2)AA(3)-AA(5)A-C(2O2)AA(5)-NH₂ ;
(N → C) p-Toluènesulfonyl-CTT-A(5)CG(6)-C(1O2)AA(8)-AA(5)A-C(1O2)AA(5)-NH₂;
(N → C) [N-(2-Phényléthyl)amino]carbonyl-CTT-A(5)CG(6)-C(1O2)AA(2O2)-AA(5)A-C(10O)A A(5)-NH₂ ;
(N → C) Féthoc-Lys-Leu-CTT-A(5)CG(6)-C(1O2)AA(4)-AA(5)A-C(1O2)AA(5)-Lys-NH₂;
(N → C) N-phényl-N-Me-CTT-A(5)CG(6)-C(1O2)AA(5)-AA(5)A-C(1O2)AA(5)-Lys-NH₂;
(N → C) Féthoc-AA(5)G-TA(5)C-TTA(5)-CG(6)C-A(5)A-NH₂ ; et,
(N → C) Féthoc-AA(5)G-TA(5)C-TTA(5)-CG(6)C-A(5)A-Lys-NH₂ ;
dans lesquels,
A, G, T et C représentant des monomères PNA avec une nucléobase naturelle d'adénine, de guanine, de thymine et de cytosine, respectivement ;
C(pOq), A(p), A(pOq), G(p) et G(pOq) représentant des monomères PNA avec une nucléobase non naturelle représentée par **la formule VI, la formule VII, la formule VIII**, **la formule IX** et **la formula X**, respectivement : 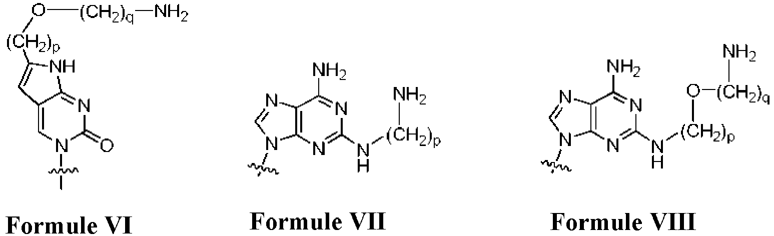 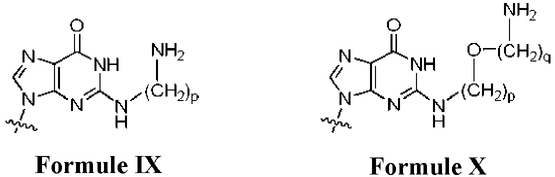
dans lesquelles,
p et q représentent des entiers ; et,
les abréviations pour les substituants des terminaisons N et C étant telles que spécifiquement décrites comme suit :« Fmoc » est l'abréviation pour « [(9-fluorényl)méthyloxy]carbonyl- » ; « Féthoc » pour « [2-(9-fluorényl)éthyl-1-oxy]carbonyl » ; « Ac- » pour « acétyl- » ; « Benzoyl- » pour « benzènecabonyl- » ; « Piv- » pour « pivalyl- » ; « Me- » pour « méthyl- » ; « n-Propyl- » pour « 1-(n-propyl)- »; « H- » pour un groupe « hydrido- » ; « p-Toluènesulfonyl » pour « (4-méthylbenzène)-1-sulfonyl- » ; « -Lys- » pour un résidu d'acide aminé « lysine » ; « -Val- » pour un résidu d'acide aminé « valine » ; « -Leu- » pour un résidu d'acide aminé « leucine » ; « -Arg- » pour un résidu d'acide aminé « arginine » ; « - Gly- » pour un résidu d'acide aminé « glycine » ; « [N-(2-Phényléthyl)amino]carbonyl- » pour « [N-1-(2-phényléthyl)amino]carbonyl- » ; « Benzyl- » pour « 1-(phényl)méthyl- » ; « Phényl- » pour « phényl- » ; « Me- » pour « méthyl- » ; « -HEX- » pour « 6-amino-1-hexanoyl- », « FAM- » pour « 5, ou 6-fluorescéin-carbonyl- (mélange isomère) » et « -NH₂ » pour un groupe « -amino » non subsitué.

9. Dérivé d'acide nucléique peptidique selon la revendication 8, qui est choisi dans le groupe des dérivés d'acide nucléique peptidique fournis ci-dessous, ou sel pharmaceutiquement acceptable de celui-ci :
(N → C) Fmoc-TA(5)A-A(5)TA(5)-CGC(1O2)-AA(5)A-A(5)A-NH₂ ;
(N → C) Féthoc-TA(5)A-A(5)TA(5)-CGC(1O2)-AA(5)A-A(5)A-NH₂ ;
(N → C) Féthoc-AC(1O2)T-TA(5)C-G(6)CA-A(5)AA(5)-AC(1O2)A-A(5)-NH₂ ; et
(N → C) Fmoc-TA(5)A-A(5)TA(5)-CGC(1O2)-AA(5)A-A(5)AC-A(5)A-NH₂.

10. Dérivé d'acide nucléique peptidique selon l'une quelconque des revendications 1 à 9, pour une utilisation dans le traitement de la douleur.

11. Dérivé d'acide nucléique peptidique selon l'une quelconque des revendications 1 à 9, pour une utilisation selon la revendication 10, ladite douleur étant une douleur chronique.

12. Dérivé d'acide nucléique peptidique selon l'une quelconque des revendications 1 à 9, pour une utilisation selon la revendication 10, ladite douleur étant une douleur neuropathique.

13. Dérivé d'acide nucléique peptidique selon l'une quelconque des revendications 1 à 9, pour une utilisation en thérapie.

14. Procédé d'induction du saut de l'exon 4 dans le pré-ARNm de SCN9A pour produire un variant d'épissage d'ARNm de SCN9A dépourvu de l'exon 4 de SCN9A dans les cellules *in vitro* comprenant la mise en contact des cellules avec le dérivé d'acide nucléique peptidique selon l'une quelconque des revendications 1 à 9.

15. Procédé selon la revendication 14, lesdites cellules exprimant un taux plus faible d'un ARNm de SCN9A pleine longueur et présentant une activité fonctionnelle Naᵥ1.7 plus faible que les cellules sans mise en contact avec le dérivé d'acide nucléique peptidique.
